# EUROPEAN PATENT APPLICATION

(11) **EP 4 053 285 A2**
(43) Date of publication of application: **07.09.2022**
(21) Application number: 20883082.8
(22) Date of filing: 29.10.2020
(51) Int. Cl.: C12N 15/85, C12N 9/22, C12N 9/78, C12N 15/10, C12N 15/113

(54) **ENGINEERED GUIDE RNA FOR INCREASING EFFICIENCY OF CRISPR/CAS12F1 SYSTEM, AND USE OF SAME**

(30) Priority: 29.10.2019 KR 20190135935
(71) Applicant: Genkore Inc, Yuseong-gu, Daejeon 34141 (KR)
(72) Inventor: KIM, Yong Sam, Daejeon 34118 (KR); KIM, Do Yon, Daejeon 34069 (KR); KO, Jeong Heon, Daejeon 34069 (KR)
(74) Representative: Schön, Christoph
(86) International application number: PCT/KR2020/014961
(87) International publication number: WO 2021/086083

(57) **Abstract**

Provided are an engineered CRISPR/Cas12f1 system of which editing efficiency is improved compared to a CRISPR/Cas12f1 system, and a use thereof. A U-rich tail sequence is provided that can be introduced into an engineered guide RNA to improve the editing efficiency of the CRISPR/Cas12f1 system. The U-rich tail sequence has uridine abundantly, and may further include additional nucleic acids in addition to uridine depending on the environment in which the guide RNA is actually used and the environment in which the guide RNA is expressed. The inventors of the present application described the structure of the U-rich tail sequence, the position of introduction, and the effect thereof in experiments.

## Description

### [Technical Field]

The present disclosure relates to a technique for an area in which a CRISPR/Cas system, particularly, a CRISPR/Cas12f1 system is used for gene editing.

### [Background Art]

A CRISPR/Cas12f1 system is a CRISPR/Cas system which is classified as Class 2, Type V. A previous study (Harrington et al., Programmed DNA destruction by CRISPR-Cas14 enzymes, Science 362, 839-842 (2018)) has reported the CRISPR system as CRISPR/Cas14 which is an Archaea-derived CRISPR/Cas system for the first time. Since then, a subsequent study (Karvelis et al., Nucleic Acids Research, Vol. 48, No. 9 5017 (2020)) classified the CRISPR/Cas14 system into a CRISPR/Cas12f system. The CRISPR/Cas12f1 system belongs to a V-F1 system which is one subtype among the CRISPR/Cas systems which are classified into the Class 2, Type V, and includes a CRISPR/Cas14a system having Cas14a as an effector protein. The CRISPR/Cas12f1 system is characterized by the compact size of an effector protein compared to that of CRISPR/Cas9 system. However, as revealed in the previous study (Harington et al., Programmed DNA destruction by miniature CRISPR-Cas14 enzymes, Science 362, 839-842 (2018), US 2020/0190494 A1), the CRISPR/Cas12f1 system, particularly, the CRISPR/Cas14a system exhibits the ability to cleave single-stranded DNA, but has no or extremely low cleavage activity for double-stranded DNA, so that its application to gene editing technology is limited.

### [Disclosure]

### [Technical Problem]

An aspect of the present disclosure provides an engineered CRISPR/Cas12f1 system with increased gene editing efficiency.

An aspect of the present disclosure provides an engineered CRISPR/Cas12f1 system having a uracil (U)-rich tail sequence.

An aspect of the present disclosure provides engineered crRNA having a U-rich tail sequence for an engineered CRISPR/Cas12f1 system.

An aspect of the present disclosure provides engineered guide RNA having a U-rich tail sequence for an engineered CRISPR/Cas12f1 system.

An aspect of the present disclosure provides an engineered CRISPR/Cas12f1 complex having a U-rich tail sequence for an engineered CRISPR/Cas12f1 system.

An aspect of the present disclosure provides a vector having a nucleic acid sequence encoding each component of an engineered CRISPR/Cas12f1 system.

An aspect of the present disclosure provides a gene editing method using the engineered CRISPR/Cas12f1 system.

An aspect of the present disclosure provides the use of the engineered CRISPR/Cas12f1 system.

### [Technical Solution]

In an embodiment, an aspect of the present disclosure provides an engineered CRISPR RNA (crRNA) for a CRISPR/Cas12f1 system capable of editing a nucleic acid including a target sequence, comprising:
a CRISPR RNA repeat sequence;
a spacer sequence which is complementary to the target sequence; and
a U-rich tail sequence which is linked to 3'-terminus of the spacer sequence,
wherein the U-rich tail sequence is represented by (UₐN)ₙU_{b},
N is one of adenosine (A), uracil (U), cytidine (C), and guanosine (G),
a is an integer between 1 to 4, n is an integer selected from 0, 1, and 2, and b is an integer between 1 to 10, and
the engineered CRISPR RNA includes the CRISPR RNA repeat sequence, the spacer sequence, and the U-rich tail sequence which are linked sequentially in the 5' -to-3' direction.

In an embodiment, an aspect of the present disclosure provides a DNA having a sequence encoding the engineered crRNA.

In an embodiment, the U-rich tail sequence may be UUUUUU, UUUUAUUUUUU, or UUUUGUUUUUU

In an embodiment, the CRISPR RNA repeat sequence may be the sequence of SEQ ID NO: 58.

In an embodiment, an aspect of the present disclosure provides an engineered guide RNA of a CRISPR/Cas12f1 system capable of editing a nucleic acid including a target sequence, comprising:
a tracrRNA sequence;
a crRNA sequence including a CRISPR RNA repeat sequence, and a spacer sequence which is complementary to the target sequence; and
a U-rich tail sequence which is linked to 3'-terminus of the spacer sequence,
wherein the U-rich tail sequence is represented by (UₐN)ₙU_{b},
N is one of adenosine (A), uracil (U), cytidine (C), and guanosine (G),
a is an integer between 1 to 4 inclusive, n is an integer selected from 0, 1, and 2, and b is an integer between 1 to 10 inclusive.

In an embodiment, the engineered guide RNA further includes a linker sequence, through which the tracrRNA sequence and the crRNA sequence are linked.

In an embodiment, the linker sequence may be 5'-gaaa-3'.

In an embodiment, the U-rich tail sequence may be UUUUUU, UUUUAUUUUUU, or UUUUGUUUUUU

In an embodiment, an aspect of the present disclosure provides a DNA having a sequence encoding the engineered guide RNA.

In an embodiment, an aspect of the present disclosure provides an engineered CRISPR/Cas12f1 complex capable of editing a nucleic acid including a target sequence, including:
a Cas12f1 protein belonging to the type CRISPR14 system; and
an engineered guide RNA including: a scaffold sequence which interacts with the Cas12f1 protein; a spacer sequence which is complementary to the target sequence; and a U-rich tail sequence,
wherein the U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive.

In an embodiment, an aspect of the present disclosure provides a vector for expressing an engineered CRISPR/Cas12f1 complex capable of editing a nucleic acid including a target sequence, the vector including:
a first sequence including a sequence that encodes a Cas12f1 protein;
a first promoter sequence operably linked to the first sequence;
a second sequence including a sequence that encodes an engineered guide RNA,
wherein the engineered guide RNA has a scaffold sequence which interacts with the Cas12f1 protein, a spacer sequence which is complementary to the target sequence, and a U-rich tail sequence linked to the 3'-terminus of the spacer sequence,
the U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive; and
a second promoter sequence operably linked to the second sequence;
wherein the vector is constructed to express the Cas12f1 protein and the engineered guide RNA,
and thus, the Cas12f1 protein and the engineered guide sequence can form the CRISPR/Cas12f1 complex in a cell, and
the nucleic acid including a target sequence can be edited by the CRISPR/Cas12f1 complex.

In an embodiment, the second promoter sequence may be a U6 promoter sequence.

In an embodiment, the vector may be a plasmid vector.

In an embodiment, the vector may be a viral vector.

In an embodiment, the vector may be one or more selected from the group consisting of a retrovirus, a lentivirus, an adenovirus, an adeno-associated virus (AAV), a vaccinia virus, a poxvirus and a herpes simplex virus.

In an embodiment, the vector may be a linear PCR amplicon.

An aspect of the present disclosure provides a vector for expressing an engineered CRISPR/Cas12f1 complex capable of editing a nucleic acid including a first target sequence and a second target sequence, the vector including:
a first sequence including a sequence that encodes a Cas12f1 protein;
a first promoter sequence operably linked to the first sequence;
a second sequence including a sequence that encodes a first engineered guide RNA,
wherein the first engineered guide RNA has a first scaffold sequence which interacts with the Cas12f1 protein, a first spacer sequence which is complementary to the first target sequence, and a first U-rich tail sequence, wherein
the first U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive;
a second promoter sequence operably linked to the second sequence;
a third sequence including a sequence that encodes a second engineered guide RNA,
wherein the second engineered guide RNA has a second scaffold sequence which may interact with the Cas12f1 protein, a second spacer sequence which is complementary to the second target sequence, and a second U-rich tail sequence,
the second U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive; and
a third promoter sequence operably linked to the third sequence.

In an embodiment, the second promoter sequence and the third promoter sequence may be the same promoter sequence.

In an embodiment, the second promoter sequence may be a HI promoter sequence, and the third promoter sequence may be a U6 promoter sequence.

In an embodiment, an aspect of the present disclosure provides a method for editing a nucleic acid including a target sequence, in a cell, the method includes:
delivering a Cas12f1 protein or a nucleic acid encoding the Cas12f1 protein, and an engineered guide RNA or a nucleic acid encoding the engineered guide RNA, into the cell,
so that a CRISPR/Cas12f1 complex can be formed in the cell, and
the nucleic acid including the target sequence can be edited by the CRISPR/Cas12f1 complex,
wherein the engineered guide RNA has a scaffold sequence which interacts with the Cas12f1 protein, a spacer sequence which is complementary to the target sequence, and a U-rich tail sequence linked to the 3'-terminus of the spacer sequence,
wherein the U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive.

In an embodiment, in the delivering, the Cas12f1 protein and the engineered guide RNA may be introduced in the form of a CRISPR/Cas12f1 complex into the cell.

In an embodiment, in the delivering, a vector including the nucleic acid encoding the Cas12f1 protein and the nucleic acid encoding the engineered guide RNA may be introduced into the cell.

In an embodiment, the vector may be selected from the group consisting of a retrovirus, a lentivirus, an adenovirus, an adeno-associated virus (AAV), a vaccinia virus, a poxvirus, and a herpes simplex virus.

In an embodiment, an aspect of the present disclosure provides a method for editing a nucleic acid including a target sequence, in the cell, the method including:
bringing a CRISPR/Cas12f1 complex in contact with the nucleic acid including the target sequence,
wherein, the CRISPR/Cas12f1 complex includes a Cas12f1 protein and an engineered guide RNA,
the engineered guide RNA includes a scaffold sequence which interacts with the Cas12f1 protein, a spacer sequence which is complementary to the target sequence, and a U-rich tail sequence,
the U-rich tail sequence is represented by (UₐN)ₙU_{b},
N is one of adenosine (A), uracil (U), cytidine (C), and guanosine (G), and
wherein a is an integer between 1 to 4 inclusive, n is an integer selected from 0, 1, and 2, and b is an integer between 1 to 10 inclusive.

### [Advantageous Effects]

The engineered CRISPR/Cas12f1 system having the U-rich tail sequence provided in the present disclosure exhibits higher gene editing efficiency when used for gene editing than when a wild-type CRISPR/Cas12f1 system is used.

### [Description of Drawings]

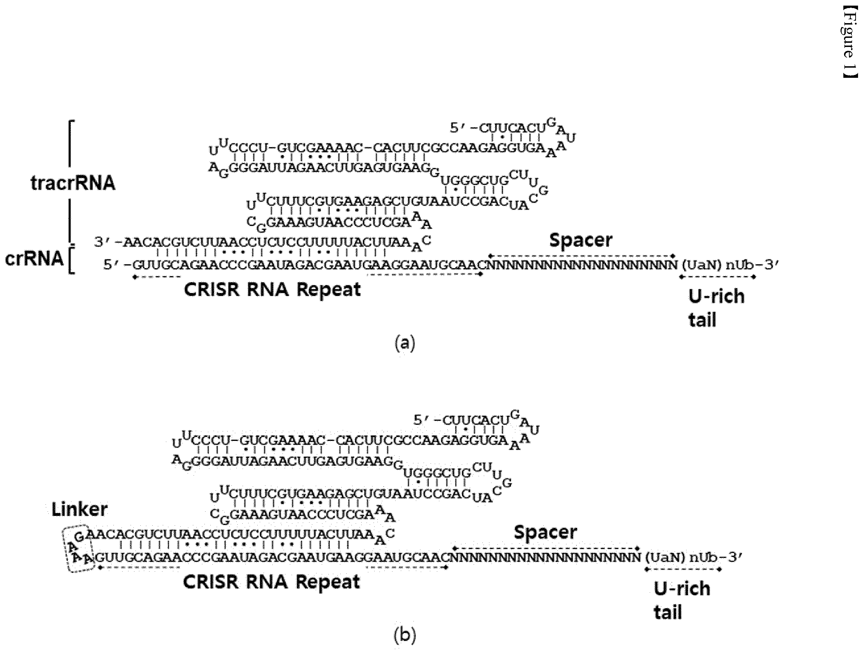

FIG. 1 shows an example structure of an engineered guide RNA provided in the present disclosure. (a) shows an example of the structure of a dual guide RNA, including a part of tracrRNA and a repeat sequence of CRISPR RNA (crRNA). The complementary sequences bind each other to form a double-stranded RNA. The names of crRNA parts (a CRISPR RNA repeat sequence part, a spacer sequence part, and a U-rich tail sequence part) are illustrated. (b) shows an example of the structure of a single guide RNA, in which the tracrRNA and the crRNA are linked via a linker. The positions of the CRISPR RNA repeat sequence part, the spacer sequence part, and the U-rich tail sequence part are illustrated. Each sequence illustrated in the drawings is an example sequence.

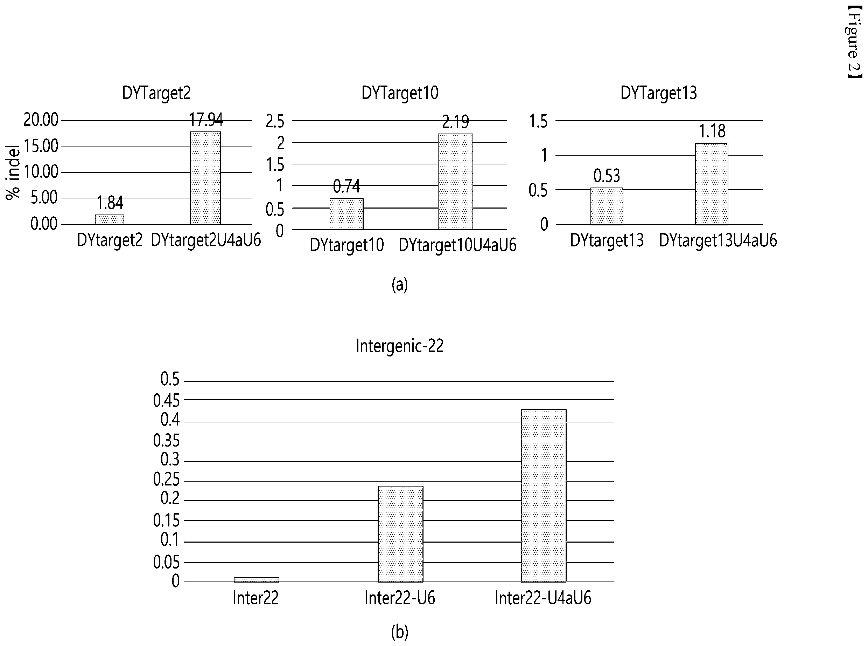

FIG. 2 is a graph showing the results of Experimental Example 3. (a) is a graph showing the indel efficiencies of CRISPR/Cas12f1 system carrying a 3'-U4AU6 U-rich tail crRNA sequence for DYTarget2, DYtarget10 and DYtarget13 in HEK293T cells. In this regard, the indel efficiency of the engineered CRISPR/Cas12f1 system having a U-rich tail sequence is remarkably higher than the indel efficiency of the wild-type CRISPR/Cas12f1 system, (b) is a graph showing the indel efficiencies which the Cas12f1 guide RNA carrying either a U6 or a U4AU6 sequence shows for Intergenic-22 in HEK293T cells.

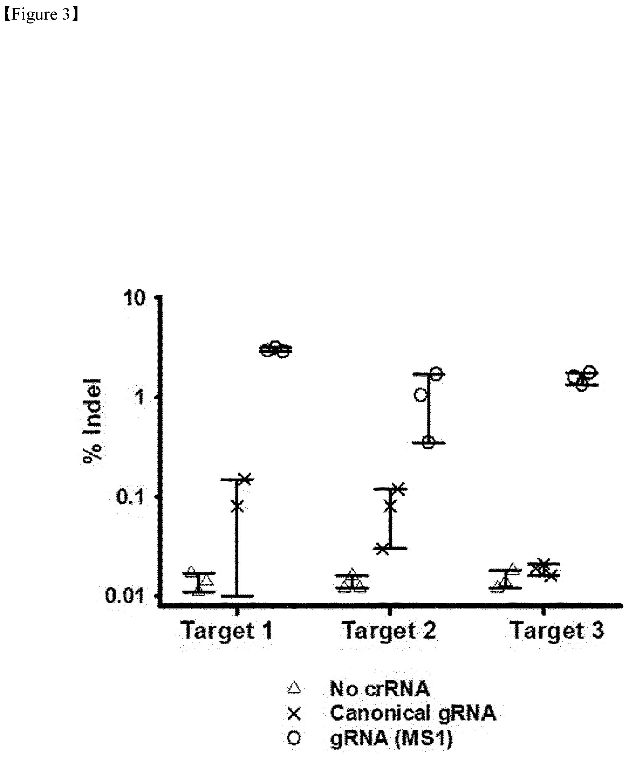

FIG. 3 is a graph showing the results of Experimental Example 4. It shows the indel efficiencies of an engineered CRISPR/Cas12f1 system with respect to Target 1 (DYtarget2), Target 2 (DYtarget10), Target 3 (Intergenic-22) in HEK293T cells. No crRNA, Canonical gRNA, and gRNA (MS1) refer to the indel efficiency of the crRNA-untreated control, the wild-type CRISPR/Cas12f1 system, and the engineered CRISPR/Cas12f1 system having a crRNA carrying a U4AU6 structure, respectively.

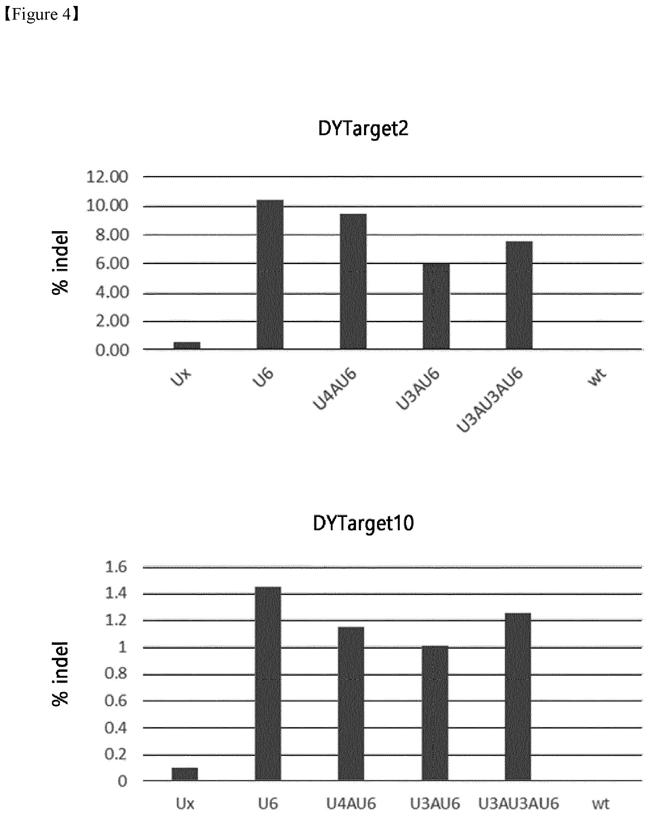

FIG. 4 is a graph showing the results of Experimental Example 7. It shows the indel efficiencies of CRISPR/Cas12f1 systems with respect to DYtarget2 or DYtarget10 in HEK293 T cells. (a) shows the indel efficiency for DYtarget2 in HEK293 T cells. WT shows the indel efficiency of an experimental group for which sgRNA is not treated. Ux shows the indel efficiency of an experimental group for which no U was not added to the 3'-terminus of spacer sequence of sgRNA. U6, U4AU6, U3AU6, and U3AU3AU6 refer to the 3'-tail sequences of sgRNA.

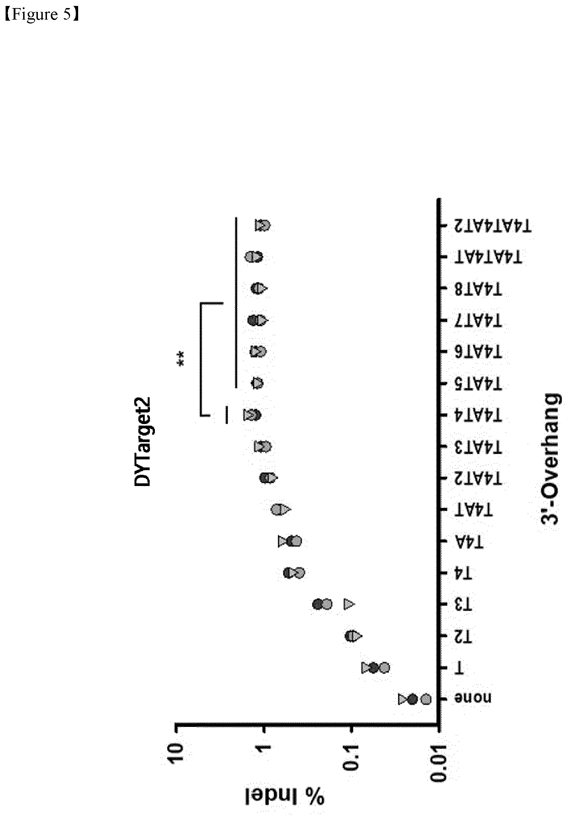

FIG. 5 is a graph showing the results of Experimental Example 8. It shows the indel efficiency of an engineered CRISPR/Cas12f1 system for DYtarget2 in HEK293 T cells. None shows an indel efficiency of an experimental group for which no U was added to an sgRNA. T, T2, T3, T4, T4A, T4AT, T4AT2, T4AT3, T4AT4, T4AT5, T4AT6, T4AT7, T4AT8, T4AT4AT, and T4AT4AT2 refer to an indel efficiency of an experimental group using an engineered sgRNA having a 3' end sequence with a U, U2, U3, U4, U4A, U4AU, U4AU2, U4AU3, U4AU4, U4AU5, U4AU6, U4AU7, U4AU8, U4AU4AU, and U4AU4AU2 structure, respectively.

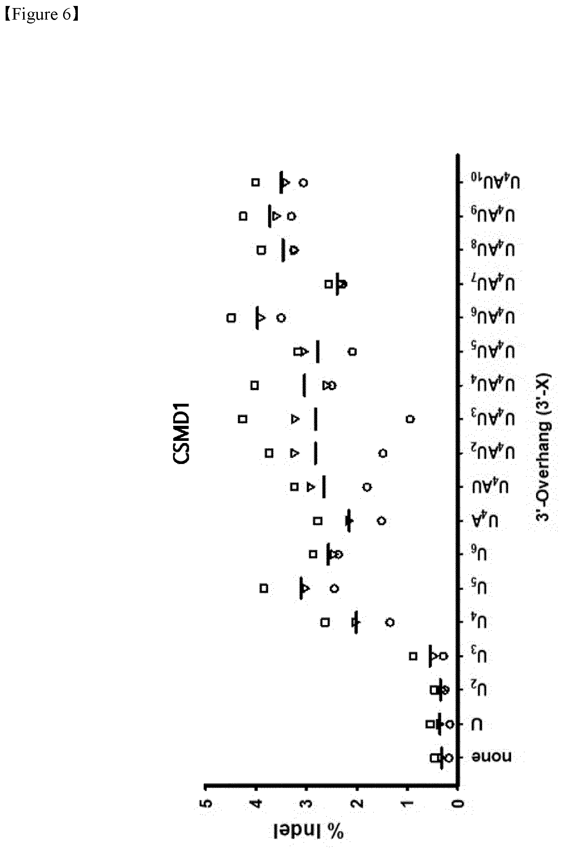

FIG. 6 is a graph showing the results of Experimental Example 9. It shows the indel efficiency of CRISPR/Cas12f1 system for CSMD1 in HEK293 T cells. None shows the indel efficiency of an experimental group for which no U was added to an sgRNA. U, U2, U3, U4, U5, U6, U4A, U4AU, U4AU2, U4AU3, U4AU4, U4AU5, U4AU6, U4AU7, U4AU8, U4AU9, and U4AU10 show the indel efficiencies of experimental groups for which an sgRNA shows the corresponding sequence at the 3'-terminus.

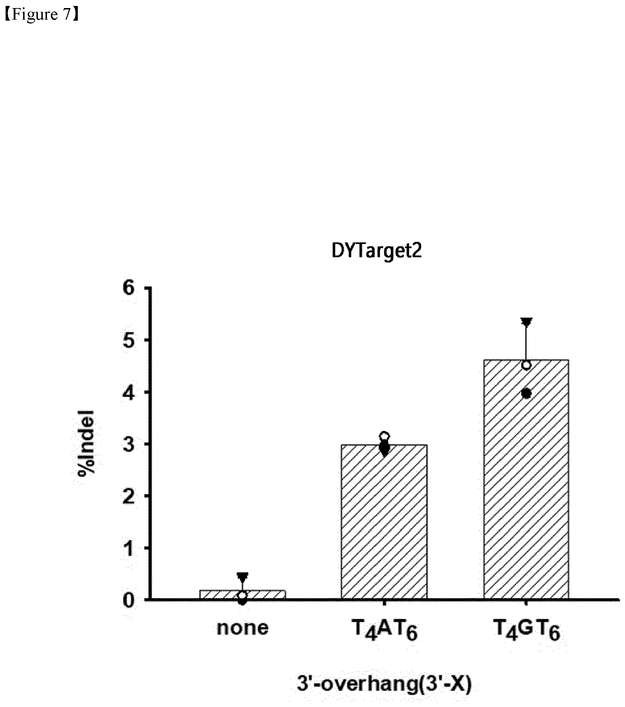

FIG. 7 is a graph showing the results of Experimental Example 10. It shows the indel efficiency of CRISPR/Cas12f1 system for DYtarget2 in HEK293 T cells. None refers to the indel efficiency of an experimental group for which no U was added to an sgRNA. T4AT6 and T4GT6 refer to an indel efficiency of an experimental group using an engineered sgRNA having a U-rich tail sequence with a U4AU6 structure, and an indel efficiency of an experimental group using an engineered sgRNA having a U-rich tail sequence with a U4GU6 structure, respectively.

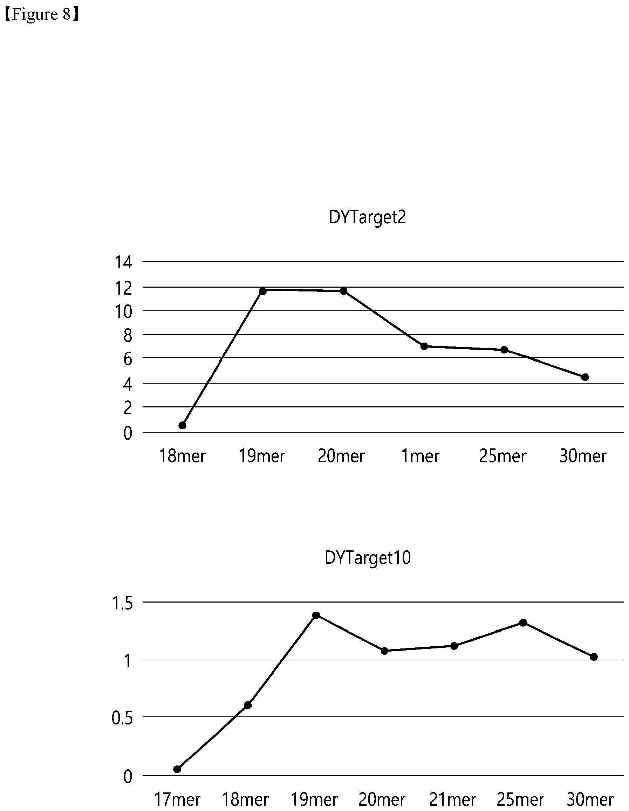

FIG. 8 is a graph showing the results of Experimental Example 11. It shows the indel efficiency of an engineered CRISPR/Cas12f1 system for DYtarget2 and DYtarget10 in HEK293 T cells. (a) indicates the indel efficiencies of the sgRNA having U4AU6 U-rich tail sequence for DYtarget2 in HEK293 T cells for all experimental groups. In this regards, 18 mer, 19 mer, 20 mer, 21 mer, 25 mer, and 30 mer on the horizontal axis of the graph indicate the length of the spacer sequence of the sgRNAs. The spacer sequence is shown in Table 25 of Experimental Example 11. (b) indicates the indel efficiencies of the sgRNA having U4AU6 U-rich tail sequence for DYtarget10 in HEK293 T cells for all experimental groups. In this regard, 17mer, 18 mer, 19 mer, 20 mer, 21 mer, 25 mer, and 30 mer on the horizontal axis of the graph indicate the length of the spacer sequence of the sgRNAs. The spacer sequence is shown in Table 24 of Experimental Example 11.

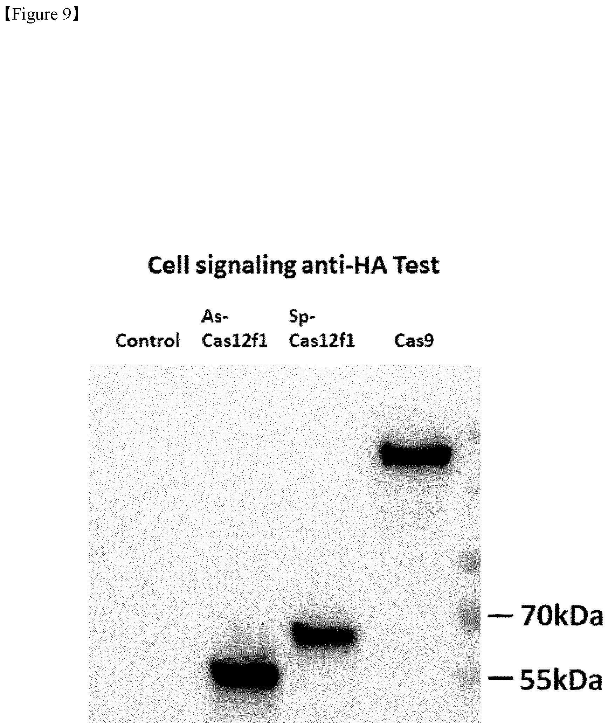

FIG. 9 shows the results of western blot analysis using HA antibody for Experimental Example 12-1. In this regard, it was confirmed that AsCas12f1 and SpCas12f1 effector proteins were expressed in HEK293T cells. The AsCas12f1 and SpCas12f1 proteins show a molecular mass of 56.55 kDa and 64.65 kDa, respectively.

### [Modes for Carrying Out the Invention]

### Definition of terms

### About

As used herein, the term "about" refers to an amount, a level, a value, a number, a frequency, a percentage, a dimension, a size, a quantity, a weight, or a length that varies to the degree of 30, 25, 20, 25, 10, 9, 8, 7, 6, 5, 4, 3, 2, or 1% with respect to a reference amount, level, value, number, frequency, percentage, dimension, size, quantity, weight, or length.

### Operably linked

As used herein, the term "operably linked" refers to the state of being linked such that in the gene expression technique, a particular component permits another component to function in an intended manner. For example, the case where a promoter sequence is operably linked to a coding sequence means that the promoter is linked so as to affect the intracellular transcription and/or expression of the coding sequence. Further, the term includes all meanings that can be recognized by those skilled in the art, and may be appropriately interpreted according to the context.

### Target gene or target nucleic acid

As used herein, "target gene" or "target nucleic acid" basically refers to an intracellular gene or nucleic acid that is targetable by the CRISPR system. The target gene or target nucleic acid may be used interchangeably and may refer to the same subject. The target gene or target nucleic acid may refer to a unique gene or nucleic acid possessed by a target cell, or an externally derived gene or nucleic acid unless otherwise described, and is not particularly limited as long as it may be the target of gene editing. The target gene or target nucleic acid may be single-stranded DNA, double-stranded DNA, and/or RNA. Further, the term includes all meanings that can be recognized by those skilled in the art, and may be appropriately interpreted according to the context.

### Target sequence

As used herein, "target sequence" refers to a particular sequence that a CRISPR/Cas complex recognizes to cleave a target gene or target nucleic acid. The target sequence may be appropriately selected according to the purpose thereof. Specifically, "target sequence" is a sequence included in a target gene or target nucleic acid sequence, and refers to a sequence that is complementary to a spacer sequence included in a guide RNA or an engineered guide RNA provided in the present specification. In general, the spacer sequence is determined in consideration of a target gene or target nucleic acid sequence and a PAM sequence recognized by an effector protein of a CRISPR/Cas system. The target sequence may refer to only a particular strand that complementarily binds to a guide RNA of the CRISPR/Cas complex, and may also refer to the entire target double strand including the particular strand part, which is appropriately interpreted according to the context. Further, the term includes all meanings that can be recognized by those skilled in the art, and may be appropriately interpreted according to the context.

### Vector

As used herein, "vector" collectively refers to all materials capable of carrying a genetic material into a cell, unless otherwise specified. For example, a vector may be a DNA molecule including a genetic material of interest, for example, a nucleic acid encoding an effector protein of the CRISPR/Cas system, and/or a nucleic acid encoding a guide RNA, but is not limited thereto. The term includes all meanings that can be recognized by those skilled in the art, and may be appropriately interpreted according to the context.

### Engineered

As used herein, "engineered" is a term used to distinguish a material, a molecule, and the like, which have a structure that is already present in nature, and refers to those in which the material, molecule, and the like are artificially modified. For example, the case of "engineered guide RNA" refers to a guide RNA in which the composition of a guide RNA that is present in nature is artificially modified. Further, the term includes all meanings that can be recognized by those skilled in the art, and may be appropriately interpreted according to the context.

### Nuclear localization sequence, or signal (NLS)

As used herein, "NLS" refers to a peptide having a certain length, which serves as a type of "tag" by being attached to a protein to be transported, or a sequence thereof, when a material outside the cell nucleus is transported into the nucleus by a nuclear transport action. Specifically, the NLS may be an NLS of SV40 virus large T-antigen having an amino acid sequence PKKKRKV (SEQ ID NO: 237); an NLS from a nucleoplasmin (for example, a nucleoplasmin bipartite NLS having a sequence KRPAATKKAGQAKKKK(SEQ ID NO: 238)); a c-myc NLS having an amino acid sequence PAAKRVKLD (SEQ ID NO: 239) or RQRRNELKRSP (SEQ ID NO: 240); a hRNPA1 M9 NLS having a sequence NQSSNFGPMKGGNFGGRSSGPYGGGGQYFAKPRNQGGY (SEQ ID NO: 241) ; a sequence RMRIZFKNKGKDTAELRRRRVEVSVELRKAKKDEQILKRRNV (SEQ ID NO: 242) of an IBB domain from importin-alpha; sequences VSRKRPRP (SEQ ID NO: 243) and PPKKARED (SEQ ID NO: 244) of a myoma T protein; a sequence PQPKKKPL (SEQ ID NO: 245) of human p53; a sequence SALIKKKKKMAP (SEQ ID NO: 246) of mouse c-abl IV; sequences DRLRR (SEQ ID NO: 247) and PKQKKRK (SEQ ID NO: 248) of an influenza virus NS1 ; a sequence RKLKKKIKKL (SEQ ID NO: 249) of a hepatitis virus delta antigen; a sequence REKKKFLKRR (SEQ ID NO: 250) of a mouse Mx1 protein; a sequence KRKGDEVDGVDEVAKKKSKK (SEQ ID NO: 251) of a human poly (ADP-ribose) polymerase; or an NLS sequence derived from a sequence RKCLQAGMNLEARKTKK (SEQ ID NO: 252) of a steroid hormone receptor (human) glucocorticoid, but is not limited thereto. As used herein, the term "NLS" includes all meanings that can be recognized by those skilled in the art, and may be appropriately interpreted according to the context.

### Nuclear export sequence, or signal (NES)

As used herein, "NES" refers to a peptide having a certain length, which serves as a type of "tag" by being attached to a protein to be transported, or a sequence thereof, when a material in the cell nucleus is transported outside the nucleus by a nuclear transport action. As used herein, the term "NES" includes all meanings that can be recognized by those skilled in the art, and may be appropriately interpreted according to the context.

### Tag

As used herein, "tag" collectively refers to a functional domain added to facilitate the tracking and/or isolation and purification of a peptide or protein. Specifically, the tag includes a tag protein such as a histidine (His) tag, a V5 tag, a FLAG tag, an influenza hemagglutinin (HA) tag, a Myc tag, a VSV-G tag and a thioredoxin (Trx), an auto-fluorescent protein such as a green fluorescent protein (GFP), a yellow fluorescent protein (YFP), a cyan fluorescent protein (CFP), a blue fluorescent protein (BFP), HcRED, and DsRed, and a reporter gene such as glutathione-S-transferase (GST), horseradish peroxidase (HRP), chloramphenicol acetyltransferase (CAT), beta-galactosidase, beta-glucuronidase, and luciferase, but is not limited thereto. As used herein, the term "tag" includes all meanings that can be recognized by those skilled in the art, and may be appropriately interpreted according to the context.

### Background Art - CRISPR/Cas12f1 system

### Overview

Various types of CRISPR/Cas systems are present in nature, and new CRISPR/Cas systems are still being discovered. Among them, the CRISPR/Cas12f1 system provided in the present disclosure specifically belongs to the Class 2, Type V classification, and is a CRISPR/Cas system belonging to the Cas 14 family (Harrington et al., Programmed DNA destruction by miniature CRISPR-Cas 14enzymes, Science 362, 839-842 (2018)). Hereinafter, what classification the CRISPR/Cas12f1 system provided in the present disclosure belongs to will be described.

### Class 2 CRISPR/Cas system

The CRISPR/Cas system is roughly divided into Class 1 and Class 2. Among them, the Class 2 CRISPR/Cas system is characterized in that the effector complex thereof includes a large single protein having multiple domains. Among the Class 2 CRISPR/Cas systems, the Type II CRISPR/Cas9 system is representative, and the CRISPR/Cas system that is being actively studied for gene editing applications, such as the CRISPR/Cpfl system, usually belongs to Class 2.

### Type V CRISPR/Cas system

The Class 2 CRISPR/Cas system is divided into Type II, V, and VI. Among them, the CRISPR/Cas12f1 system provided in the present disclosure belongs to the Type V CRISPR/Cas system. An effector protein of the Type V CRISPR/Cas system is named Cas12, and is named Cas12a, Cas12b, and the like, according to a detailed classification. The Cas12 protein has one nuclease domain (RuvC-like nuclease), which is a feature distinguishing from Type II effector proteins (for example, Cas9 protein) having two nuclease domains (HNH, and RuvC domain). To date, the type V CRISPR/Cas system is divided into 11 subtypes, of which the CRISPR/Cas12f1 system provided in the present disclosure belongs to V-F1, which is a variant of one of the subtypes V-F (Makarova et. al., Nature Reviews, Microbiology volume18, 67 (2020).

### CRISPR/Cas 12f1 system

The CRISPR/Cas12f system belongs to the V-F subtype of the type V CRISPR/Cas system, which is further subdivided into variants of V-F1 to V-F3. The CRISPR/Cas12f system includes a CRISPR/Cas14 system including Cas14a, Cas14b, and Cas14c variants among the effector proteins named Cas14 in a previous study (Harrington et al., Programmed DNA destruction by miniature CRISPR-Cas14 enzymes, Science 362, 839-842 (2018)). Among them, the CRISPR/Cas14a system including the Cas14a effector protein is classified as the CRISPR/Cas12f1 system (Makarova et al., Nature Reviews, Microbiology volume 18, 67 (2020)). However, the CRISPR/Cas12f1 includes not only a Cas14a family (Harrington et al., Programmed DNA destruction by miniature CRISPR-Cas14 enzymes, Science 362, 839-842 (2018)), but also a CRISPR/Cas system in which an effector system is named c2c10 (Karvelis et al., Nucleic Acids Research, Vol. 48, No. 9 5017 (2020), Makarova et al., Nature Reviews, Microbiology volume 18, 67 (2020)). Therefore, in the present disclosure, the CRISPR/Cas12f1 system is a concept that includes both the CRISPR/Cas14a system and the CRISPR/c2c10 system unless otherwise described, and among them, when referring to the CRISPR/Cas14a system, it refers to the "CRISPR/Cas14a system" or the "CRISPR/Cas12f1 system belonging to the Cas14 family". The term has a meaning that those skilled in the art can appropriately interpret according to the context.

### Background Art - design of CRISPR/Cas system expression vector

### Overview

In order to use a CRISPR/Cas system for gene editing, a method has been widely used, in which each component of the CRISPRCas system is expressed in the cell by introducing a vector having a sequence encoding each component of the CRISPR/Cas system into the cell. Hereinafter, the components of a vector that allow the CRISPR/Cas system to be expressed in the cell will be described.

### Nucleic acid encoding component of CRISPR/Cas system

Since the purpose of the vector is to express each component of the CRISPR Cas system in the cell, the sequence of the vector should essentially include one or more of the nucleic acid sequences encoding each component of the CRISPR/Cas system. Specifically, the sequence of the vector includes a nucleic acid sequence encoding a guide RNA, and/or a Cas protein included in a CRISPR/Cas system to be expressed. In this regard, the sequence of the vector may include not only a nucleic acid sequence encoding a wild-type guide RNA and a wild-type Cas protein, but also a nucleic acid sequence encoding an engineered guide RNA and a codon optimized Cas protein, or a nucleic acid sequence encoding an engineered Cas protein, according to the purpose thereof.

### Regulatory/control component

In order to express the vector in the cell, one or more regulatory/control components should be included. Specifically, the regulatory/control component may include a promoter, an enhancer, an intron, a polyadenylation signal, a Kozak consensus sequence, an internal ribosome entry site (IRES), a splice acceptor, a 2A sequence and/or a replication origin, but is not limited thereto. In this regard, the replication origin may be an f1 replication origin, an SV40 replication origin, a pMB1 replication origin, an adeno replication origin, an AAV replication origin, and/or a BBV replication origin, but is not limited thereto.

### Promoter

In order to express an expression target of the vector in the cell, an RNA transcription factor should be activated in the cell by operably linking a promoter sequence to a sequence encoding each component. The promoter sequence may be designed differently depending on the corresponding RNA transcription factor or expression environment, and is not limited as long as the promoter sequence can appropriately express the component of the CRISPR/Cas system in the cell. The promoter sequence may be a promoter that promotes transcription of an RNA polymerase (for example, RNA Pol I, Pol II, or Pol III). For example, the promoter may be one of an SV40 initial promoter, a mouse mammary tumor virus long terminal repeat (LTR) promoter, an adenovirus major late promoter (Ad MLP), a herpes simplex virus (HSV) promoter, a cytomegalovirus (CMV) promoter such as a CMV immediate early promoter region (CMVIE), a Rous sarcoma virus (RSV) promoter, a human U6 small nuclear promoter (U6) (Miyagishi et al., Nature Biotechnology 20, 497 - 500 (2002)), an enhanced U6 promoter (e.g., Xia et al., Nucleic Acids Res. 2003 Sep 1;31(17)), and a human HI promoter (HI), but is not limited thereto.

### Termination signal

When the vector sequence includes the promoter sequence, the transcription of a sequence operably linked to the promoter is induced by an RNA transcription factor, and a sequence that induces the transcription termination of the RNA transcription factor is called a termination signal. The termination signal may vary depending on the type of promoter sequence. For example, when the promoter is a U6 or HI promoter, the promoter recognizes a continuous thymidine sequence (for example, a TTTTTT (T6) sequence) as a termination signal.

### Additional expression component

The vector may include a nucleic acid sequence encoding an additional expression component intended to be expressed by those skilled in the art, if necessary, in addition to the wild-type CRISPR/Cas system configuration and/or the engineered CRISPR/Cas system. For example, the additional expression component may be one of the tags described in the "Tag" section of the <Description of terms> section, but is not limited thereto. For example, the additional expression component may be a herbicide resistance gene such as glyphosate, glufosinate ammonium or phosphinothricin, and an antibiotic resistance gene such as ampicillin, kanamycin, G418, bleomycin, hygromycin, and chloramphenicol, but is not limited thereto.

### Form of expression vector

The expression vector may be designed in the form of a linear or circular vector.

### Limitations of related art

The major limitation of the CRISPR/Cas12f1 system, particularly the CRISPR/Cas14a system belonging to the Cas14 family, is that the gene editing activity in the cell does not appear or is too low. In a prior document reported for the first time about the CRISPR/Cas14 system (Harrington et al., Programmed DNA destruction by miniature CRISPR-Cas14 enzymes, Science 362, 839-842 (2018)), prior researchers have found that the CRISPR/Cas14 system exhibits cleavage activity for single-stranded DNA in prokaryotic cells, but fails to exhibit cleavage activity for double-stranded DNA in the cell. In this regard, prior researchers believed that when considering that the CRISPRCas14 system is a system discovered in archaeons, the CRISPR/Cas system in the early stages of evolution shows only cleavage activity for single-stranded DNA, and then has acquired cleavage activity for double-stranded DNA (for example, the CRISPR/Cas9 system) while undergoing evolution. In another prior document (US 2020/0190494 A1), prior researchers have reported the fact that the CRISPR/Cas14 system exhibits cleavage activity for double-stranded DNA in eukaryotic cells, but the cleavage efficiency is 0.1% or less, which appears to be remarkably low. Therefore, according to the prior documents, the CRISPR/Cas12f1 system belonging to the Cas14 family does not show cleavage activity for double-stranded DNA in the cell, or even when the CRISPR/Cas12f1 system shows cleavage activity, the efficiency thereof is very low, so that the CRISPR/Cas12f1 system had a limitation in that it is difficult to actively utilize the CRISPR/Cas12f1 system in gene editing.

### U-rich tail sequence

### U-rich tail sequence - overview

An aspect of the present disclosure provides a U-rich tail sequence to improve the gene editing efficiency of the CRISPR/Cas12f1 system. The U-rich tail sequence is basically characterized by the presence of an abundant uridine, in which one or more uridines are included. In an embodiment, the U-rich tail sequence may include a sequence of 1 to 10 repeating uridines. The U-rich tail sequence may further include additional nucleotides in addition to uridine depending on the actual usage and expression environments of the engineered CRISPR/Cas12f1 system (for example, eukaryotic or prokaryotic internal environment). In an embodiment, the U-rich tail sequence may include a sequence in which one or more of UV, UUV, UUUV, and/or UUUUV are repeated. In this regard, V is any one of adenosine (A), cytidine (C), and guanosine (G). The U-rich tail sequence is characterized by being linked to 3' end of the crRNA sequence included in the CRISPR/Cas12f1 system. In the present disclosure, the U-rich tail sequence serves to increase the indel efficiency of the engineered CRISPR-Cas12f1 system for a target nucleic acid. In this regard, the target nucleic acid may be single-stranded DNA, double-stranded DNA, and/or RNA. The term "U-rich tail sequence" used herein refers to a RNA sequence including an abundant uridine, or a DNA sequence encoding thereof and is appropriately interpreted according to the context. In the present disclosure, the structure of the U-rich tail sequence and the effects thereof are described in detail, which will be further described in the following paragraphs.

### Background of introducing U-rich tail sequence - A related study on the CRISPR/Cpfl system

In the previous study, the present inventors have found that when the U-rich tail sequence is linked to the 3'-terminus of the crRNA sequence included in the CRISPR/Cpfl system, the nucleic acid cleavage efficiency of the CRISPR/Cpfl system is remarkably increased (Moon et al., Highly efficient genome editing by CRISPR-Cpfl using CRISPR RNA with a uridinylate-rich 3'-overhang. Nature Commun 9, 3651 (2018)). The present inventors paid attention to the fact that Cpf1 belongs to the Type V CRISPR/Cas system (hereinafter, a CRISPR/Cas12 system), and the CRISPR/Cas12 system shares several features. Therefore, we explored whether the gene editing efficiency of the CRISPR/Cas12 complex was increased by adding a continuous uridine sequence to the crRNA 3' end included in the CRISPR/Cas12 system.

### Background of introducing U-rich tail sequence - effective in CRISPR/Cas12f1 system

Through experiments, the present inventors have found that when the U-rich tail sequence is linked to 3' end of the crRNA of Cas12f1, particularly the CRISPR/Cas12f1 system belonging to the Cas14 family, gene editing efficiency is remarkably increased. Additionally, various configurations of U-rich tail sequence were investigated with respect to the effects on the indel efficiency, and an optimal structure for the U-rich tail sequence is provided in the present disclosure.

### Background of introducing U-rich tail sequence - not applicable to all CRISPR/Cas12 systems

However, as a result of introducing the U-rich tail sequence into other CRISPR/Cas12 systems, it was found that just belonging to the Type V CRISPR/Cas system does not enhance the gene editing efficiency of the CRISPR/Cas12 complex (see Experimental Example 00). It can be inferred that this brings different results for the U-rich tail sequence because even the same Type V group shows different aspects in terms of the presence of tracrRNA, the structure of a Cas protein, and the like. As far as the present inventors know, it has not been clarified by what function and mechanism the U-rich tail sequence added to 3'-terminus of the crRNA increases the nucleic acid cleavage efficiency in the CRISPR Cas system to date, and it is also difficult to determine whether the U-rich tail sequence added to 3'-terminus of the crRNA affects gene cleavage activity based on the structure of the guide RNA and/or Cas protein alone. Therefore, clarifying the role of the U-rich tail sequence and its effect on the CRISPR/Cas system will be a topic for future research. Hereinafter, the U-rich tail sequence introduced into the CRISPR/Cas12f1 system, particularly the system belonging to the Cas14 family will be described in more detail.

### U-rich tail sequence structure - continuous uridine sequence

One of the important points in designing the U-rich tail sequence is to abundantly include a sequence in which one or more uridines are continuous. Through experiments, the present inventors have found that the gene editing efficiency of the CRISPR/Cas12f1 complex was improved when the U-rich tail sequence, which is a sequence in which one or more uridines are continuous, was introduced into the CRISPR/Cas12f1 system. Therefore, the U-rich tail sequence provided in the present disclosure includes a sequence in which one or more uridines are continuous. In an embodiment, the U-rich tail sequence may include a sequence in which 1, 2, 3, 4, 5, 6, 7, 8, 9, 10, 11, 12, 13, 14, 15, 16, 17, 18, 19, or 20 uridines are contiguous.

### U-rich tail sequence structure - problems that may occur when a continuous uridine sequence is included alone

When the U-rich tail sequence includes a long continuous (for example, a sequence in which 5 or more uridines are continuous) uridine sequence, the following problems may occur when crRNA is expressed by introducing a vector or the like into the cell. When designing a vector expressing the aforementioned continuous uridine sequence, a continuous thymidine sequence corresponding to the continuous uridine sequence is inevitably included in the vector. In this regard, since some (for example, a U6 promoter, and the like) of the promoters used to express the vector in eukaryotic cells use a continuous thymidine sequence, for example, a T6 sequence as a termination signal, the continuous thymidine sequence corresponding to the continuous uridine sequence may be recognized as a termination signal. When the continuous uridine sequence includes 1 to 4 uridines, a sequence of 1 to 4 continuous thymidines is included in the vector, which does not act as a termination signal, so that there is no particular problem in expressing the U-rich tail sequence in which up to 4 contiguous thymidines are included. However, when the continuous uridine sequence includes 6 or more uridines, a T6 sequence is included in the vector, which serves as a termination signal, so that a U-rich tail sequence including 5 or more contiguous uridines is not likely to be expressed as intended. Therefore, in order to include abundant uridines in the U-rich tail sequence while using a promoter that recognizes the continuous thymidine sequence as a termination sequence, a structure in which 6 or more uridines are contiguous needs to be avoided.

### U-rich tail sequence structure - modified continuous uridine sequence

In order to solve the problem as described above, the U-rich tail sequence provided in the present disclosure may include a modified uridine repeat sequence in which one ribonucleoside (A, C, G) is incorporated in every 1 to 5 uridines. In an embodiment, the U-rich tail sequence may include a sequence in which one or more of UV, UUV, UUUV, UUUUV, and/or UUUUUV are repeated. In this regard, the V is one of adenosine (A), cytidine (C), and guanosine (G). The modified continuous uridine sequence is useful in designing a vector expressing an engineered crRNA.

### Example of U-rich tail sequence - Uₓ form

In an embodiment, the U-rich tail sequence may be represented by Uₓ. In an embodiment, x may be 1, 2, 3, 4, 5, 6, 7, 8, 9, 10, 11, 12, 13, 14, 15, 16, 17, 18, 19, or 20. In an embodiment, x may be an integer within the numerical range between two of these numbers. For example, x may be an integer between 1 to 6. In an embodiment, x may be an integer between 1 to 20. In an embodiment, x may be an integer of 20 or more.

### Example of U-rich tail sequence - (UₐN)ₙU_{b} form

In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 5, and n is an integer of 0 or more. In an embodiment, n may be an integer between 0 to 2. In an embodiment, b may be 1, 2, 3, 4, 5, 6, 7, 8, 9, or 10. In an embodiment, b may be an integer within the numerical range between two of these numbers. For example, b may be an integer between 1 to 6.

### Example embodiment of U-rich tail sequence - (UₐV)ₙ form

In an embodiment, the U-rich tail sequence may be represented by (UₐV)ₙ. In this regard, V is one of adenosine (A), cytidine (C), and guanosine (G). In this regard, a may be an integer between 1 to 4, and n may be an integer of 1 or more.

In an embodiment, the U-rich tail sequence may be represented by (UₐM)ₙ. In this regard, M is adenosine (A), or cytidine (C). In this regard, a may be an integer between 1 to 4, and n may be an integer of 1 or more.

In an embodiment, the U-rich tail sequence may be represented by (UaR)n. In this regard, R is adenosine (A), or guanosine (G). In this regard, a may be an integer between 1 to 4, and n may be an integer of 1 or more.

In an embodiment, the U-rich tail sequence may be represented by (UaS)n. In this regard, S is cytidine (C), or guanosine (G). In this regard, a may be an integer between 1 to 4, and n may be an integer of 1 or more.

In an embodiment, the U-rich tail sequence may be represented by (UₐA)ₙ. In this regard, a may be an integer between 1 to 4, and n may be an integer of 1 or more.

In an embodiment, the U-rich tail sequence may be represented by (UₐC)ₙ. In this regard, a may be an integer between 1 to 4, and n may be an integer of 1 or more.

In an embodiment, the U-rich tail sequence may be represented by (UₐG)ₙ. In this regard, a may be an integer between 1 to 4, and n may be an integer of 1 or more.

### Example of U-rich tail sequence - (UₐV)ₙU_{b} form

In an embodiment, the U-rich tail sequence may be represented by (UₐV)ₙU_{b}. In this regard, V is one of adenosine (A), cytidine (C), and guanosine (G). In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In an embodiment, b may be an integer within the numerical range between two of these numbers. For example, b may be an integer between 1 to 6. In an embodiment, b may be an integer between 1 to 20. In an embodiment, b may be an integer of 20 or more.

In an embodiment, the U-rich tail sequence may be represented by (UaM)n. In this regard, M is adenosine (A), or cytidine (C). In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In an embodiment, b may be an integer within the numerical range between two of these numbers. In an embodiment, b may be an integer between 1 to 6. In an embodiment, b may be an integer between 1 to 20. In an embodiment, b may be an integer of 20 or more.

In an embodiment, the U-rich tail sequence may be represented by (UₐR)ₙ. In this regard, R is adenosine (A), or guanosine (G). In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In an embodiment, b may be an integer within the numerical range between two of these numbers. In an embodiment, b may be an integer between 1 to 6. In an embodiment, b may be an integer between 1 to 20. In an embodiment, b may be an integer of 20 or more.

In an embodiment, the U-rich tail sequence may be represented by (UₐS)ₙ. In this regard, S is cytidine (C), or guanosine (G). In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In this regard, b may be an integer within the numerical range between two of these numbers. In an embodiment, b may be an integer between 1 to 6. In an embodiment, b may be an integer between 1 to 20. In an embodiment, b may be an integer of 20 or more.

In an embodiment, the U-rich tail sequence may be represented by (UaA)nUb. In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In this regard, b may be an integer within the numerical range between two of these numbers. In an embodiment, b may be an integer between 1 to 6. In an embodiment, b may be an integer between 1 to 20. In an embodiment, b may be an integer of 20 or more.

In an embodiment, the U-rich tail sequence may be represented by (UaC)nUb. In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In this regard, b may be an integer within the numerical range between two of these numbers. In an embodiment, b may be an integer between 1 to 6. In an embodiment, b may be an integer between 1 to 20. In an embodiment, b may be an integer of 20 or more.

In an embodiment, the U-rich tail sequence may be represented by (UₐG)ₙU_{b}. In this regard, a is an integer between 1 to 4, and n is an integer of 0 or more. In an embodiment, n may be 1 or 2. In an embodiment, b may be 1, 2, 3, 4, 5, 6, 7, 8, 9, 10, 11, 12, 13, 14, 15, 16, 17, 18, 19, or 20. In this regard, b may be an integer within the numerical range between two of these numbers. In an embodiment, b may be an integer between 1 to 6. In an embodiment, b may be an integer between 1 to 20. In an embodiment, b may be an integer of 20 or more.

### Example of U-rich tail sequence - a combination of Uₓ and (UₐV)ₙ

In an embodiment, the U-rich tail sequence may be in a combined form of a sequence represented by Uₓ and a sequence represented by (UₐV)ₙ. In an embodiment, the U-rich tail sequence may be represented by (U)ₙ₁-V1-(U)ₙ₂-V2-Uₓ. In this regard, V1 and V2 may each be one of adenosine (A), cytidine (C), and guanosine (G). In this regard, n1 and n2 may each be an integer between 1 to 4. In this regard, x may be an integer between 1 to 20.

### U-rich tail sequence - total length

In an embodiment, the length of the U-rich tail sequence may be 1nt, 2nt, 3nt, 4nt, 5nt, 6nt, 7nt, 8nt, 9nt, 10nt, 11nt, 12nt, 13nt, 14nt, 15nt, 16nt, 17nt, 18nt, 19nt, or 20nt. In an embodiment, the length of the U-rich tail sequence may be 20nt or more.

### U-rich tail sequence - example sequence

In an embodiment, the U-rich tail sequence may be U, UU, UUU, UUUU, UUUUU, UUUUUU, UUUAUUU, UUUAUUUAUUU (SEQ ID NO: 11), UUUUAU, UUUUAUU, UUUUAUUU, UUUUAUUUU, UUUUAUUUUU (SEQ ID NO: 4), UUUUAUUUUUU (SEQ ID NO: 5), UUUGUUU, UUUGUUUGUUU (SEQ ID NO: 29), UUUUGU, UUUUGUU, UUUUGUUU, UUUUGUUUU, UUUUGUUUUU (SEQ ID NO: 22), or UUUUGUUUUUU (SEQ ID NO: 23).

In an embodiment, the U-rich tail sequence may be any one selected from the group consisting of SEQ ID NOS: 3 to 57.

In an embodiment, the U-rich tail sequence may be UUUUUU, UUUUAUUUUUU, or UUUUGUUUUUU

### Advantage 1 of introducing U-rich tail sequence - increase in gene editing efficiency

When the U-rich tail sequence provided in the present disclosure is introduced into the CRISPR/Cas12f1 system, the gene editing efficiency of the resulting engineered CRISPR/Cas12f1 complex may be remarkably increased. Specifically, the engineered CRISPR/Cas12f1 complex exhibits the cleavage activity on double-stranded DNA which cannot be cleaved by the wild-type CRISPR/Cas12f1 complex in eukaryotic cells, or a substantial increase in the cleavage activity on double-stranded DNA in eukaryotic cells compared to the wild-type CRISPR/Cas12f1 complex. This was experimentally proven by the present inventors and is explained in detail in the part of the Experimental Examples of the present disclosure.

### Advantage 2 of introducing U-rich tail sequence - advantages of Cas12f1 can be used

As described above, the Cas12f1 protein has a remarkably smaller size than Cas9 and Cpf1, which are currently actively studied. Therefore, the lengths of the sequences encoding the Cas12f1 protein and guide RNA thereof are so short that the sequences can all be inserted into one AAV vector. Furthermore, even when a functional domain such as a base editor or a prime editor is added to the Cas12f1 protein, the sequences can all be included in one AAV vector. Since the AAV vector has high intracellular delivery efficiency and is approved as a gene therapy agent for humans, when the CRISPR/Cas system or a CRISPR/Cas system to which an appropriate functional domain is added is entirely included in one AAV vector, the AAV vector can be utilized for a wider gene editing technology. The total sequence of the CRISPR/Cas9 system is so long that the CRISPR/Cas9 system cannot be inserted into one AAV vector. In this aspect, the CRISPRCas12f1 system may be quite attractive as a gene editing tool. However, prior studies show that Cas12f1 proteins, particularly proteins belonging to the Cas14 family, cannot cleave double-stranded DNA or have significantly reduced cleavage efficiency. However, the present inventors have shown that the U-rich tail sequence can be introduced into the CRISPR/Cas12f1 system to significantly increase the gene editing efficiency of the CRISPR/Cas12f1 complex. In conclusion, since an engineered CRISPR/Cas12f1 complex into which the U-rich tail sequence provided in the present disclosure is introduced exhibits high nucleic acid cleavage efficiency while having the advantages of the aforementioned Cas12f1 protein, the engineered CRISPR/Cas12f1 complex can be applied to various gene editing techniques.

### Engineered CRISPR RNA (crRNA) for CRISPR/Cas12f1 system

### Engineered crRNA - overview

An aspect of the present disclosure provides an engineered CRISPR RNA for the CRISPR/Cas12f1 system (hereinafter, crRNA). The engineered crRNA is a crRNA in which a U-rich tail sequence is linked to 3'-terminus of the crRNA sequence of the CRISPR/Cas12f1 system. In this regard, the case where "CRISPR RNA" or "crRNA" is described in the present disclosure without any other modification is a concept distinguished from the engineered crRNA, and basically refers to a CRISPR RNA having a configuration which is present in nature, particularly the CRISPR RNA included in the CRISPR/Cas12f1 system. The U-rich tail sequence has the same features and structure as those described in the <U-rich tail sequence> section. The crRNA includes a CRISPR RNA repeat sequence and a spacer sequence. The spacer sequence is a sequence associated with a target sequence included in a target nucleic acid to be edited by the engineered CRISPR/Cas12f1 complex. The sequence of the engineered crRNA provided in the present disclosure includes a CRISPR RNA repeat sequence, a spacer sequence, and a U-rich tail sequence.

In an embodiment, in the sequence of the engineered crRNA, the CRISPR RNA repeat sequence, the spacer sequence, and the U-rich tail sequence may be linked sequentially in the 5'-to-3' direction.

In an embodiment, the spacer sequence may be a sequence which is complementary to the target sequence of the target nucleic acid.

In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4 inclusive, n is an integer selected from 0, 1, and 2, and b is an integer between 1 to 10 inclusive.

In an embodiment, the U-rich tail sequence may be represented by (UₐV)ₙU_{b}. In this regard, a, n, and b may be an integer, a may be 1 to 4, n may be 0 or more, and b may be 1 to 10. Additionally, an aspect of the present disclosure provides a DNA encoding the engineered crRNA. Hereinafter, the configuration of the engineered crRNA will be described in more detail.

### CRISPR RNA repeat sequence - definition

A CRISPR RNA repeat sequence is a sequence determined by the type of Cas12f1 protein of the CRISPR/Cas12f1 system, and refers to a sequence linked to 5' end of the spacer sequence. The CRISPR RNA repeat sequence may vary depending on the type of Cas12f1 protein included in the CRISPR/Cas12f1 system. The CRISPR RNA repeat sequence may interact with at least some of a tracrRNA of a CRISPR/Cas12f1 system, and may also interact with a Cas12f1 protein.

### CRISPR RNA repeat sequence - example of sequence

In an embodiment, the CRISPR RNA repeat sequence may be the sequence of SEQ ID NO: 58.

### CRISPR RNA repeat sequence - sequence can be modified

As used herein, "CRISPR RNA repeat sequence" refers to a CRISPR RNA repeat sequence commonly found in nature, but is not limited thereto, and may refer to a CRISPR RNA repeat sequence in which part or all of the CRISPR RNA repeat sequence found in nature is modified within a range not impairing the function of the CRISPR/Cas12f1 system.

In an embodiment, the CRISPR RNA repeat sequence may be a CRISPR RNA repeat sequence in which all or part of the wild-type CRISPR RNA is modified.

In an embodiment, the CRISPR RNA repeat sequence may be a sequence which is 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, or 50% identical or homologous to a wild-type CRISPR RNA repeat sequence. In an embodiment, the CRISPR RNA repeat sequence may be a sequence which is identical to a wild-type CRISPR RNA repeat sequence within the numerical range between two of these values. For example, the CRISPR RNA repeat sequence can be a sequence which is 90% to 100% identical to a wild-type CRISPR RNA repeat sequence. In an embodiment, the CRISPR RNA repeat sequence may be a sequence which is 60% to 80% identical to a wild-type CRISPR RNA repeat sequence.

In an embodiment, the wild-type CRISPR RNA repeat sequence may be the sequence of SEQ ID NO: 58.

### Spacer sequence - definition

The spacer sequence is a sequence which plays a central role in allowing the CRISPR/Cas complex to exhibit target-specific gene editing activity. As used herein, a spacer sequence refers to a spacer sequence in a crRNA (or an engineered crRNA) included in the CRISPR/Cas12f1 complex, unless otherwise specified. The spacer sequence is designed to locate to a target sequence included in the sequence of a target nucleic acid to be edited using the CRISPR/Cas12f1 complex. In other words, the engineered crRNA sequences provided in the present disclosure may have various spacer sequences depending on the target sequence. In this regard, the target nucleic acid may be single-stranded DNA, double-stranded DNA, or RNA.

### Spacer sequence - relationship between target nucleic acid and target sequence

The spacer sequence is a sequence which is complementary to a target sequence, and is linked to a 3'-terminus side of a CRISPR RNA repeat sequence. The spacer sequence is a sequence which is homologous to a protospacer sequence adjacent to a protospacer adjacent motif (PAM) sequence recognized by the Cas12f1 protein, and has a sequence in which thymidine of the protospacer sequence is substituted with uridine. In this regard, the target sequence and the protospacer sequence are determined in a sequence adjacent to the PAM sequence included in the target nucleic acid, and accordingly, the spacer sequence is determined.

In an embodiment, the spacer sequence portion of the crRNA may complementarily bind to the target nucleic acid. In an embodiment, the spacer sequence portion of the crRNA may complementarily bind to the target sequence portion of the target nucleic acid. In an embodiment, when the target nucleic acid is double-stranded DNA, the spacer sequence may be a sequence which is complementary to a target sequence included on a target strand of the double-stranded DNA. In an embodiment, when the target nucleic acid is double-stranded DNA, the spacer sequence may be a sequence which is homologous to a protospacer sequence included on a non-target strand of the double-stranded DNA. Specifically, the spacer sequence has the same base sequence as the protospacer sequence, but may have a sequence in which each of the thymidines (T) included in the base sequence is substituted with uracil (U). In an embodiment, the spacer sequence may be an RNA sequence corresponding to the DNA sequence of the protospacer.

### Spacer sequence - length of sequence

In an embodiment, the length of the spacer sequence may be 10nt, 11nt, 12nt, 13nt, 14nt, 15nt, 16nt, 17nt, 18nt, 19nt, 20nt, 21nt, 22nt, 23nt, 24nt, 25nt, 26nt, 27nt, 28nt, 29nt, 30nt, 31nt, 32nt, 33nt, 34nt, 35nt, 36nt, 37nt, 38nt, 39nt, or 40nt. In an embodiment, the spacer sequence may have a length within the numerical range between two of these numbers. For example, the length of the spacer sequence may be 17nt to 23nt. In an embodiment, the length of the spacer sequence may be 17nt to 30nt.

### U-rich tail sequence

The engineered crRNA is characterized in that the U-rich tail sequence is linked to the 3'-terminus portion of the spacer sequence included in the crRNA sequence. The U-rich tail sequence has the same configuration and features as those described in the <U-rich tail sequence> section.

### DNA encoding engineered crRNA

An aspect of the present disclosure provides a DNA encoding the engineered crRNA. In an embodiment, the DNA sequence may have a sequence in which all uridines are substituted with thymidine in the sequence of the engineered crRNA.

### Use of engineered crRNA

The engineered crRNA may constitute an engineered guide RNA for the CRISPR/Cas12f1 system along with a tracrRNA for Cas12f1. The engineered guide RNA including the engineered crRNA may bind to the Cas12f1 protein to form a CRISPR/Cas12f1 complex.

### Engineered guide RNA for CRISPR/Cas12f1 system

### Engineered guide RNA - overview

An aspect of the present disclosure provides an engineered guide RNA for the CRISPR/Cas12f1 system. The sequence of the engineered guide RNA includes a tracrRNA sequence, a crRNA sequence, and a U-rich tail sequence. In this regard, the U-rich tail sequence is linked to 3'-terminus of the crRNA sequence. In other words, the sequence of the engineered guide RNA includes a tracrRNA sequence and an engineered crRNA sequence. The crRNA includes a CRISPR RNA repeat sequence and a spacer sequence. In this regard, the CRISPR RNA repeat sequence and the spacer sequence have the same features and structure as each component described in the <Engineered CRISPR RNA for CRISPR/Cas12f1 system> section. The U-rich tail has the same features and structure as those described in the <U-rich tail sequence> section.

In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4, n is an integer selected from 0, 1, and 2, and b is an integer between 1 to 10 inclusive.

In an embodiment, the U-rich tail sequence may be represented by (UₐV)ₙU_{b}, and in this regard, a, n, and b are an integer, a may be between 1 to 4 inclusive, n may be 0 or more, and b may be between 1 to 10 inclusive.

At least a part of the tracrRNA included in the engineered guide RNA, and at least a part of the crRNA, may have a sequence which is complementary to each other to form a double-stranded RNA. Specifically, at least a part of the tracrRNA, and at least a part of the CRISPR RNA repeat sequence included in the crRNA sequence may be complementary to each other to form a double-stranded RNA. The engineered guide RNA may bind to the Cas12f1 protein to form a CRISPR/Cas12f1 complex, and recognize a target sequence which is complementary to a spacer sequence included in the crRNA sequence to allow the CRISRP/Cas12f1 complex to edit a target nucleic acid including the target sequence.

### tracrRNA - definition

A tracrRNA, along with crRNA, is an important component which constitutes the guide RNA of the CRISPR/Cas system. As used herein, the tracrRNA refers to a tracrRNA which constitutes the guide RNA of the CRISPR/Cas12f1 system, unless otherwise described. At least a part of the tracrRNA may complementarily bind to at least a part of crRNA to form a double-strand. More specifically, a partial sequence of the tracrRNA has a sequence which is complementary to all or part of the CRISPR RNA repeat sequence included in the crRNA.

### tracrRNA - complementarity of tracrRNA and CRISPR RNA repeat sequence

In an embodiment, the sequence of the tracrRNA may include a sequence which is 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% complementary to the CRISPR RNA repeat sequence. In an embodiment, the sequence of the tracrRNA may include a sequence which is complementary to the CRISPR RNA repeat sequence within the numerical range between two of these numbers. For example, the sequence of the tracrRNA may include a sequence which is 60% to 90% complementary to the CRISPR RNA repeat sequence. In an embodiment, the sequence of the tracrRNA may include a sequence which is 70% to 100% complementary to the CRISPR RNA repeat sequence.

### tracrRNA - number of mismatches in tracrRNA and CRISPR RNA repeat sequence

In an embodiment, the sequence of the tracrRNA may include a complementary sequence with 0, 1, 2, 3, 4, 5, 6, 7, 8, 9, 10, 11, 12, 13, 14, 15, 16, 17, 18, 19, or 20 mismatches with the CRISPR RNA repeat sequence. In an embodiment, the complementary sequence of the tracrRNA may have mismatches with the CRISPR RNA repeat sequence within the numerical range selected in the immediately preceding sentence. For example, the tracrRNA may include a complementary sequence with 0 to 8 mismatches with the CRISPR RNA repeat sequence. In an embodiment, the tracrRNA sequence may include a complementary sequence with 8 to 12 mismatches with the CRISPR RNA repeat sequence.

### tracrRNA - example of sequence

In an embodiment, the tracrRNA sequence may be the sequence of SEQ ID NO: 60.

### tracrRNA - sequence can be modified

As used herein, "tracrRNA" refers to a tracrRNA commonly found in nature, but is not limited thereto, and may refer to a tracrRNA in which part or all of the tracrRNA sequence found in nature is modified within a range not impairing the function of the CRISRP/Cas12f1 system.

In an embodiment, the tracrRNA sequence may be a tracrRNA sequence in which all or part of a wild type tracrRNA sequence is modified.

In an embodiment, the tracrRNA repeat sequence may be a sequence which is 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, or 50% identical or homologous to a wild-type tracrRNA repeat sequence. In an embodiment, the tracrRNA sequence may be a sequence which is identical to a wild-type tracrRNA sequence within the numerical range between two of these numbers. For example, the tracrRNA sequence may be a sequence which is 90% to 100% identical to a wild-type tracrRNA sequence. In an embodiment, the tracrRNA sequence may be a sequence which is 60% to 80% identical to a wild-type tracrRNA sequence.

In an embodiment, the wild-type tracrRNA sequence may be the sequence of SEQ ID NO: 60.

### Dual guide RNA - definition and structure

The engineered guide RNA provided in the present disclosure may be a dual guide RNA. This means that the tracrRNA and the crRNA form separate RNA molecules. A part of the tracrRNA and a part of the crRNA complementarily bind to each other to form a double strand. Specifically, in the dual guide RNA, a part including a 3'-terminus of the tracrRNA and a part including the CRISPR RNA repeat sequence of the crRNA may form a double strand.

In an embodiment, the engineered guide RNA may be a dual guide RNA. In an embodiment, when the engineered guide RNA is a dual guide RNA, the tracrRNA and the crRNA are separate RNA molecules.

### Dual guide RNA - example of sequence

In an embodiment, the tracrRNA may be the sequence of SEQ ID NO: 60. In an embodiment, the CRISPR RNA repeat sequence included in the crRNA may be the sequence of SEQ ID NO: 58. In an embodiment, at least a part of a trancrRNA having the sequence of SEQ ID NO: 60 and the CRISPR RNA repeat sequence having the sequence of SEQ ID NO: 58 may complementarily bind to each other to form a double strand.

### Single guide RNA - definition

In order to facilitate the formation of a guide RNA, the design of the tracrRNA and the crRNA into one molecule is referred to as a single guide RNA. The engineered guide RNA provided in the present disclosure may be a single guide RNA. When the engineered guide RNA is a single guide RNA, the single guide RNA is one molecule of RNA including all of the tracrRNA, the crRNA, and the U-rich tail. In an embodiment, when the engineered guide RNA is a single guide RNA, the sequence of the tracrRNA and the sequence of the crRNA may both be included in the sequence of one molecule of RNA.

### Single guide RNA - example of linker and sequence

In an embodiment, when the engineered guide RNA is a single guide RNA, the sequence of the engineered guide RNA further comprises a linker sequence, and the tracrRNA sequence and the crRNA sequence may be linked via the linker sequence. In an embodiment, the linker sequence may be 5'-gaaa-3'.

### Single guide RNA - structure

In the sequence of the single guide RNA, a tracrRNA sequence, a linker sequence, a crRNA sequence, and a U-rich tail sequence are linked sequentially in the 5'-to-3' direction. A part of the tracrRNA sequence and all or part of the CRISPR RNA repeat sequence included in the crRNA sequence have a sequence which is complementary to each other. Therefore, a part of the tracrRNA, and the CRISPR RNA repeat sequence part in the crRNA complementarily bind to each other to form a double-stranded RNA.

### Single guide RNA - example of sequence

The single guide RNA may have a sequence selected from the group consisting of SEQ ID NOS: 75 to 125 and 261 to 280.

### Scaffold sequence

When the sequences of the engineered guide RNA provided in the present disclosure are functionally divided, the sequences may be divided into 1) a sequence portion which interacts with a Cas12f1 protein to form the CRISPR/Cas12f1 complex, 2) a sequence portion which allows the CRISPR/Cas12f1 complex to find a target nucleic acid, and 3) a U-rich tail sequence portion. In this regard, the sequence portion which interacts with the Cas12f1 protein to form the CRISPR/Cas12f1 complex may be said to be a scaffold sequence. In this regard, the scaffold sequence collectively refers to a portion which interacts with the Cas12f1 protein, and is not limited to the RNA sequence of one molecule. Specifically, the scaffold sequence may include sequences of two or more molecules of RNA.

In an embodiment, when the engineered guide RNA is a dual guide RNA, the scaffold sequence may include a tracrRNA sequence and a CRISPR RNA repeat sequence included in a crRNA among the engineered guide RNA sequences. In an embodiment, the tracrRNA sequence may be a tracrRNA sequence in which all or part of the naturally occurring tracrRNA sequence is modified. In an embodiment, the CRISPR RNA repeat sequence may be a CRISPR RNA sequence in which all or part of a CRISPR RNA repeat sequence found in nature is modified.

In an embodiment, when the engineered guide RNA is a single guide RNA, the scaffold sequence may include the tracrRNA sequence, the linker sequence, and a CRISPR RNA repeat sequence included in the crRNA sequence. In an embodiment, the tracrRNA sequence may be a tracrRNA sequence in which all or part of the naturally occurring tracrRNA sequence is modified. In an embodiment, the CRISPR RNA repeat sequence may be a CRISPR RNA sequence in which all or part of a CRISPR RNA repeat sequence found in nature is modified.

In an embodiment, the scaffold sequence may be a sequence selected from the group consisting of SEQ ID NOS: 253 and 254. In an embodiment, the scaffold sequence may be a sequence which is 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, or 50% identical or homologous to the sequence selected from the group consisting of SEQ ID NOS: 253 and 254. In an embodiment, the scaffold sequence may be a sequence which is identical to the sequence selected from the group consisting of SEQ ID NOS: 253 and 254 within the numerical range between two of these numbers. For example, the scaffold sequence may be a sequence which is 90% to 100% identical to the scaffold sequence selected from the group consisting of SEQ ID NOS: 253 and 254. In an embodiment, the scaffold sequence may be a sequence which is 60% to 80% identical to the scaffold sequence selected from the group consisting of SEQ ID NOS: 253 and 254.

### Engineered CRISPR/Cas12f1 complex

### CRISPR/Cas12f1 complex - overview

An aspect of the present disclosure provides an engineered CRISPR/Cas12f1 complex. The engineered CRISPR/Cas12f1 complex comprises a Cas12f1 protein and an engineered guide RNA. In an embodiment, the Cas12f1 protein may be derived from the Cas14 family. The engineered guide RNA sequence includes a tracrRNA sequence, a crRNA sequence, and a U-rich tail sequence. In other words, the engineered guide RNA sequence includes a scaffold sequence, a spacer sequence, and a U-rich tail sequence. The U-rich tail sequence may be linked to 3'-terminus of the crRNA sequence. The U-rich tail has the same features and structure as those described in the <U-rich tail sequence> section.

In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4, n is an integer selected from 0, 1, and 2, and b is an integer between 1 to 10 inclusive.

In an embodiment, the U-rich tail sequence may be represented by (UₐV)ₙU_{b}, and in this regard, a, n, and b are an integer, a may be between 1 to 4, n may be 0 or more, and b may be between 1 to 10.

The scaffold sequence may interact with the Cas12f1 protein, and plays an important role when the engineered guide RNA and the Cas12f1 protein form a complex.

### Cas12f1 protein - overview

The engineered CRISPR/Cas12f1 complex may include a Cas12f1 protein. Basically, the Cas12f1 protein may be a wild-type Cas12f1 protein which is present in nature. The sequence encoding the Cas12f1 protein may be a human codon-optimized Cas12f1 sequence for the wild-type Cas12f1 protein. Further, the Cas12f1 protein may have the same function as a wild-type Cas12f1 protein which is present in nature. However, unless otherwise specified, as used herein, the "Cas12f1 protein" may refer to not only a wild-type or codon-optimized Cas12f1 protein but also a modified Cas12f1 protein or Cas12f1 fusion protein. In addition, the Cas12f1 protein may collectively refer to not only a Cas12f1 protein having the same function as a wild-type Cas12f1 protein, but also a Cas12f1 protein in which all or part of the function is modified, a Cas12f1 protein in which all or part of the function is lost, and/or a Cas12f1 protein to which an additional function is added. The meaning of the Cas12f1 protein may be appropriately interpreted depending on the context, and is interpreted in the broadest sense unless there are special cases. Hereinafter, the configuration or function of the Cas12f1 protein will be described in detail.

### Cas12f1 protein - wild-type Cas12f1 protein

The engineered CRISPR/Cas12f1 complex may include a Cas12f1 protein. In an embodiment, the Cas12f1 protein may be a wild-type Cas12f1 protein. In an embodiment, the Cas12f1 protein may be derived from the Cas14 family (Harrington et al., Programmed DNA destruction by miniature CRISPR-Cas14 enzymes, Science 362, 839-842 (2018)). In an embodiment, the Cas12f1 protein may be a Cas14a protein derived from uncultured archaeons (Harrington et al., Programmed DNA destruction by miniature CRISPR-Cas14 enzymes, Science 362, 839-842 (2018)). In an embodiment, the Cas12f1 protein may be a Cas14a1 protein.

### Cas12f1 protein - modified Cas12f1 protein

The engineered CRISPR/Cas12f1 complex may include a modified Cas12f1 protein. The modified Cas12f1 refers to a Cas12f1 in which at least some sequences of wild-type or codon-optimized Cas12f1 protein sequences are modified. The Cas12f1 protein modification may consist of individual amino acid units, and may consist of functional domain units of the protein. In an embodiment, the modification of the protein may be a modification in which one or more amino acids, peptides, polypeptides, proteins, and/or domains are individually substituted for, deleted from, and/or added to a wild-type or codon-optimized Cas12f1 protein sequence. In an embodiment, the Cas12f1 protein may be a Cas12f1 protein which one or more amino acids, peptides, and/or polypeptides in a RuvC domain included in a wild-type Cas12f1 protein are substituted for, deleted from, and added to.

### Cas12f1 protein - Cas12f1 fusion protein

The engineered CRISPR/Cas12f1 complex provided in the present disclosure may include a Cas12f1 fusion protein. In this regard, the Cas12f1 fusion protein refers to a protein in which a wild-type or modified Cas12f1 protein is fused with an additional amino acid, peptide, polypeptide, protein, or domain.

In an embodiment, a Cas12f1 protein may be a fusion of a wild-type Cas12f1 protein with a base editor and/or reverse transcriptase. In an embodiment, the base editor may be adenosine deaminase, and/or cytosine deaminase. In an embodiment, the reverse transcriptase may be a Moloney Murine Leukemia Virus (M-MLV) reverse transcriptase, or a variant thereof. In this regard, a Cas12f1 protein fused with the reverse transcriptase may function as a prime editor.

In an embodiment, a Cas12f1 protein may be a fusion of a wild-type Cas12f1 protein with various enzymes capable of participating in the intracellular gene expression process. In this regard, the Cas12f1 protein fused with the enzymes may bring about various quantitative and qualitative changes in gene expression in the cell. In an embodiment, the enzyme may be DNMT, TET, KRAB, DHAC, LSD, and/or p300.

### Cas12f1 protein - change in function

The Cas12f1 protein included in the engineered CRISPR/Cas12f1 complex provided in the present disclosure may have similar functions to the wild-type Cas12f1 protein. The Cas12f1 protein included in the engineered CRISPR/Cas12f1 complex provided in the present disclosure may be a Cas12f1 protein with a changed function when compared to the wild-type Cas12f1 protein. Specifically, the change may be a modification of all or part of the function, a loss of all or part of the function, or an addition of additional functions. In an embodiment, the Cas12f1 protein is not particularly limited as long as it has a modification applicable to the Cas protein of the CRISPR/Cas system by those skilled in the art. In this regard, the change may be made by a known technique. In an embodiment, the Cas12f1 protein may be modified to cleave only one strand of the double strands of a target nucleic acid. Furthermore, the Cas12f1 protein may be changed such that only one strand of the double strands of the target nucleic acid can be cleaved and base editing or prime editing can be performed on the uncleaved strand. In an embodiment, the Cas12f1 protein may be changed such that both of the double strands of the target nucleic acid cannot be cleaved. Furthermore, the Cas12f1 protein may be changed such that both of the double strands of the target nucleic acid cannot be cleaved, and base editing, prime editing, or gene expression regulation function can be performed on the target nucleic acid.

### Cas12f1 protein - example of other modifications

In an embodiment, the Cas12f1 protein may include a nuclear localization sequence (NLS), or a nuclear export sequence (NES). Specifically, the NLS may be any one of those exemplified in the NLS section in the <Definition of terms>, but is not limited thereto. In an embodiment, the Cas12f1 protein may include a tag. Specifically, the tag may be any one of those exemplified in the tag section in the <Definition of terms>, but is not limited thereto.

### Cas12f1 protein - PAM sequence

Two conditions are required for the CRISPR/Cas12f1 complex to cleave a target gene or target nucleic acid. First, there must be a base sequence having a certain length, which the Cas12f1 protein can recognize in the target gene or the target nucleic acid. Second, there must be a sequence capable of complementarily binding to a spacer sequence included in a guide RNA around the base sequence having a certain length. When the two conditions are satisfied to allow 1) the Cas12f1 protein recognize the base sequence having a certain length and 2) the spacer sequence portion complementarily bind to the sequence portion around the base sequence having a certain length, the target gene or the target nucleic acid is cleaved. In this regard, the base sequence having a certain length recognized by the Cas12f1 protein is called a protospacer adjacent motif (PAM) sequence. The PAM sequence is a unique sequence determined according to the Cas12f1 protein, and is accompanied by a constraint that the target sequence must be determined in the sequences adjacent to the PAM sequence, when the target sequence of the CRISPR Cas12f1 complex is determined.

### Cas12f1 protein - example of PAM sequence

In an embodiment, the PAM sequence of the Cas12f1 protein may be a T-rich sequence. In an embodiment, the PAM sequence of the Cas12f1 protein may be TTTN in the 5'-to-3' direction. In this regard, N is one of deoxythymidine (T), deoxyadenosine (A), deoxycytidine (C), or deoxyguanosine (G). In an embodiment, the PAM sequence of the Cas12f1 protein may be TTTA, TTTT, TTTC, or TTTG in the 5'-to-3' direction. In an embodiment, the PAM sequence of the Cas12f1 protein may be TTTA or TTTG in the 5'-to-3' direction. In an embodiment, the PAM sequence of the Cas12f1 protein may be different from a PAM sequence of a wild-type Cas12f1 protein.

### Cas12f1 protein - example sequence

In an embodiment, the Cas12f1 protein may have an amino acid sequence selected from the group consisting of SEQ ID NOS: 281, 317, 319, 321, and 323.

In an embodiment, the DNA sequence encoding the Cas12f1 protein may be a human codon-optimized sequence.

In an embodiment, the DNA sequence encoding the Cas12f1 protein may be a DNA sequence selected from the group consisting of SEQ ID NOS: 1, 2, 316, 318, 320, and 322.

### Engineered guide RNA

An engineered guide RNA constituting the CRISPR/Cas12f1 complex provided in the present disclosure have the same features and structure as those described in the <Engineered guide RNA for CRISPR/Cas12f1 system> section.

### CRISPR/Cas12f1 complex - example of configuration

In an embodiment, the Cas12f1 protein may have an amino acid sequence of SEQ ID NO: 281, the engineered guide RNA may have an amino acid sequence selected from the group consisting of SEQ ID NOS: 75 to 125, and the Cas12f1 protein and the engineered guide RNA may bind to each other to form a CRISPR/Cas12f1 complex.

In an embodiment, the Cas12f1 protein may have an amino acid sequence selected from the group consisting of SEQ ID NOS: 317, 319, 321, and 323, the engineered guide RNA may have a sequence selected from the group consisting of SEQ ID NOS: 75 to 125 and 261 to 280, and the CRISPR/Cas 12f1 complex formed by binding the Cas12f1 protein and the engineered guide RNA may have a base editing function.

### Vector for expressing CRISPR/Cas12f1 system

### Overview

An aspect of the present disclosure provides a vector for expressing the components of a CRISPR/Cas12f1 system. The vector is constructed to express the Cas12f1 protein and/or the engineered guide RNA. The sequence of the vector may include a nucleic acid sequence encoding one of the components of the CRISPR/Cas12f1 system, or may include a nucleic acid sequence encoding two or more components. The sequence of the vector includes a nucleic acid sequence encoding the Cas12f1 protein and/or a nucleic acid sequence encoding the engineered guide RNA. The sequence of the vector includes one or more promoter sequences. The promoter is operably linked to a nucleic acid sequence encoding the Cas12f1 protein and/or a nucleic acid sequence encoding the engineered guide RNA to promote transcription of the nucleic acid sequence in the cell. The Cas12f1 protein has the same features and configuration as those of the Cas12f1 protein, the modified Cas12f1 protein, or the Cas12f1 fusion protein described in the <Engineered CRISPR/Cas12f1 complex> section. The engineered guide RNA has the same features and configuration as those of the engineered guide RNA described in the <Engineered guide RNA for CRISPR/Cas12f1 system> section.

The sequence of the vector may include a nucleic acid sequence encoding the Cas12f1 protein and a nucleic acid sequence encoding the engineered guide RNA. In an embodiment, the sequence of the vector may include a first sequence including a nucleic acid sequence encoding the Cas12f1 protein and a second sequence including a nucleic acid sequence encoding the engineered guide RNA. The sequence of the vector includes a promoter sequence for expressing the nucleic acid sequence encoding the Cas12f1 protein in the cell, and a promoter sequence for expressing the nucleic acid sequence encoding the engineered guide RNA in the cell, and the promoters are operably linked to each expression target. In an embodiment, the sequence of the vector may include a first promoter sequence operably linked to the first sequence, and a second promoter sequence operably linked to the second sequence.

The sequence of the vector may include a nucleic acid sequence encoding the Cas12f1 protein and a nucleic acid sequence encoding two or more different engineered guide RNAs. In an embodiment, the sequence of the vector may include a first sequence including a nucleic acid sequence encoding the Cas12f1 protein, a second sequence including a nucleic acid sequence encoding the engineered guide RNA, and a third sequence including a nucleic acid sequence encoding the second engineered guide RNA. Furthermore, the sequence of the vector may include a first promoter sequence operably linked to the first sequence, a second promoter sequence operably linked to the second sequence, and a third promoter sequence operably linked to the third sequence.

### Expression target - Cas12f1 protein

The vector may be constructed to express a Cas12f1 protein. In this regard, the Cas12f1 protein has the same configuration and features as those described in the <Engineered CRISPR/Cas12f1 complex> section. In an embodiment, the vector may be constructed to express a wild-type Cas12f1 protein. In this regard, the wild-type Cas12f1 protein may be Cas14a1. In an embodiment, the vector may be constructed to express a Cas12f1 protein which has been changed to cleave only one strand of the double strands of the target nucleic acid. Furthermore, the changed Cas12f1 protein may be changed such that only one strand of the double strands of the target nucleic acid can be cleaved and base editing or prime editing can be performed on the uncleaved strand. In an embodiment, the Cas12f1 protein may have been changed such that both of the double strands of the target nucleic acid cannot be cleaved. Furthermore, the Cas12f1 protein may be changed such that both of the double strands of the target nucleic acid cannot be cleaved, and base editing, prime editing, or gene expression regulation function can be performed on the target nucleic acid.

### Expression target - engineered guide RNA

The vector may be constructed to express an engineered guide RNA. The engineered guide RNA has the same features and configuration as those of the engineered guide RNA described in the <Engineered guide RNA for CRISPR/Cas12f1 system> section. In an embodiment, the vector may be constructed to express an engineered guide RNA. In an embodiment, the engineered guide RNA sequence may include a scaffold sequence, a spacer sequence, and a U-rich tail sequence. In an embodiment, the engineered guide RNA sequence may include a tracrRNA sequence, a crRNA sequence, and a U-rich tail sequence. In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4 inclusive, n is an integer selected from 0, 1, and 2, and b is an integer between 1 to 10 inclusive. In an embodiment, the U-rich tail sequence may be represented by (UₐV)ₙU_{b}. In this regard, a, n, and b may be an integer, a may be 1 to 4, n may be 0 or more, and b may be 1 to 10.

The vector may be constructed to express two or more engineered guide RNAs. In an embodiment, the vector may be constructed to express a first engineered guide RNA and a second engineered guide RNA. In an embodiment, the first engineered guide RNA sequence may include a first scaffold sequence, a first spacer sequence, and a first U-rich tail sequence, and the second engineered guide RNA sequence may include a second scaffold sequence, a second spacer sequence, and a second U-rich tail sequence.

### Expression target - additional component

The vector may be constructed to express an additional component such as NLS and a tag protein in addition to the above-described expression targets. In an embodiment, the additional component may be expressed independently from the Cas12f1, the modified Cas12f1, and/or the engineered guide RNA. In an embodiment, the additional component may be expressed while being linked to the Cas12f1, the modified Cas12f1, and/or the engineered guide RNA. In this regard, the additional component may be a component which is generally expressed when the CRISPR/Cas system is intended to be expressed, and a known technique may be referenced. The additional component may be one or more of the components described in the <Related Art - design of CRISPR/Cas system expression vector> section.

### Vector component - Cas12f1 protein expression sequence

The vector sequence may include a nucleic acid sequence encoding the Cas12f1 protein. In this regard, the Cas12f1 protein has the same configuration and features as those described in the <Engineered CRISPR/Cas12f1 complex> section. In an embodiment, the sequence of the vector may include a sequence encoding a wild-type Cas12f1 protein. In this regard, the wild-type Cas12f1 protein may be Cas14a1. In an embodiment, the sequence of the vector may include a human codon-optimized nucleic acid sequence encoding a Cas12f1 protein. In this regard, the human codon-optimized nucleic acid sequence encoding the Cas12f1 protein may be a human codon-optimized nucleic acid sequence encoding a Cas14a1 protein. In an embodiment, the sequence of the vector may be a sequence encoding a Cas12f1 protein or a Cas12f1 fusion protein. In an embodiment, the sequence of the vector may include a sequence encoding a Cas12f1 protein changed such that only one strand of the double strands of the target nucleic acid can be cleaved and base editing or prime editing can be performed on the uncleaved strand. In an embodiment, the Cas12f1 protein may include a sequence encoding a Cas12f1 protein changed such that both of the double strands of the target nucleic acid cannot be cleaved, and base editing, prime editing, or gene expression regulation function can be performed on the target nucleic acid.

### Vector component - engineered guide RNA expression sequence

In an embodiment, the sequence of the vector may include a sequence encoding an engineered guide RNA. In an embodiment, the sequence of the engineered guide RNA may include a scaffold sequence, a spacer sequence, and a U-rich tail sequence. In an embodiment, the sequence of the engineered guide RNA sequence may include a tracrRNA sequence, a crRNA sequence, and a U-rich tail sequence. In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4 inclusive, n is an integer selected from 0, 1, and 2, and b is an integer between 1 to 10 inclusive. In an embodiment, the U-rich tail sequence may be represented by (UₐV)ₙU_{b}. In this regard, a, n, and b may be an integer, a may be 1 to 4, n may be 0 or more, and b may be 1 to 10.

In an embodiment, the sequence of the vector may include a sequence encoding two or more different engineered guide RNAs. In an embodiment, the sequence of the vector may include a sequence encoding a first engineered guide RNA, and a sequence encoding a second engineered guide RNA. In this regard, a spacer sequence included in the first engineered guide RNA sequence may be different from a spacer sequence included in the second engineered guide RNA sequence. Furthermore, a U-rich tail sequence included in the first engineered guide RNA sequence may be different from a U-rich tail sequence included in the second engineered guide RNA sequence. In an embodiment, the first engineered guide RNA sequence may include a first scaffold sequence, a first spacer sequence, and a first U-rich tail sequence, and the second engineered guide RNA sequence may include a second scaffold sequence, a second spacer sequence, and a second U-rich tail sequence.

### Vector component - promoter sequence

The vector sequence includes a promoter sequence operably linked to a sequence encoding each component. Specifically, the promoter sequence may be one of the promoters disclosed in the promoter portion in the <Related Art - design of CRISPR/Cas system expression vector> section, but is not limited thereto.

In an embodiment, the vector sequence may include a sequence encoding a Cas12f1 protein, and a promoter sequence. In this regard, the promoter sequence is operably linked to a sequence encoding the Cas12f1 protein. In an embodiment, the vector sequence may include a sequence encoding an engineered guide RNA, and a promoter sequence. In this regard, the promoter sequence is operably linked to a sequence encoding the engineered guide RNA. In an embodiment, the vector sequence may include a sequence encoding a Cas12f1 protein, a sequence encoding an engineered guide RNA, and a promoter sequence. In this regard, the promoter sequence is operably linked to a sequence encoding the Cas12f1 protein and a sequence encoding the engineered guide RNA, and a transcription factor activated by the promoter sequence expresses the Cas12f1 protein and the engineered guide RNA.

### Vector configuration - two or more promoter sequences can be included

In an embodiment, the vector sequence may include a first sequence encoding a Cas12f1 protein, a first promoter sequence, a second sequence encoding an engineered guide RNA, and a second promoter sequence. In this regard, the first promoter sequence is operably linked to the first sequence, the second promoter sequence is operably linked to the second sequence, transcription of the first sequence is induced by the first promoter sequence, and transcription of the second sequence is induced by the second promoter sequence. In this regard, the first promoter and the second promoter may be the same type of promoter. In this regard, the first promoter and the second promoter may be different types of promoters.

In an embodiment, the vector sequence may include a first sequence encoding a Cas12f1 protein, a first promoter sequence, a second sequence encoding a first engineered guide RNA, a third sequence including a sequence that encodes a second engineered guide RNA, and a third promoter sequence. In this regard, the first promoter sequence is operably linked to the first sequence, the second promoter sequence is operably linked to the second sequence, the third promoter sequence is operably linked to the third sequence, transcription of the first sequence is induced by the first promoter sequence, transcription of the second sequence is induced by the second promoter sequence, and transcription of the third sequence is induced by the third promoter sequence. In this regard, the second promoter and the third promoter may be the same type of promoter. Specifically, the second promoter sequence and the third promoter sequence may be a U6 promoter sequence, but are not limited thereto. In this regard, the second promoter and the third promoter may be different types of promoters. Specifically, the second promoter sequence and the third promoter sequence may be a U6 promoter sequence and a HI promoter sequence, respectively, but are not limited thereto.

### Vector component - termination signal

The vector may include a termination signal operably linked to the promoter sequence. In this regard, the termination signal may be one of the termination signals disclosed in the termination signal portion in the <Related Art - design of CRISPR/Cas system expression vector> section, but is not limited thereto. The termination signal may vary depending on the type of promoter sequence. In an embodiment, when the vector sequence includes a U6 promoter sequence, a continuous thymidine sequence operably linked to the U6 promoter sequence may act as a termination signal. In an embodiment, the thymidine sequence may be a sequence in which five or more thymidines are continuously linked. In an embodiment, when the vector sequence includes a HI promoter sequence, a continuous thymidine sequence operably linked to the HI promoter sequence may act as a termination signal. In an embodiment, the thymidine sequence may be a sequence in which five or more thymidines are continuously linked.

### Vector component - relationship between sequence encoding U-rich tail and termination signal

The sequence of the engineered guide RNAs provided in the present disclosure includes a U-rich tail sequence at 3'-terminus thereof. Therefore, the sequence encoding the engineered guide RNA will include a T-rich sequence corresponding to the U-rich tail sequence at 3'-terminus thereof. As described above, since some promoter sequences recognize a continuous thymidine sequence, for example, a sequence in which five or more thymidines are continuously linked as a termination signal, the T-rich sequence may be recognized as a termination signal in some cases. In other words, when the vector sequence according to the present disclosure includes a sequence encoding an engineered guide RNA, the sequence encoding the U-rich tail sequence included in the engineered guide RNA sequence may be used as a termination signal. In an embodiment, when the vector sequence a U6 or HI promoter sequence, and includes a sequence encoding an engineered guide RNA operably linked to the U6 or HI promoter sequence, a sequence portion encoding a U-rich tail sequence included in the engineered guide RNA sequence may be recognized as a termination signal. In this regard, the U-rich tail sequence includes a sequence in which five or more uridines are continuously linked.

### Vector component - other components

The vector sequence may include a component required depending on the purpose, in addition to the configuration. In an embodiment, the vector sequence may include a regulatory/control component sequence and/or an additional component sequence. In an embodiment, the additional component may be added for the purpose of distinguishing transfected cells from non-transfected cells. In this regard, the regulatory/control component sequence and the additional component may be one of those disclosed in the <Related Art - design of CRISPR/Cas system expression vector>, but is not limited thereto.

### Type of vector - viral vector

The vector may be a viral vector. In an embodiment, the viral vector may be one or more selected from the group consisting of a retrovirus, a lentivirus, an adenovirus, an adeno-associated virus (AAV), a vaccinia virus, a poxvirus and a herpes simplex virus. In an embodiment, the viral vector may be adeno-associated virus (AAV).

### Type of vector - non-viral vector

The vector may be a non-viral vector. In an embodiment, the non-viral vector may be one or more selected from the group consisting of a plasmid, a phage, naked DNA, a DNA complex, and mRNA. In an embodiment, the plasmid may be selected from the group consisting of the pcDNA series, pSC101, pGV1106, pACYC177, ColE1, pKT230, pME290, pBR322, pUC8/9, pUC6, pBD9, pHC79, pIJ61, pLAFR1, pHV14, the pGEX series, the pET series, and pUC19. In an embodiment, the phage may be selected from the group consisting of λgt4λB, λ-Charon, λΔz1, and M13. In an embodiment, the vector may be a PCR amplicon.

### Vector form - circular or linear vector

The vector may be in a circular or linear form. When the vector is a linear vector, RNA transcription is terminated at a 3'-terminus thereof, even though the linear vector sequence does not include a termination signal separately. In comparison, when the vector is a circular vector, RNA transcription is not terminated unless the circular vector sequence includes a termination signal separately. Therefore, when a circular vector is used as the vector, a termination signal corresponding to a transcription factor related to each promoter sequence must be included in order to express an intended target. In an embodiment, the vector may be a linear vector. In an embodiment, the vector may be a linear amplicon. In an embodiment, the vector may be a linear vector including a sequence selected from the group consisting of SEQ ID NOS: 1, 2, and 127 to 177. In an embodiment, the vector may be a circular vector. In an embodiment, the vector may be a circular vector including a sequence selected from the group consisting of SEQ ID NOS: 1, 2, and 127 to 177.

### Vector - example of sequence

In an embodiment, the vector sequence may include one or more sequences selected from the group consisting of SEQ ID NOS: 1, 2, and 127 to 177. In an embodiment, the vector sequence may include one or more sequences selected from the group consisting of SEQ ID NOS: 1, 2, 316, 318, 320, and 322.

### Chemical modification of nucleic acid

An aspect of the present disclosure provides a component including or consisting of an engineered crRNA or a nucleic acid encoding the engineered crRNA, an engineered guide RNA or a nucleic acid encoding the engineered guide RNA, and/or a nucleic acid such as a vector for expressing a component of a CRISPR/Cas12f1 system. In this regard, the "nucleic acid" used herein may be DNA or RNA present in nature, and may be a modified nucleic acid in which part or all of the nucleic acid is chemically modified. In an embodiment, the nucleic acid may be DNA and/or RNA present in nature. In an embodiment, the nucleic acid may be a nucleic acid in which one or more nucleotides are chemically modified. In this regard, the chemical modification includes all nucleic acid modifications known in the art. Specifically, the chemical modification may include all of the nucleic acid modifications described in (WO 2019089820 A1), but is not limited thereto.

### Gene editing method using engineered crRNA

### Overview

An aspect of the present disclosure provides a method for editing a target gene or a target nucleic acid in a target cell using an engineered crRNA. The target gene or the target nucleic acid includes a target sequence. The target nucleic acid may be single-stranded DNA, double-stranded DNA, and/or RNA. The gene editing method includes: delivering an engineered guide RNA, a Cas12f1 protein, or a nucleic acid encoding each of the engineered guide RNA and the Cas12f1 protein into a target cell including a target gene or a target nucleic acid. As a result, an engineered CRISPR/Cas12f1 complex is injected into the target cell, or the formation of the engineered CRISPR/Cas12f1 complex is induced, and a target gene is edited by the engineered CRISPR/Cas12f1 complex. The engineered guide RNA has the same features and structure as those described in the <Engineered guide RNA for CRISPR/Cas12f1 system> section. The Cas12f1 protein has the same features and configuration as the Cas12f1 protein and/or the modified Cas12f1 protein described in the <Engineered CRISPR/Cas12f1 complex> section.

In an embodiment, the gene editing method may include: delivering an engineered guide RNA or a nucleic acid encoding the engineered guide RNA, and a Cas12f1 protein or a nucleic acid encoding the Cas12f1 protein into a target cell. In this regard, the engineered guide RNA sequence includes a scaffold sequence, a spacer sequence, and a U-rich tail sequence. In this regard, the spacer sequence may complementarily bind to a target gene or a target nucleic acid included in the target cell. In an embodiment, the U-rich tail sequence may be represented by (UₐV)ₙU_{b}. In this regard, a, n, and b are an integer, a is 1 to 4, n is 0 or more, and b is 1 to 10 inclusive. In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4 inclusive, n is an integer selected from 0, 1, and 2, and b is an integer between 1 to 10 inclusive.

### Target cell

In an embodiment, the target cell may be a prokaryotic cell. In an embodiment, the target cell may be a eukaryotic cell. Specifically, the eukaryotic cell may be a plant cell, an animal cell, and/or a human cell, but is not limited thereto.

### Determination of target sequence

A target gene or nucleic acid and a target sequence to be edited using a CRISPR Cas12f1 complex may be determined in consideration of the purpose of gene editing, the target cell environment, the PAM sequence recognized by the Cas12f1 protein, and/or other variables. In this regard, the method is not particularly limited as long as a target sequence having an appropriate length can be determined, and a known technique may be utilized.

### Determination of spacer sequence according to target sequence

Once the target sequence is determined, the corresponding spacer sequence is designed. The spacer sequence is designed as a sequence capable of complementarily binding to the target sequence. In an embodiment, the spacer sequence is designed as a sequence capable of complementarily binding to the target gene. In an embodiment, the spacer sequence is designed so as to be capable of complementarily binding to the target nucleic acid. In an embodiment, the spacer sequence is designed as a sequence which is complementary to a target sequence included in a target strand sequence of the target nucleic acid. In an embodiment, the spacer sequence is designed as an RNA sequence corresponding to a DNA sequence of a protospacer included in a non-target strand sequence of the target nucleic acid. Specifically, the spacer sequence has the same base sequence as the protospacer sequence, but is designed as a sequence in which each of the thymidines included in the base sequence is substituted with uridine.

### Complementarity of target sequence and spacer sequence

In an embodiment, the spacer sequence may be a sequence which is 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% complementary to the target sequence. In an embodiment, the spacer sequence may be a sequence which is complementary to the target sequence within the numerical range selected in the immediately preceding sentence. For example, the spacer sequence may be a sequence which is 60% to 90% complementary to the target sequence. In an embodiment, the spacer sequence may be a sequence which is 90% to 100% complementary to the target sequence.

### Number of mismatches between target sequence and spacer sequence

In an embodiment, the spacer sequence may be a complementary sequence with 0, 1, 2, 3, 4, 5, 6, 7, 8, 9, or 10 mismatches with the target sequence. In an embodiment, the spacer sequence may have mismatches within the numerical range of two of these values. For example, the spacer sequence may have 1 to 5 mismatches with the target sequence. In an embodiment, the spacer sequence may have 6 to 10 mismatches with the target sequence.

### Use of CRISPR/Cas12f1 complex

The gene editing method provided in the present disclosure uses the fact that the engineered CRISPR/Cas12f1 complex has the activity of cleaving a gene or a nucleic acid in a target-specific manner. The engineered CRISPR/Cas12f1 complex has the same features and configuration as the engineered CRISPR/Cas12f1 complex described in the <Engineered CRISPR/Cas12f1 complex> section.

### Delivery of each component of CRISRP/Cas12f1 complex into cell

In the gene editing method provided in the present disclosure, it is assumed that a CRISPR/Cas12f1 complex engineered within the target cell is brought into contact with a target gene or nucleic acid. Therefore, in order to induce contact of the engineered CRISPR/Cas12f1 complex with the target gene or the target nucleic acid, the gene editing method includes: delivering each component of the engineered CRISPRCas12f1 complex into a target cell. In an embodiment, the gene editing method may include delivering an engineered guide RNA or a nucleic acid encoding the engineered guide RNA and a Cas12f1 protein or a nucleic acid encoding the Cas12f1 protein into a target cell. In an embodiment, the gene editing method may include delivering an engineered guide RNA and a Cas12f1 protein into a target cell. In an embodiment, the gene editing method may include delivering a nucleic acid encoding an engineered guide RNA and a Cas12f1 protein into a target cell. In an embodiment, the gene editing method may include delivering an engineered guide RNA and a nucleic acid encoding a Cas12f1 protein into a target cell. In an embodiment, the gene editing method may include delivering a nucleic acid encoding an engineered guide RNA and a nucleic acid encoding a Cas12f1 protein into a target cell. An engineered guide RNA or a nucleic acid encoding the engineered guide RNA and a Cas12f1 protein or a nucleic acid encoding the Cas12f1 protein may be delivered in various delivery forms into a target cell using various delivery methods.

### Delivery form - RNP

As a delivery form, ribonucleoprotein particles (RNPs) in which an engineered guide RNA and a Cas12f1 protein are combined may be used. In an embodiment, the gene editing method may include injecting a CRISPR/Cas12f1 complex in which an engineered guide RNA and a Cas12f1 protein are combined into a target cell.

### Delivery form - non-viral vector

As another delivery form, a non-viral vector including a nucleic acid sequence encoding an engineered guide RNA and a nucleic acid sequence encoding a Cas12f1 protein may be used. In an embodiment, the gene editing method may include injecting a non-viral vector including a nucleic acid sequence encoding an engineered guide RNA and a nucleic acid sequence encoding a Cas12f1 protein into a target cell. Specifically, the non-viral vector may be a plasmid, naked DNA, a DNA complex, or mRNA, but is not limited thereto. In an embodiment, the gene editing method may include injecting a first non-viral vector including a nucleic acid sequence encoding an engineered guide RNA and a second non-viral vector including a nucleic acid sequence encoding a Cas12f1 protein into a target cell. Specifically, the first non-viral vector and the second non-viral vector may be each one selected from the group consisting of a plasmid, naked DNA, a DNA complex, and mRNA, but are not limited thereto.

### Delivery form - viral vector

As still another delivery form, a viral vector including a nucleic acid sequence encoding an engineered guide RNA and a nucleic acid sequence encoding a Cas12f1 protein may be used. In an embodiment, the gene editing method may include: injecting a nucleic acid sequence encoding an engineered guide RNA and a nucleic acid sequence encoding a Cas12f1 protein into a target cell. Specifically, the viral vector may be one selected from the group consisting of a retrovirus, a lentivirus, an adenovirus, an adeno-associated virus (AAV), a vaccinia virus, a poxvirus, and a herpes simplex virus, but is not limited thereto. In an embodiment, the viral vector may be adeno-associated virus (AAV).

In an embodiment, the gene editing method may include: injecting a first viral vector including a nucleic acid sequence encoding an engineered guide RNA and a second viral vector including a nucleic acid sequence encoding a Cas12f1 protein into a target cell. Specifically, the first viral vector and the second viral vector may be each one selected from the group consisting of a retrovirus, a lentivirus, an adenovirus, an adeno-associated virus (AAV), a vaccinia virus, a poxvirus and a herpes simplex virus, but are not limited thereto.

### Delivery method - General delivery means

The delivery method is not particularly limited as long as it can deliver an engineered guide RNA or a nucleic acid encoding the engineered guide RNA, and a Cas12f1 protein or a nucleic acid encoding the Cas12f1 protein in an appropriate delivery form into a cell. In an embodiment, the delivery method may be electroporation, a gene gun method, ultrasonic perforation, magnetofection, and/or transient cell compression or squeezing.

### Delivery method - nanoparticles

The delivery method may be delivering at least one component included in the CRISPR/Cas12f1 system using nanoparticles. In this regard, the delivery method may be a known method which can be appropriately selected by those skilled in the art. For example, the nanoparticle delivery method may be the method disclosed in (WO 2019/089820A1), but is not limited thereto.

In an embodiment, the delivery method may be delivering a Cas12f1 protein or a nucleic acid encoding the Cas12f1 protein and/or an engineered guide RNA or a nucleic acid encoding the engineered guide RNA using nanoparticles. In an embodiment, the delivery method may be delivering a Cas12f1 protein or a nucleic acid encoding the Cas12f1 protein, a first engineered guide RNA or a nucleic acid encoding the first engineered guide RNA and/or a second engineered guide RNA or a nucleic acid encoding the second engineered guide RNA using nanoparticles. In this regard, the delivery method may be a cationic liposome method, a lithium acetate-DMSO method, lipid-mediated transfection, a calcium phosphate precipitation method, lipofection, polyethyleneimine (PEI)-mediated transfection, DEAE-dextran-mediated transfection, and/or nanoparticle-mediated nucleic acid delivery (see Panyam et., al Adv Drug Deliv Rev. 2012 Sep 13. pii: S0169-409X(12)00283-9. doi: 10.1016/j.addr.2012.09.023), but is not limited thereto. In this regard, the component of the CRISPR/Cas12f1 system may be in the form of a RNP, a non-viral vector, and/or a viral vector. For example, the component of the CRISPR/Cas12f1 system may be in the form of mRNA encoding each component, but is not limited thereto.

### Delivery form and method - possible combination

The gene editing method includes: delivering an engineered guide RNA or a nucleic acid encoding the engineered guide RNA, and a Cas12f1 protein or a nucleic acid encoding the Cas12f1 protein into a cell, and in this regard, the delivery form and/or delivery method of the components may be the same as or different from each other. In an embodiment, the gene editing method may include: delivering an engineered guide RNA or a nucleic acid encoding the engineered guide RNA in a first delivery form and delivering a Cas12f1 protein or a nucleic acid encoding the Cas12f1 protein in a second delivery form. In this regard, the first delivery form and the second delivery form may be each any one of the above-described delivery forms. In an embodiment, the gene editing method may include: delivering an engineered guide RNA or a nucleic acid encoding the engineered guide RNA by a first delivery method, and delivering a Cas12f1 protein or a nucleic acid encoding the Cas12f1 protein by a second delivery method. In this regard, the first delivery method and the second delivery method may be each any one of the above-described delivery methods.

### Delivery order

The gene editing method includes: delivering an engineered guide RNA or a nucleic acid encoding the engineered guide RNA, and a Cas12f1 protein or a nucleic acid encoding the Cas12f1 protein into a cell, and in this regard, the components may be simultaneously delivered into the cell, but may be sequentially delivered with a time interval there between.

In an embodiment, the gene editing method may include: simultaneously delivering an engineered guide RNA or a nucleic acid encoding the engineered guide RNA and a Cas12f1 protein or a nucleic acid encoding the Cas12f1 protein. In an embodiment, the gene editing method may include: delivering an engineered guide RNA or a nucleic acid encoding the engineered guide RNA into the cell, and then delivering a Cas12f1 protein or a nucleic acid encoding the Cas12f1 protein into the cell after a time interval. In an embodiment, the gene editing method may include: delivering a Cas12f1 protein or a nucleic acid encoding the Cas12f1 protein into the cell, and then delivering an engineered guide RNA into the cell after a time interval. In an embodiment, the gene editing method may include: delivering a nucleic acid encoding a Cas12f1 protein into the cell, and then delivering an engineered guide RNA in the cell after a time interval.

### Delivery of a plurality of engineered guide RNAs or nucleic acid encoding the plurality of engineered guide RNAs

The gene editing method provided in the present disclosure may include delivering a Cas12f1 protein or a nucleic acid encoding the Cas12f1 protein, and two or more engineered guide RNAs or a nucleic acid encoding the two or more engineered guide RNAs into a target cell. By the method, two or more CRISPR/Cas12f1 complexes targeting different sequences may be injected into a target cell, or may be formed in the target cell. As a result, two or more target genes or target nucleic acids included in the cell may be edited. In an embodiment, the gene editing method include: delivering a Cas12f1 protein or a nucleic acid encoding the Cas12f1 protein, a first engineered guide RNA or a nucleic acid encoding the first engineered guide RNA, and a second engineered guide RNA or a nucleic acid encoding the second engineered guide RNA into a target cell including a target gene or target nucleic acid. In this regard, each of the components may be delivered into the cell using one or more of the delivery forms and methods described above. In this regard, two or more components may be simultaneously delivered into the cell, or may be sequentially delivered into the cell with a time interval therebetween.

### Bringing CRISPR/Cas12f1 complex and target nucleic acid in contact with each other

In the gene editing method provided in the present disclosure, editing of a target gene or target nucleic acid is performed while the engineered CRISPR/Cas12f1 complex in the target cell is being brought into contact with the target gene or the target nucleic acid. Therefore, the gene editing method may include bringing an engineered CRISPR/Cas12f1 complex into contact in a target cell, or inducing the contact. In an embodiment, the gene editing method may include: bringing an engineered CRISPR/Cas12f1 complex into contact with a target nucleic acid in a target cell. In an embodiment, the gene editing method may include: inducing a contact of an engineered CRISPR/Cas12f1 complex with a target nucleic acid in a target cell. In this regard, the induction is not particularly limited as long as it is a method for bringing the engineered CRISPR/Cas12f1 complex into contact with the target nucleic acid in the cell. In an embodiment, the induction may include: delivering an engineered guide RNA or a nucleic acid encoding the engineered guide, and a Cas12f1 protein or a nucleic acid encoding the Cas12f1 protein into the cell.

### Gene editing results - indel

As a result of performing the gene editing method provided in the present disclosure, an indel may be generated in a target gene or target nucleic acid. In this regard, the indel may occur inside and/or outside a target sequence portion and/or a protospacer sequence portion. The indel refers to a mutation in a nucleotide sequence of a nucleic acid, in which a part of the nucleotide sequence is deleted, an arbitrary nucleotide is inserted, and/or the insertion and the deletion are combined from the pre-edited sequence. In general, the occurrence of an indel in the target gene or the target nucleic acid sequence inactivates the corresponding gene or nucleic acid. In an embodiment, as a result of performing the gene editing method, one or more nucleotides in the target gene or the target nucleic acid may be deleted and/or added.

### Gene editing results - base editing

As a result of performing the gene editing method provided in the present disclosure, base editing may occur within the target gene or the target nucleic acid. This means that unlike an indel, in which a target gene or any base in the target nucleic acid is deleted or added, one or more specific bases in the nucleic acid are changed as intended. In other words, base editing causes a predetermined point mutation at a specific position in the target gene or nucleic acid. In an embodiment, as a result of performing the gene editing method, one or more bases in the target gene or the target nucleic acid may be substituted with other bases.

### Gene editing results - insertion

As a result of performing the gene editing method provided in the present disclosure, a knock-in may occur in the target gene or the target nucleic acid. The knock-in means inserting an additional nucleic acid sequence into the target gene or the target nucleic acid sequence. In addition to the CRISPR/Cas12f1 complex, a donor including the additional nucleic acid sequence is further required for the knock-in to occur. When the CRISPR/Cas12f1 complex cleaves a target gene or target nucleic acid in the cell, repair of the cleaved target gene or target nucleic acid will occur. In this regard, the donor is involved in the repair process, so that the additional nucleic acid sequence is inserted into the target gene or the target nucleic acid. In an embodiment, the gene editing method may further include: delivering a donor into a target cell. In this regard, the donor includes an additional target nucleic acid, and the donor induces the insertion of the additional target nucleic acid into the target gene or the target nucleic acid. In this regard, when the donor is delivered into a target cell, the above-described delivery form and/or delivery method may be used.

### Gene editing results - deletion

As a result of performing the gene editing method provided in the present disclosure, all or part of the target gene or the target nucleic acid sequence may be deleted. The deletion means deleting a partial base sequence in the target gene or the target nucleic acid. In an embodiment, the gene editing method includes: introducing a Cas12f1 protein or a nucleic acid encoding the Cas12f1 protein, a first engineered guide RNA or a nucleic acid encoding the first engineered guide RNA, and a second engineered guide RNA or a nucleic acid encoding the second engineered guide RNA into a cell including a target gene or target nucleic acid. Accordingly, the deletion of the specific sequence portion in the target gene or the target nucleic acid occurs as a result of the gene editing.

### Example of gene editing method

In an embodiment, the gene editing method may include: delivering a CRISPR/Cas12f1 complex in the form of ribonucleoprotein particles which an engineered guide RNA and a Cas12f1 protein are combined into eukaryotic cells. In this regard, the delivery may be performed by electroporation or lipofection.

In an embodiment, the gene editing method may include: delivering a nucleic acid encoding an engineered guide RNA and a nucleic acid encoding a Cas12f1 protein into eukaryotic cells. In this regard, the delivery may be performed by electroporation or lipofection.

In an embodiment, the gene editing method may include: delivering an adeno-associated virus (AAV) vector including a nucleic acid sequence encoding an engineered guide RNA and a nucleic acid sequence encoding a Cas12f1 protein into eukaryotic cells.

In an embodiment, the gene editing method may include: delivering an adeno-associated virus (AAV) including a nucleic acid sequence encoding a first engineered guide RNA, a nucleic acid sequence encoding a second engineered guide RNA, and a nucleic acid sequence encoding a Cas12f1 protein into eukaryotic cells.

### Use of engineered crRNA

### Gene editing use of engineered crRNA

In an embodiment, the engineered crRNA may be used for gene editing of a target gene or nucleic acid including a target sequence.

In an embodiment, the engineered guide RNA may be used for gene editing of a target gene or nucleic acid including a target sequence.

In an embodiment, the engineered CRISPR/Cas12f1 complex may be used for gene editing of a target gene or nucleic acid including a target sequence.

In this regard, the engineered crRNA, the engineered guide RNA, and the engineered CRISPR/Cas12f1 complex have the same features and configuration as those described in each section.

In an embodiment, the use of gene editing of the target gene or nucleic acid may be used for performing the method described in the <Gene editing method using engineered crRNA> section.

In an embodiment, the use may include: delivering an engineered guide RNA or a nucleic acid encoding the engineered guide RNA, and a Cas12f1 protein or a nucleic acid encoding the Cas12f1 protein into a target cell. In this regard, the engineered guide RNA sequence includes a scaffold sequence, a spacer sequence, and a U-rich tail sequence. In this regard, the spacer sequence may complementarily bind to a target gene or a target nucleic acid included in the target cell. In an embodiment, the U-rich tail sequence may be represented by (UₐV)ₙU_{b}. In this regard, a, n, and b are an integer, a is 1 to 4 inclusive, n is 0 or more, and b is 1 to 10 inclusive. In an embodiment, the U-rich tail sequence may be represented by (UₐN)ₙU_{b}. In this regard, N is one of adenosine (A), uracil (U), cytidine (C), and guanosine (G). In this regard, a is an integer between 1 to 4 inclusive, n is an integer selected from 0, 1, and 2, and b is an integer between 1 to 10 inclusive.

In an embodiment, the use may include bringing an engineered CRISPR/Cas12f1 complex into contact with a target nucleic acid in a target cell.

In this regard, the engineered CRISPR/Cas12f1 complex has the same features and structure as those described in the <Engineered CRISPR/Cas12f1 complex> section.

In an embodiment, the use may include: inducing a contact of an engineered CRISPR/Cas12f1 complex with a target nucleic acid in a target cell. In this regard, the induction is not particularly limited as long as it is a method for bringing the engineered CRISPR/Cas12f1 complex into contact with the target nucleic acid in the cell. In an embodiment, the induction may include: delivering an engineered guide RNA or a nucleic acid encoding the engineered guide, and a Cas12f1 protein or a nucleic acid encoding the Cas12f1 protein into the cell.

### Use of engineered crRNA for preparing composition for gene editing

In an embodiment, an engineered crRNA may be used for preparing a composition for gene editing.

In an embodiment, an engineered guide RNA may be used for preparing a composition for gene editing.

In an embodiment, an engineered CRISPR/Cas12f1 complex may be used as a use for preparing a composition for gene editing.

In an embodiment, the composition for gene editing may include a Cas12f1 protein or a nucleic acid encoding the Cas12f1 protein, an engineered crRNA or a nucleic acid encoding the engineered crRNA, and a tracrRNA or a nucleic acid encoding the tracrRNA.

In an embodiment, the composition for gene editing may include a Cas12f1 protein or a nucleic acid encoding the Cas12f1 protein, and an engineered guide RNA or a nucleic acid encoding the engineered guide RNA.

In an embodiment, the composition for gene editing may include a CRISPR/Cas12f1 complex.

In an embodiment, the composition for gene editing may include a vector having a nucleic acid sequence encoding a Cas12f1 protein and a nucleic acid sequence encoding an engineered guide RNA.

### [Example Mode of the invention]

Hereinafter, the invention provided in the present disclosure will be described in more detail through Experimental Examples and Examples. These Examples are only for exemplifying the content disclosed by the present disclosure, and it will be obvious to those skilled in the art that the scope of the content disclosed by the present disclosure should not be interpreted as being limited by these Examples.

### Experimental Example 1 Preparation of experimental materials and experimental methods

### Experimental Example 1-1 Human codon-optimized Cas14a1 sequence

A Cas12f1 protein belonging to the Cas14 family (hereinafter, Cas14a1, Doudna et al., Programmed DNA destruction by miniature CRISPR-Cas14 enzymes, Science 362, 839-842 (2018)) was codon-optimized using a codon optimization program, and a sequence in which NLS sequence was added to the 5'- end and 3'-terminus was gene-synthesized to secure CDS. PCR amplification was performed using the synthesized gene as a template, and cloning was performed according to a desired cloning sequence for a vector having a promoter capable of expression in a eukaryotic system and a polyA signal sequence by the Gibson assembly method. The sequence of a plasmid vector obtained after cloning was completed was finally confirmed by the Sanger sequencing method. The amino acid sequence of the Cas14a1 protein and the human codon-optimized nucleic acid sequence encoding the Cas14a1 protein are shown in Table 1.

**[Table 1]**

| | Sequence information of NLS-Cas14a1-NLS protein | |
|---|---|---|
| Subject | Sequence information | SEQ ID NO |
| Cas14a1 (Amino acid) | PKKKRKVGIHGVPAAMAKNTITKTLKLRIVRPYNSAEVEKIVADEKNNRE KIALEKNKDKVKEACSKHLKVAAYCTTQVERNACLFCKARKLDDKFYQK LRGQFPDAVFWQEISEIFRQLQKQAAEIYNQSLIELYYEIFIKGKGIANASSV EHYLSDVCYTRAAELFKNAAIASGLRSKIKSNFRLKELKNMKSGLPTTKS DNFPIPLVKQKGGQYTGFEISNHNSDFIIKIPFGRWQVKKEIDKYRPWEKF DFEQVQKSPKPISLLLSTQRRKRNKGWSKDEGTEAEIKKVMNGDYQTSYI EVKRGSKIGEKSAWMLNLSIDVPKIDKGVDPSIIGGIDVGVKSPLVCAINN AFSRYSISDNDLFHFNKKMFARRRILLKKNRHKRAGHGAKNKLKPITILTE KSERFRKKLIERWACEIADFFIKNKVGTVQMENLESMKRKEDSYFNIRLR GFWPYAEMQNKIEFKLKQYGIEIRKVAPNNTSKTCSKCGHLNNYFNFEYR KKNKFPHFKCEKCNFKENADYNAALNISNPKLKSTKEEPKRPAATKKAGQ AKKKK | 281 |
| Cas14a1 (Human codon-optimize d nucleic acid) | ccaaagaagaagcggaaggtcggtatccacggagtcccagcagccATGGCCAAGAACACAATTA CAAAGACACTGAAGCTGAGGATCGTGAGACCATACAACAGCGCTGAG GTCGAGAAGATTGTGGCTGATGAAAAGAACAACAGGGAAAAGATCGC CCTCGAGAAGAACAAGGATAAGGTGAAGGAGGCCTGCTCTAAGCACC TGAAAGTGGCCGCCTACTGCACCACACAGGTGGAGAGGAACGCCTGT CTGTTTTGTAAAGCTCGGAAGCTGGATGATAAGTTTTACCAGAAGCTGC GGGGCCAGTTCCCCGATGCCGTCTTTTGGCAGGAGATTAGCGAGATCTT CAGACAGCTGCAGAAGCAGGCCGCCGAGATCTACAACCAGAGCCTGA TCGAGCTCTACTACGAGATCTTCATCAAGGGCAAGGGCATTGCCAACG CCTCCTCCGTGGAGCACTACCTGAGCGACGTGTGCTACACAAGAGCCG CCGAGCTCTTTAAGAACGCCGCTATCGCTTCCGGGCTGAGGAGCAAGA TTAAGAGTAACTTCCGGCTCAAGGAGCTGAAGAACATGAAGAGCGGCC TGCCCACTACAAAGAGCGACAACTTCCCAATTCCACTGGTGAAGCAGA AGGGGGGCCAGTACACAGGGTTCGAGATTTCCAACCACAACAGCGAC TTTATTATTAAGATCCCCTTTGGCAGGTGGCAGGTCAAGAAGGAGATTG ACAAGTACAGGCCCTGGGAGAAGTTTGATTTCGAGCAGGTGCAGAAG AGCCCCAAGCCTATTTCCCTGCTGCTGTCCACACAGCGGCGGAAGAGG AACAAGGGGTGGTCTAAGGATGAGGGGACCGAGGCCGAGATTAAGAA AGTGATGAACGGCGACTACCAGACAAGCTACATCGAGGTCAAGCGGG GCAGTAAGATTGGCGAGAAGAGCGCCTGGATGCTGAACCTGAGCATTG ACGTGCCAAAGATTGATAAGGGCGTGGATCCCAGCATCATCGGAGGGA TCGATGTGGGGGTCAAGAGCCCCCTCGTGTGCGCCATCAACAACGCCT TCAGCAGGTACAGCATCTCCGATAACGACCTGTTCCACTTTAACAAGAA GATGTTCGCCCGGCGGAGGATTTTGCTCAAGAAGAACCGGCACAAGCG GGCCGGACACGGGGCCAAGAACAAGCTCAAGCCCATCACTATCCTGAC CGAGAAGAGCGAGAGGTTCAGGAAGAAGCTCATCGAGAGATGGGCCT GCGAGATCGCCGATTTCTTTATTAAGAACAAGGTCGGAACAGTGCAGAT GGAGAACCTCGAGAGCATGAAGAGGAAGGAGGATTCCTACTTCAACAT TCGGCTGAGGGGGTTCTGGCCCTACGCTGAGATGCAGAACAAGATTGA GTTTAAGCTGAAGCAGTACGGGATTGAGATCCGGAAGGTGGCCCCCAA CAACACCAGCAAGACCTGCAGCAAGTGCGGGCACCTCAACAACTACT TCAACTTCGAGTACCGGAAGAAGAACAAGTTCCCACACTTCAAGTGCG AGAAGTGCAACTTTAAGGAGAACGCCGATTACAACGCCGCCCTGAACA TCAGCAACCCTAAGCTGAAGAGCACTAAGGAGGAGCCCaaaaggccggcggc cacgaaaaaggccggccaggcaaaaaagaaaaag | |

### Experimental Example 1-2 Preparation of plasmid vector

An oligonucleotide (Bionics) including a human codon-optimized Cas14a1 DNA sequence used in this experiment was synthesized. An oligonucleotide for the Cas14a1 protein sequence was cloned into a plasmid including a Chicken β-actin promoter, NLS at the 5' end and 3' end, and T2A-linked eGFP. A template DNA for a canonical guide RNA used in this experiment was synthesized (Twist Bioscience) and cloned into a pTwist Amp plasmid for replication. A template DNA for an engineered guide RNA was constructed using an enzyme cloning technique and cloned into a pTwist Amp plasmid for replication. By using the plasmid as a template if necessary, an amplicon of a guide RNA or engineered guide RNA was prepared using a U6-complementary forward primer and a protospacer sequence-complementary reverse primer. Further, the prepared amplicon was cloned into a T-blunt plasmid (Biofact) for replication, if necessary. In order to prepare a (engineered) dual guide RNA, oligonucleotides encoding a tracrRNA and a (engineered) crRNA were replicated within pSilencer 2.0 (ThermoFisher Scientific) using BamHI and HindIII restriction enzymes (New England Biolabs).

### Experimental Example 1-3 Preparation of viral vector

An adeno-associated virus (AAV) inverted terminal repeat vector including a guide RNA (or an engineered guide RNA) sequence and a Cas12f1 protein sequence linked to an enhanced green fluorescent protein (eGFP) through an NLS and a self-cleaving T2A sequence was prepared. The transcription of Cas12f1 and a guide RNA was promoted by a chicken β-actin and a U6 promoter, respectively. To prepare a rAAV2 vector, pAAV-ITR-sgRNA-Cas12f1, pAAVED29 and a helper plasmid were transfected into HEK293T cells. The transfected HEK293T cells were cultured in a DMEM medium containing 2% FBS. A recombinant pseudotyped AAV vector stock was produced using Polyplus-transfection (PEIpro) and PEI coprecipitation using triple-transfection for the plasmid at the same molar ratio. After being cultured for 72 hours, the cells were lysed and the lysate was purified by iodixanol (Sigma-Aldrich) stepgradient ultracentrifugation.

### Experimental Example 1-4 Preparation of guide RNA amplicon

In order to prepare a guide RNA and an engineered guide amplicon, PCR amplification was performed using a U6-complementary forward primer and a protospacer sequence-complementary reverse primer for a template DNA plasmid for a canonical guide RNA and an engineered guide RNA template DNA plasmid, using KAPA HiFi HotStart DNA polymerase (Roche) or Pfu DNA polymerase (Biofact). The PCR amplification product was purified using the Higene Gel&PCR purification system (Biofact) to obtain guide RNA and engineered guide RNA amplicons.

### Experimental Example 1-5 Preparation of guide RNA

One of the following methods was used to prepare a guide RNA (or an engineered guide RNA).

A guide RNA was prepared by chemically synthesizing a pre-designed guide RNA.

A PCR amplicon including a pre-designed guide RNA sequence and a T7 promoter sequence was prepared. In vitro transcription was performed using a NEB T7polymerase with the PCR amplicon as a template. After the resulting product obtained by performing the in vitro transcription was treated with NEB DNase I, the treated product was purified using a Monarch RNA Cleanup Kit (NEB), and then a guide RNA was obtained.

A plasmid vector including a pre-designed guide RNA sequence and a T7 promoter sequence was prepared by the Tblunt plasmid cloning method in Experimental Example 1-2. After the vector was purified by double-cutting both ends of a guide RNA sequence including a T7 promoter sequence, in vitro transcription was performed on the resulting product using NEB T7 polymerase. After the resulting product obtained by performing the in vitro transcription was treated with NEB DNase I, the treated product was purified using a Monarch RNA Cleanup Kit (NEB), and then a guide RNA was obtained.

### Experimental Example 1-6 Preparation of recombinant Cas12f1 protein

The human codon-optimized Cas14a1 DNA sequence used in this experiment was replicated in a pMAL-c2 plasmid vector. The plasmid vector was used for transformation of BL21 (DE3) E. coli cells. The transformed E. coli colonies were grown in LB broth at 37°C until the optical density reached 0.7. The transformed E. coli cells were cultured at 18°C overnight in the presence of 0.1 mM isopropylthio-β-D-galactoside. The cells were then centrifuged at 3,500 g for 30 minutes and collected. The cells were resuspended in 20 mm Tris-HCl (pH 7.6), 500 mM NaCl, 5 mM β-mercaptoethanol, 5% glycerol. The cells were lysed and the lysed cells were disrupted by sonication. After a sample including the disrupted cells was centrifuged at 15,000 g for 30 minutes, the supernatant was filtered through a 0.4-µm syringe filter (Millipore). The filtered supernatant was loaded onto a Ni²⁺ -affinity column using a FPLC purification system (ÄKT Aurora, GE Healthcare). The bound fractions were eluted at a gradient of 80-400 mM imidazole, 20 mM Tris-HCl (pH 7.5). The eluted protein was treated with TEV protease for 16 hours. The cleaved protein was purified in a Heparin column at a 0.15-1.6 M NaCl linear concentration gradient. A recombinant Cas12f1 protein purified in the Heparin column was dialyzed against 20 mM Tris pH 7.6, 150 mM NaCl, 5 mM β-mercaptoethanol, and 5% glycerol. The dialyzed proteins were purified by passing through an MBP column, and then re-purified in a monoS column (GE Healthcare) or Enrich S at a linear gradient of 0.5-1.2 M NaCl. The re-purified proteins were collected and dialyzed against 20 mm Tris pH 7.6,150 mM NaCl, 5 mM β-mercaptoethanol, and 5% glycerol to prepare a Cas12f1 protein. The concentration of the protein produced was quantified by Bradford quantification using bovine serum albumin as a standard and measured electrophoretically on a Coomassie blue-stained SDS-PAGE gel.

### Experimental Example 1-7 Preparation of RNP

Ribonucleoprotein particles (RNPs) were prepared by incubating the recombinant Cas12f1 protein prepared according to Experimental Example 1-6 and the guide RNA (or engineered guide RNA) prepared according to Experimental Example 1-5 at 300 nM and 900 nM, respectively at room temperature for 10 minutes.

### Experimental Example 1-8 Cell culture

HEK-293T cells (ATCC CRL-11269) were cultured in a DMEM medium under the conditions of 37°C and 5% CO₂ with the addition of 10% heat-inactivated FBS (Corning) and 1% penicillin/streptomycin.

### Experimental Example 1-9 Transfection of plasmid vector or PCR amplicon in cells

Cell transfection was performed by electroporation or lipofection. In the case of electroporation, 4×10⁵ HEK-293 T cells were transfected with 2 to 5 µg of each of a plasmid vector encoding the Cas12f1 protein prepared in Experimental Example 1-2 and DNA encoding a guide RNA (and an engineered guide RNA) using a Neon transfection system (Invitrogen). The electroporation was performed under the conditions of 1300 V, 10 mA, and 3 pulses. For lipofection, 3 to 15 µL of FuGene reagent (Promega) was mixed with 1 to 5 µg of a plasmid vector encoding a Cas12f1 protein and 0.3 to 2 µg of a PCR amplicon for 15 minutes. The mixture was added to 1 ml of a DMEM medium plated with 1×10⁶ cells on day 1 prior to transfection. The cells were cultured in the presence of the mixture for 72 to 96 hours. After the culture, the cells were collected and the genomic DNA of the cells was manually isolated using a PureHelixTM genomic DNA preparation kit (NanoHelix) or a Maxwell RSC Cultured cells DNA Kit (Promega).

### Experimental Example 1-10 Intracellular RNP transfection

The ribonucleoprotein particles (RNPs) prepared in Experimental Example 1-7 were transfected using the electroporation in Experimental Example 1-9, or the plasmid encoding the Cas12f1 protein prepared in Experimental Example 1-2 was transfected by the lipofection method in Experimental Example 1-9 on day 1 prior to the transfection of a guide RNA (and an engineered guide RNA) and the guide RNA (and the engineered guide RNA) prepared by Experimental Example 1-5 was transfected using the electroporation in Experimental Example 1-9 after one day.

### Experimental Example 1-11 Transfection of viral vector in cells

Human HEK293T cells were infected with rAAV2-Cas12f1-sgRNA at different multiplicities of infection (MOIs) of 1, 5, 10, 50, and 100 determined by quantitative PCR. The transfected HEK293T cells were cultured in a DMEM medium containing 2% FBS. At different time points, cells were collected to isolate genomic DNA.

### Experimental Example 1-12 Quantitative real-time PCR (quantitative rtPCR)

A guide RNA (or an engineered guide RNA) or a genomic DNA was each extracted from HEK293 T cells using RNeasy Miniprep kit (Qiagen) or Maxwell RSC miRNA Tissue Kit (Promega) or DNeasy Blood & Tissue Kit (Qiagen). To quantify the guide RNA, a RNA-specific primer was ligated and cDNA was synthesized using a crRNA-specific primer. The cDNA was used as a template for quantitative real-time PCR. Real-time PCR was analyzed using the KAPA SYBR FASTqPCR Master Mix (2X) Kit (KapaBiosystems).

### Experimental Example 1-13 Western blot for confirming Cas protein expression

A plasmid vector encoding the Cas protein prepared by Experimental Example 1-2 was prepared. In this regard, the plasmid further has a sequence (5'-TACCCATACGATGTTCCAGATTACGCTTATCCCTACGACGTGCCTGATTATG CATACCCATATGATGTCCCCGACTATGCC-3', SEQ ID NO: 315) encoding an HA tag such that the HA tag can be linked and expressed when the Cas protein is expressed in the cell. The HEK293T cells cultured in Experimental Example 1-8 were transfected with the plasmid vector by the method disclosed in Experimental Example 1-9. The HEK293T cells were inoculated into a DMEM medium containing penicillin/streptomycin and statically cultured in an incubator at 37°C and 5% CO2 incubator for 48 hours. Then, the HEK293T cells were diluted with PBS and then disrupted by sonication. After a sample containing disrupted cells was centrifuged at 15,000 g and 4°C for 10 minutes, the supernatant was quantified by the Bradford quantification method. It was tested by western blot whether a quantified HA antibody was expressed in vivo.

### Experimental Example 1-14 Indel efficiency analysis

Among the genomic DNA isolated from HEK-293T cells, a region containing a protospacer was subjected to PCR using a target-specific primer in the presence of KAPA HiFi HotStart DNA polymerase (Roche). The amplification method followed the manufacturer's instructions. A PCR amplicon, which is the resulting product of the amplification including Illumina TruSeq HT dual indexes, was subjected to 150-bp paired end sequencing using Illumina iSeq 100. An indel frequency was calculated using MAUND.MAUND is provided at https://github.com/ibscge/maund.

### Experimental Example 1-15 Analysis of T7 endonuclease I (T7E1)

A PCR product was obtained using BioFACTTM Lamp Pfu DNA polymerase. The PCR product (100 to 300 µg) was reacted with 10 units of T7E1 enzyme (New England Biolabs) in a 25 µg reaction mixture at 37°C for 30 minutes. The 20 µl reaction mixture was loaded directly onto a 10% arylamide gel and the cleaved PCR product was run in a TBE buffer system. The gel image was stained with an ethidium bromide solution, and then digitized using a Printgraph 2M gel imaging system (Atto). Gene editing efficiency was evaluated by analyzing the digitized result.

### Experimental Example 1-16 Statistical analysis

Statistical significance verification by a two-tailed Student's t-test was performed by Sigma Plot software (ver. 14.0). When a p-value less than 0.05 appears, it was considered statistically significant, and the p-value is illustrated in each drawing. In a box plot and a dot plot, the data points indicate the full range of values, and each box spans the interquartile range (25 to 75 percentile). The median and mean values are indicated by parallel lines of black and red, respectively. Error bars for all dot plot and bar plot data are illustrated using Sigmaplot (v. 41.0) and mean the standard deviation value of each data. The sample size was not pre-determined based on statistical methods.

### Experimental Example 2 Indel efficiency comparison 1 of engineered CRISPR/Cas14a1 system having U-rich tail

Experiments were conducted to evaluate the indel efficiency of an engineered CRISPR/Cas14a1 system having a U-rich tail sequence, using various gene sequences as a target nucleic acid. The protospacer sequence of the target nucleic acid and each PAM sequence are disclosed in Table 2.

**[Table 2]**

| | Target nucleic acid of Experimental Example 2 | |
|---|---|---|
| Target nucleic acid | Protospacer sequence (5' to 3') of target nucleic acid | PAM |
| RNF2 | CACGTCTCATATGCCCCTTG (SEQ ID NO: 62) | TTTA |
| DYtarget2 | CACACACACAGTGGGCTACC (SEQ ID NO: 63) | TTTG |
| DYtarget9 | GACGTGGGAGACACACACAC (SEQ ID NO: 64) | TTTA |
| DYtarget10 | CATCCCCAGGACACACACAC (SEQ ID NO: 65) | TTTG |
| DYtargetl3 | AGCACATGTGCACACACACA (SEQ ID NO: 66) | TTTG |

1) A plasmid vector including a human codon-optimized sequence encoding a Cas14a1 protein was prepared according to Experimental Example 1-2.
2) An amplicon capable of expressing a wild-type, or (an engineered) single guide RNA having a U-rich tail sequence was prepared according to Experimental Example 1-4. DNA sequences encoding the single guide RNA and primer sequences used to prepare an amplicon are disclosed in Tables 3 to 5.

**[Table 3]**

| DNA sequences encoding wild-type (WT) sgRNA used in Experimental Example 2 | | | | | |
|---|---|---|---|---|---|
| Label | Target nucleic acid | sgRNA common sequence (5' to 3') | Protospacer sequence (5' to 3') | U-rich tail sequence(3'to 5') | SEQ ID NO |
| WT | RNF2 | CTTCACTGATAAA GTGGAGAACCGC TTCACCAAAAGC TGTCCCTTAGGG GATTAGAACTTG AGTGAAGGTGGG CTGCTTGCATCAG | CACGTCTCATATGCCCCTT G | - | 127 |
| | DYtarget2 | | CACACACACAGTGGGCTA CC | | 129 |
| | DYtarget9 | CCTAATGTCGAG AAGTGCTTTCTTC GGAAAGTAACCC TCGAAACAAATT CATTTTTCCTCTC CAATTCTGCACA AgaaaGTTGCAGA ACCCGAATAGAC GAATGAAGGAAT GCAAC (SEQ ID NO: 126) | GACGTGGGAGACACACAC AC | | 156 |
| | DYtarget1 0 | | CATCCCCAGGACACACAC AC | | 158 |
| | DYtarget1 3 | | AGCACATGTGCACACACA CA | | 171 |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 4]**

| DNA sequences encoding engineered sgRNA used in Experimental Example 2 | | | | | |
|---|---|---|---|---|---|
| Label | Target nucleic | sgRNA common | Protospacer sequence | U-rich tail sequence (3' to 5') | SEQ ID NO |
| Engi neere d sgR NA | RNF2 | CTTCACTGATA AAGTGGAGAAC CGCTTCACCAA AAGCTGTCCCT TAGGGGATTAG AACTTGAGTGA AGGTGGGCTGC TTGCATCAGCC TAATGTCGAGA AGTGCTTTCTT CGGAAAGTAAC CCTCGAAACAA ATTCATTTTTCC TCTCCAATTCTG CACAAgaaaGTT GCAGAACCCGA ATAGACGAATG AAGGAATGCAA C (SEQ ID NO: 126) | CACGTCTCATATGCCCCTT G | TTTTATTTT TT | 128 |
| | DYtarget2 | | CACACACACAGTGGGCTA CC | | 142 |
| | DYtarget9 | | GACGTGGGAGACACACAC AC | | 157 |
| | DYtarget1 0 | | CATCCCCAGGACACACAC AC | | 159 |
| | DYtarget1 3 | | AGCACATGTGCACACACA CA | | 172 |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 5]**

| | Primer sequences used in Experimental Example 2 | | | |
|---|---|---|---|---|
| sgRNA | Target nucleic acid | Forward primer (5' to 3') | Reverse primer (5' to 3') | SEQ ID NO |
| W T | RNF2 | GAGGGCCTATTT CCCATGATT (SEQ ID NO: 183) | CAAGGGGCATATGAGACGTGGTTGCATT CCTTCATTCGTCTATTC | 185 |
| | DYtarget2 | | GGTAGCCCACTGTGTGTGTGGTTGCATT CCTTCATTCGTCTATTC | 187 |
| | DYtarget9 | | GTGTGTGTGTCTCCCACGTCGTTGCATTC CTTCATTCGTCTATTC | 214 |
| | DYtarget10 | | GTGTGTGTGTCCTGGGGATGGTTGCATT CCTTCATTCGTCTATTC | 216 |
| | DYtarget13 | | TGTGTGTGTGCACATGTGCTGTTGCATTC CTTCATTCGTCTATTC | 229 |
| E n gi n ee re d sg R N A | RNF2 | | aaaaaataaaa CAAGGGGCATATGAGACGTGGTTGCATT CCTTCATTCGTCTATTC | 186 |
| | DYtarget2 | | aaaaaataaaaGTAGCCCACTGTGTGTGGT TGCATTCCTTCATTCGTCTATTC | 200 |
| | DYtarget9 | | aaaaaataaaaTGTGTGTGTCTCCCACGTCGT TGCATTCCTTCATTCGTCTATTC | 215 |
| | DYtarget10 | | aaaaaataaaaTGTGTGTGTCCTGGGATGGT TGCATTCCTTCATTCGTCTATTC | 217 |
| | DYtarget13 | | aaaaaataaaaGTGTGTGTGCACATGTGCTGT TGCATTCCTTCATTCGTCTATTC | 230 |

3) HEK-293T cells cultured according to Experimental Example 1-8 were transfected with a plasmid vector including the Cas14a1 sequence and an amplicon capable of expressing the single guide RNA by the method disclosed in Experimental Example 1-9.

4) The indel efficiencies for the target nucleic acids of each example were analyzed by the methods disclosed in Experimental Examples 1-12 to 1-15. The indel efficiency analysis results are shown in the following Table 6.

**[Table 6]**

| | Indel efficiency analysis results of Experimental Example 2 | | | |
|---|---|---|---|---|
| Target nucleic acid | | Control | WT | Engineered sgRNA |
| RNF2 | | 0 | 0 | 0.029 |
| DYtarget2 | | 0 | 0.028 | 0.749 |
| DYtarget9 | | 0 | 0 | 0.238 |
| DYtarget10 | | 0.029 | 0.031 | 1.076 |
| DYtarget13 | | 0.529 | 0.526 | 1.176 |

In this regard, those transfected with only a plasmid vector including the Cas14a1 protein sequence without sgRNA were used as a control.

### Experimental Example 3 Indel efficiency comparison 2 of engineered CRISPR/Cas14a1 system having U-rich tail

Experiments were conducted to evaluate the indel efficiency of an engineered CRISPR/Cas14a1 system having a U-rich tail sequence, using the DYtarget2, DYtarget10, DYtarget13, and Intergenic-22 as a target nucleic acid. The protospacer sequence of the target nucleic acid and each PAM sequence are disclosed in Table 7.

**[Table 7]**

| Target nucleic acid sequences used in Experimental Example 3 | | |
|---|---|---|
| Target nucleic acid | Protospacer sequence (5' to 3') of target nucleic acid | PAM |
| Intergenic-22 | AGAACACATACCCCTGGGCC (SEQ ID NO: 67) | TTTA |
| DYtarget2 | CACACACACAGTGGGCTACC (SEQ ID NO: 63) | TTTG |
| DYtarget10 | CATCCCCAGGACACACACAC (SEQ ID NO: 65) | TTTG |
| DYtarget13 | AGCACATGTGCACACACACA (SEQ ID NO: 66) | TTTG |

1) A plasmid vector including a human codon-optimized Cas14a1 sequence was prepared according to Experimental Example 1-2.
2) An amplicon capable of expressing a wild-type, or (an engineered) single guide RNA having a U-rich tail sequence was prepared according to Experimental Example 1-2. DNA sequences encoding the single guide RNA and primer sequences used to prepare an amplicon are shown in Tables 8 and 9.

**[Table 8]**

| | DNA sequences encoding sgRNA of Experimental Example 3 | | | | | |
|---|---|---|---|---|---|---|
| Label | | Target nucleic acid | sgRNA common (5' to 3') | Protospacer sequence (5' to 3') | U-rich tail sequence (3' to 5') | SEQ ID NO |
| Inter22 | | Intergenic-22 | CTTCACTGAT AAAGTGGAG AACCGCTTC ACCAAAAGC TGTCCCTTAG GGGATTAGA ACTTGAGTG AAGGTGGGC TGCTTGCATC AGCCTAATGT CGAGAAGTG CTTTCTTCGG AAAGTAACC CTCGAAACA AATTCATTTT TCCTCTCCAA TTCTGCACAA gaaaGTTGCAG AACCCGAATA GACGAATGA AGGAATGCA AC (SEQ ID NO: 126) | AGAACACATACCCCTG GGCC | | 173 |
| Inter22-U6 | | Intergenic-22 | | AGAACACATACCCCTG GGCC | TTTTTT | 174 |
| Inter22-U4aU6 | | Intergenic-22 | | AGAACACATACCCCTG GGCC | TTTTATT TTTT | 175 |
| DYtarget2 | | DYtarget2 | | CACACACACAGTGGGC TACC | | 129 |
| DYtarget2U4aU6 | | DYtarget2 | | CACACACACAGTGGGC TACC | TTTTATT TTTT | 142 |
| DYtarget10 | | DYtarget10 | | CACACACACAGTGGGC TACC | | 158 |
| DYtarget10U4a U6 | | DYtarget10 | | CATCCCCAGGACACAC ACAC | TTTTATT TTTT | 159 |
| DYtarget13 | | DYtarget13 | | CACACACACAGTGGGC TACC | | 171 |
| DYtargetl3U4a U6 | | DYtarget13 | | AGCACATGTGCACACA CACA | TTTTATT TTTT | 172 |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 9]**

| | Primer sequences used in Experimental Example 3 | | | | |
|---|---|---|---|---|---|
| Label | | Target nucleic acid | Forward primer (5' to 3') | Reverse primer (5' to 3') | SEQ ID NO |
| Inter22 | | Intergenic-22 | GAGGGCC TATTTCCC ATGATT (SEQ ID NO: 183) | GGCCCAGGGGTATGTGTTCTGTTGCATTCCT | 231 |
| Inter22-U6 | | Intergenic-22 | | aaaaaaGGCCCAGGGGTATGTGTTCTGTTGCAT TCCT | 232 |
| Inter22-U4aU6 | | Intergenic-22 | | aaaaaataaaaGGCCCAGGGGTATGTGTTCTGTTG CATTCCT | 233 |
| DYtarget2 | | DYtarget2 | | GGTAGCCCACTGTGTGTGTGGTTGCATTCCT TCATTCGTCTATTC | 187 |
| DYtarget2 U4aU6 | | DYtarget2 | | aaaaaataaaaGGTAGCCCACTGTGTGTGTGGTTG CATTCCTTCATTCGTCTATTC | 200 |
| DYtarget10 | | DYtarget10 | | GTGTGTGTGTCCTGGGGATGGTTGCATTCCT TCATTCGTCTATTC | 216 |
| DYtarget10U4aU6 | | DYtarget10 | | aaaaataaaaGTGTGTGTGTCCTGGGGATGGTTGC ATTCCTTCATTCGTCaTATTC | 217 |
| DYtarget13 | | DYtarget13 | | TGTGTGTGTGCACATGTGCTGTTGCATTCCTT CATTCGTCTATTC | 229 |
| DYtarget13U4aU6 | | DYtarget13 | | aaaaaataaaaTGTGTGTGTGCACATGTGCTGTTG CATTCCTTCATTCGTCTATTC | 230 |

3) HEK-293T cells cultured according to Experimental Example 1-8 were transfected with a plasmid vector including the Cas14a1 sequence and an amplicon of the single guide RNA by the method disclosed in Experimental Example 1-9.

4) The indel efficiencies for the target nucleic acids of each example were analyzed by the methods disclosed in Experimental Examples 1-12 to 1-13. The indel efficiency analysis results are shown in FIG. 2.

As disclosed in FIG. 2, the CRISPR/Cas14a1 system including an engineered guide RNA with a U-rich tail sequence linked to the 3'-terminus thereof showed higher indel efficiency than a the wild-type.

### Experimental Example 4 Indel efficiency comparison 3 of engineered CRISPR/Cas14a1 system having U-rich tail

Experiments were conducted to evaluate the indel efficiency of an engineered CRISPR/Cas14a1 system having a U-rich tail sequence, using the DYtarget2, DYtarget10, and Intergenic-22 as a target nucleic acid. The protospacer sequence of the target nucleic acid and each PAM sequence are disclosed in Table 10.

**[Table 10]**

| Target nucleic acid sequences used in Experimental Example 4 | | |
|---|---|---|
| Target nucleic acid | Protospacer sequence (5' to 3') of target nucleic acid | PAM |
| DYtarget2 | CACACACACAGTGGGCTACC (SEQ ID NO: 63) | TTTG |
| DYtarget10 | CATCCCCAGGACACACACAC (SEQ ID NO: 65) | TTTG |
| Intergenic-22 | AGAACACATACCCCTGGGCC (SEQ ID NO: 67) | TTTA |

1) A plasmid vector including a human codon-optimized Cas14a1 sequence was prepared according to Experimental Example 1-2.
2) According to Experimental Examples 1-2 and 1-4, plasmid vectors and amplicons capable of expressing a tracrRNA, and plasmid vectors and amplicons capable of expressing a (engineered) crRNA with a wild-type or U-rich tail sequence were prepared. DNA sequences encoding the tracrRNA and crRNA, and primer sequences used to prepare plasmid vectors and amplicons are shown in Tables 11 to 13.

**[Table 11]**

| DNA sequence encoding tracrRNA of Experimental Example 4 | |
|---|---|
| Amplicon sequence encoding tractrRNA (5' to 3') | SEQ ID NO |
| CTTCACTGATAAAGTGGAGAACCGCTTCACCAAAAGCTGTCCCTTAG GGGATTAGAACTTGAGTGAAGGTGGGCTGCTTGCATCAGCCTAATGT CGAGAAGTGCTTTCTTCGGAAAGTAACCCTCGAAACAAATTCATTTT TCCTCTCCAATTCTGCACAA | 61 |

In this regard, the amplicon sequence or plasmid vector sequence capable of expressing the tracrRNA is an amplicon sequence or plasmid vector sequence in which a U6 promoter is operably linked to the DNA sequence encoding the tracrRNA.

**[Table 12]**

| DNA sequences encoding crRNA of Experimental Example 4 | | | | | |
|---|---|---|---|---|---|
| Label | Target nucleic acid | crRNA common sequence (5' to 3') | Protospacer sequence (5' to 3') | U-rich tail sequence (3' to 5') | SEQ ID NO |
| Canonical crRNA | DYtarget2 (Target 1) | GTTGCAGAA CCCGAATAGA CGAATGAAG GAATGCAAC (SEQ ID NO: 255) | CACACACACAGTGGGC TACC | | 149 |
| gRNA (MS1) | | | CACACACACAGTGGGC TACC | TTTTATT TTTT | 150 |
| Canonical crRNA | DYtarget10 (Target 2) | | CATCCCCAGGACACAC ACAC | | 163 |
| gRNA (MS1) | | | CATCCCCAGGACACAC ACAC | TTTTATT TTTT | 164 |
| Canonical crRNA | Intergenic-22 | | AGAACACATACCCCTG GGCC | | 176 |
| gRNA (MS1) | (Target 3) | | AGAACACATACCCCTG GGCC | TTTTATT TTTT | 177 |

In the above table, for the DNA sequence encoding crRNA for each target nucleic acid, a crRNA common sequence - a protospacer sequence - a U-rich tail sequence are linked in the 5'-to-3' direction, and a crRNA repeat sequence in the crRNA common sequence is the sequence of SEQ ID NO: 58. The amplicon sequence or plasmid sequence capable of expressing the (engineered) crRNA is an amplicon sequence or plasmid sequence in which a U6 promoter is operably linked to the DNA sequence encoding the crRNA.

**[Table 13]**

| Primer sequences used in Experimental Example 4 | | | | |
|---|---|---|---|---|
| Label | Target nucleic acid | Forward primer (5' to 3') | Reverse primer (5' to 3') | SEQ ID NO |
| tracrRNA | - | GAGGGCC TATTTCCC ATGATT (SEQ ID NO: 183) | TTGTGCAGAATTGGAGAGGA | 236 |
| Canonical crRNA | DYtarget2 | | GGTAGCCCACTGTGTGTGTGGTTGCATTCCT TCATTCGTCTATTC | 207 |
| gRNA (MS1) | DYtarget2 | | aaaaaataaaaGGTAGCCCACTGTGTGTGTGGTTG CATTCCTTCATTCGTCTATTC | 208 |
| Canonical crRNA | DYtarget1 0 | | GTGTGTGTGTCCTGGGGATGGTTGCATTCCT TCATTCGTCTATTC | 221 |
| gRNA (MS1) | DYtarget1 0 | | aaaaataaaaGTGTGTGTGTCCTGGGGATGGTTGC ATTCCTTCATTCGTCaTATTC | 222 |
| Canonical crRNA | Intergenic -22 | | GGCCCAGGGGTATGTGTTCTGTTGCATTCCT | 234 |
| gRNA (MS1) | Intergenic -22 | | aaaaaataaaaGGCCCAGGGGTATGTGTTCTGTTG CATTCCT | 235 |

3) HEK-293T cells cultured according to Experimental Example 1-8 were transfected with a plasmid vector including the Cas14a1 sequence, a plasmid vector or amplicon of the tracrRNA, and a plasmid vector or amplicon of the crRNA by the method disclosed in Experimental Example 1-9. In this regard, the one into which crRNA was not injected was used as a control (No crRNA data in FIG. 3).

4) The indel efficiencies for the target nucleic acids of each example were analyzed by the methods disclosed in Experimental Examples 1-12 to 1-14. The indel efficiency analysis results are shown in FIG. 3.

As disclosed in FIG. 3, the CRISPR/Cas14a1 system including an engineered guide RNA with a U-rich tail sequence linked to 3'-terminus showed higher indel efficiency than a the wild-type.

### Experimental Example 5 Indel efficiency comparison 4 of engineered CRISPR/Cas14a1 system having U-rich tail

Experiments are conducted to evaluate the indel efficiency of an engineered CRISPR/Cas14a1 system having a U-rich tail sequence, using various target sequences as a target nucleic acid.

Experiments are conducted on the target nucleic acid disclosed in Experimental Example 3 or 4, and the experiments follow the following method instead of transfecting HEK-293 T cells with a plasmid vector including a Cas14a1 sequence and an amplicon of a single guide RNA.
1) A single guide RNA having the sgRNA sequence disclosed in Experimental Examples 3 and 4 is prepared according to Experimental Example 1-5.
2) A Cas14a1 protein is prepared according to Experimental Example 1-6.
3) RNPs are prepared from the resulting products of 1) and 2) according to Experimental Example 1-7.
4) The HEK-293 T cells prepared according to Experimental Example 1-8 are transfected with the RNPs according to Experimental Example 1-10.
5) The following indel efficiency analysis method is as described in Experimental Example 2 or 3.

### Experimental Example 6 Indel efficiency comparison 5 of engineered CRISPR/Cas14a1 system having U-rich tail

Experiments are conducted to evaluate the indel efficiency of an engineered CRISPR/Cas14a1 system having a U-rich tail sequence, using various target genes as a target nucleic acid.

Experiments are conducted on the target nucleic acid disclosed in Experimental Example 3 or 4, and the experiments follow the following method instead of transfecting HEK-293 T cells with a plasmid vector including a Cas14a1 sequence and an amplicon of a single guide RNA.
1) Viral vectors having the sgRNA sequence disclosed in Experimental Examples 3 and 4 and a sequence encoding a Cas14a1 protein are prepared according to Experimental Example 1-3.
4) The HEK-293 T cells prepared according to Experimental Example 1-8 are transfected with the viral vector according to Experimental Example 1-11.
5) The following indel efficiency analysis method is as described in Experimental Example 2 or 3.

### Experimental Example 7 Indel efficiency comparison 1 according to structure of U-rich tail

Experiments were conducted to evaluate the indel efficiency of an engineered CRISPR/Cas14a1 system according to the structure of the U-rich tail sequence, using the DYtarget2 and DYtarget10 as a target nucleic acid. The protospacer sequence of the target nucleic acid and each PAM sequence are disclosed in Table 14.

**[Table 14]**

| Target nucleic acid sequences used in Experimental Example 7 | | |
|---|---|---|
| Target nucleic acid | Protospacer sequence (5' to 3') of target nucleic acid | PAM |
| DYtarget2 | CACACACACAGTGGGCTACC (SEQ ID NO: 63) | TTTG |
| DYtarget10 | CATCCCCAGGACACACACAC (SEQ ID NO: 65) | TTTG |

1) A plasmid vector including a human codon-optimized Cas14a1 sequence was prepared according to Experimental Example 1-2.
2) An amplicon capable of expressing a wild-type, or (an engineered) single guide RNA having a U-rich tail sequence was prepared according to Experimental Example 1-4. DNA sequences encoding the single guide RNA and primer sequences used to prepare an amplicon are shown in Tables 15 and 16.

**[Table 15]**

| DNA sequences encoding sgRNA of Experimental Example 7 | | | | | |
|---|---|---|---|---|---|
| Label | Target nucleic acid | sgRNA common sequence (5' to 3') | Protospacer sequence (5' to 3') | U-rich tail sequence (3' to 5') | SEQ ID NO |
| Ux | DYtarget2 | CTTCACTGAT AAAGTGGAG AACCGCTTC ACCAAAAGC TGTCCCTTAG GGGATTAGA ACTTGAGTG AAGGTGGGC TGCTTGCATC AGCCTAATGT CGAGAAGTG CTTTCTTCGG AAAGTAACC CTCGAAACA AATTCATTTT | CACACACACAGTGGGC TACC | | 129 |
| U6 | | | | TTTTTT | 135 |
| U4AU6 | | | | TTTTATT TTTT | 142 |
| U3AU6 | | | | TTTATTT TTT | 147 |
| U3AU3AU6 | | TCCTCTCCAA TTCTGCACAA gaaaGTTGCAG AACCCGAATA GACGAATGA AGGAATGCA AC (SEQ ID NO: 126) | | TTTATTT ATTTTTT | 148 |
| Ux | DYtarget10 | | CATCCCCAGGACACAC ACAC | | 158 |
| U6 | | | | TTTTTT | 160 |
| U4AU6 | | | | TTTTATT TTTT | 159 |
| U3AU6 | | | | TTTATTT TTT | 161 |
| U3AU3AU6 | | | | TTTATTT ATTTTTT | 162 |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 16]**

| Primer sequences used in Experimental Example 7 | | | | |
|---|---|---|---|---|
| Label | Target nucleic acid | Forward primer (5' to 3') | Reverse primer (5' to 3') | SEQ ID NO |
| Ux | DYtarget2 | GAGGGCC TATTTCCC ATGATT (SEQ ID NO: 183) | GGTAGCCCACTGTGTGTGTGGTTGCATTCCT TCATTCGTCTATTC | 187 |
| U6 | | | aaaaaaGGTAGCCCACTGTGTGTGTGGTTGCAT TCCTTCATTCGTCTATTC | 193 |
| U4AU6 | | | aaaaaataaaaGGTAGCCCACTGTGTGTGTGGTTG CATTCCTTCATTCGTCTATTC | 200 |
| U3AU6 | | | aaaaaataaaGGTAGCCCACTGTGTGTGTGGTTG CATTCCTTCATTCGTCTATTC | 205 |
| U3AU3A U6 | | | aaaaaataaataaaGGTAGCCCACTGTGTGTGTGGT TGCATTCCTTCATTCGTCTATTC | 206 |
| Ux | DYtarget1 0 | | GTGTGTGTGTCCTGGGGATGGTTGCATTCCT TCATTCGTCTATTC | 216 |
| U6 | | | aaaaaaGTGTGTGTGTCCTGGGGATGGTTGCAT TCCTTCATTCGTCTATTC | 218 |
| U4AU6 | | | aaaaaataaaaGTGTGTGTGTCCTGGGGATGGTTG CATTCCTTCATTCGTCTATTC | 217 |
| U3AU6 | | | aaaaaataaaGTGTGTGTGTCCTGGGATGGTTGC ATTCCTTCATTCGTCTATTC | 219 |
| U3AU3A U6 | | | aaaaaataaataaaGTGTGTGTGTCCTGGGATGGT TGCATTCCTTCATTCGTCTATTC | 220 |

3) HEK-293T cells cultured according to Experimental Example 1-8 were transfected with a plasmid vector including the Cas14a1 sequence and an amplicon of the single guide RNA by the method disclosed in Experimental Example 1-9.

4) The indel efficiencies for the target nucleic acids of each example were analyzed by the methods disclosed in Experimental Examples 1-12 to 1-14. The indel efficiency analysis results are shown in FIG. 4. In this regard, HEK-293T cells that had not been subjected to any treatment were used as a control (see the WT data in FIG. 4).

### Experimental Example 8 Indel efficiency comparison 2 according to structure of U-rich tail

Experiments were conducted to evaluate the indel efficiency of an engineered CRISPR/Cas14a1 system according to the structure of the U-rich tail sequence, using the DYTarget2 as a target nucleic acid. The protospacer sequence of the target nucleic acid and the PAM sequence are shown as "DYTarget2" in Table 14 of Experimental Example 7.
1) A plasmid vector including a human codon-optimized Cas14a1 sequence was prepared according to Experimental Example 1-2.
2) An amplicon capable of expressing a wild-type, or (an engineered) single guide RNA having a U-rich tail sequence was prepared according to Experimental Example 1-4. DNA sequences encoding the single guide RNA and primer sequences used to prepare an amplicon are shown in Tables 17 and 18.

**[Table 17]**

| | DNA sequences encoding sgRNA of Experimental Example 8 | | | | |
|---|---|---|---|---|---|
| Label | Target nucleic acid | sgRNA common sequence (5' to 3') | Protospacer sequence (5' to 3') | U-rich tail sequence (3' to 5') | SEQ ID NO |
| None | DYtarget2 | CTTCACTGAT AAAGTGGAG AACCGCTTC ACCAAAAGC TGTCCCTTAG GGGATTAGA ACTTGAGTG AAGGTGGGC TGCTTGCATC AGCCTAATGT CGAGAAGTG CTTTCTTCGG AAAGTAACC CTCGAAACA AATTCATTTT TCCTCTCCAA TTCTGCACAA gaaaGTTGCAG AACCCGAATA GACGAATGA AGGAATGCA AC (SEQ ID NO: 126) | CACACACACAGTGGGC TACC | | 129 |
| T | | | | T | 130 |
| T2 | | | | TT | 131 |
| T3 | | | | TTT | 132 |
| T4 | | | | TTTT | 133 |
| T4A | | | | TTTTA | 136 |
| T4AT | | | | TTTTAT | 137 |
| T4AT2 | | | | TTTTATT | 138 |
| T4AT3 | | | | TTTTATT T | 139 |
| T4AT4 | | | | TTTTATT TT | 140 |
| T4AT5 | | | | TTTTATT TTT | 141 |
| T4AT6 | | | | TTTTATT TTTT | 142 |
| T4AT7 | | | | TTTTATT TTTTT | 143 |
| T4AT8 | | | | TTTTATT TTTTTT | 144 |
| T4AT4AT | | | | TTTTATT TTAT | 256 |
| T4AT4AT2 | | | | TTTTATT TTATT | 257 |

In the above table, for the amplicon sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 18]**

| | Primer sequences used in Experimental Example 8 | | | |
|---|---|---|---|---|
| Label | Target nucleic acid | Forward primer (5' to 3') | Reverse primer (5' to 3') | |
| None | DYtarget2 | GAGGGCC TATTTCCC ATGATT | GGTAGCCCACTGTGTGTGTGGTTGCATTCCT TCATTCGTCTATTC | 187 |
| T | | (SEQ ID NO: 183) | aGGTAGCCCACTGTGTGTGTGGTTGCATTCCT TCATTCGTCTATTC | 188 |
| T2 | | | aaGGTAGCCCACTGTGTGTGTGGTTGCATTCC TTCATTCGTCTATTC | 189 |
| T3 | | | aaaGGTAGCCCACTGTGTGTGTGGTTGCATTC CTTCATTCGTCTATTC | 190 |
| T4 | | | aaaaGGTAGCCCACTGTGTGTGTGGTTGCATT CCTTCATTCGTCTATTC | 191 |
| T4A | | | taaaaGGTAGCCCACTGTGTGTGTGGTTGCATT CCTTCATTCGTCTATTC | 194 |
| T4AT | | | ataaaaGGTAGCCCACTGTGTGTGTGGTTGCAT TCCTTCATTCGTCTATTC | 195 |
| T4AT2 | | | aataaaaGGTAGCCCACTGTGTGTGTGGTTGCAT TCCTTCATTCGTCTATTC | 196 |
| T4AT3 | | | aaataaaaGGTAGCCCACTGTGTGTGTGGTTGCA TTCCTTCATTCGTCTATTC | 197 |
| T4AT4 | | | aaaataaaaGGTAGCCCACTGTGTGTGTGGTTGC ATTCCTTCATTCGTCTATTC | 198 |
| T4AT5 | | | aaaaataaaaGGTAGCCCACTGTGTGTGTGGTTG CATTCCTTCATTCGTCTATTC | 199 |
| T4AT6 | | | aaaaaataaaaGGTAGCCCACTGTGTGTGTGGTTG CATTCCTTCATTCGTCTATTC | 200 |
| T4AT7 | | | aaaaaaataaaaGGTAGCCCACTGTGTGTGTGGTT GCATTCCTTCATTCGTCTATTC | 201 |
| T4AT8 | | | aaaaaaaaataaaaGGTAGCCCACTGTGTGTGTGGTT GCATTCCTTCATTCGTCTATTC | 202 |
| T4AT4AT | | | ataaaataaaaGGTAGCCCACTGTGTGTGTGGTTG CATTCCTTCATTCGTCTATTC | 258 |
| T4AT4AT 2 | | | aataaaataaaaGGTAGCCCACTGTGTGTGTGGTT GCATTCCTTCATTCGTCTATTC | 259 |

1) HEK-293T cells cultured according to Experimental Example 1-8 were transfected with a plasmid vector including the Cas14a1 sequence and an amplicon of the single guide RNA by the method disclosed in Experimental Example 1-9.
2) The indel efficiencies for the target nucleic acids of each example were analyzed by the methods disclosed in Experimental Examples 1-12 to 1-14. The indel efficiency analysis results are shown in FIG. 5.

### Experimental Example 9 Indel efficiency comparison 3 according to structure of U-rich tail

Experiments were conducted to evaluate the indel efficiency of an engineered CRISPR/Cas14a1 system according to the structure of the U-rich tail sequence, using CSMD1 as a target nucleic acid. The protospacer sequence of the target nucleic acid and the PAM sequence are shown in Table 19.

**[Table 19]**

| Target nucleic acid sequence used in Experimental Example 9 | | |
|---|---|---|
| Target nucleic acid | Protospacer sequence (5' to 3') of target nucleic acid | PAM |
| CSMD1 | GAATACCCCCATTCTTCAGG (SEQ ID NO: 260) | TTTA |

1) A recombinant Cas12f1 protein was prepared according to Experimental Example 1-6.
2) A wild-type, or (an engineered) single guide RNA having a U-rich tail sequence was synthesized according to Experimental Example 1-5. The sequences of the single guide RNA are shown in the following Table 20.

**[Table 20]**

| | sgRNA sequences of Experimental Example 9 | | | | |
|---|---|---|---|---|---|
| Label | Target nucleic acid | sgRNA common sequence (5' to 3') | Spacer sequence (5' to 3') | U-rich tail sequence (3' to 5') | SEQ ID NO |
| None | CSMD1 | CUUCACUGAUA AAGUGGAGAAC CGCUUCACCAA | GAAUACCCCC AUUCUUCAGG | | 261 |
| U | | AAGCUGUCCCU UAGGGGAUUAG AACUUGAGUGA AGGUGGGCUGC UUGCAUCAGCC UAAUGUCGAGA AGUGCUUUCUU CGGAAAGUAAC CCUCGAAACAA AUUCAUUUUUC CUCUCCAAUUC UGCACAAgaaaGU UGCAGAACCCG AAUAGACGAAU GAAGGAAUGCA AC (SEQ ID NO: 74) | | U | 262 |
| U2 | | | | UU | 263 |
| U3 | | | | UUU | 264 |
| U4 | | | | UUUU | 265 |
| U5 | | | | UUUUU | 266 |
| U6 | | | | UUUUUU | 267 |
| U4A | | | | UUUUA | 268 |
| U4AU | | | | UUUUAU | 269 |
| U4AU2 | | | | UUUUAUU | 270 |
| U4AU3 | | | | UUUUAUUU | 271 |
| U4AU4 | | | | UUUUAUUUU | 272 |
| U4AU5 | | | | UUUUAUUUUU | 273 |
| U4AU6 | | | | UUUUAUUUUUU | 274 |
| U4AU7 | | | | UUUUAUUUUUUU | 275 |
| U4AU8 | | | | UUUUAUUUUUUU U | 276 |
| U4AU9 | | | | UUUUAUUUUUUU UU | 277 |
| U4AU10 | | | | UUUUAUUUUUUU UUU | 278 |

In the above table, for the sgRNA sequence for each target nucleic acid, an sgRNA common sequence - a spacer sequence - a U-rich tail sequence are linked in the 5'-to-3' direction, and a scaffold sequence in the sgRNA common sequence is a sequence portion of SEQ ID NO: 253.

3) RNPs were prepared according to Experimental Example 1-7 using the recombinant Cas14a1 and the single guide RNA, and the HEK293 T cells cultured according to Experimental Example 1-8 were transfected with the RNPs by the method disclosed in Experimental Example 1-7.

2) The indel efficiencies for the target nucleic acids of each example were analyzed by the methods disclosed in Experimental Examples 1-12 to 1-13. The indel efficiency analysis results are shown in FIG. 6.

As a result of the experiments, it could be seen that that the engineered guide RNA having a U-rich tail sequence linked to 3'-terminus showed a remarkably higher indel efficiency than the wild-type guide RNA having no U-rich tail sequence.

In particular, it could be seen that the engineered guide RNA with a sequence with 10 or more linked uridines showed an excellent indel efficiency compared to the wild-type guide RNA.

### Experimental Example 10 Indel efficiency comparison 3 according to structure of U-rich tail

Experiments were conducted to evaluate the indel efficiency of an engineered CRISPR/Cas14a1 system according to the structure of a modified continuous uridine sequence among the U-rich tail sequences, using the DYtarget2 as a target nucleic acid. The protospacer sequence of the target nucleic acid and the PAM sequence are shown as "DYTarget2" in Table 14 of Experimental Example 7.
1) A plasmid vector including a human codon-optimized Cas14a1 sequence was prepared according to Experimental Example 1-2.
2) An amplicon capable of expressing a wild-type, or (an engineered) single guide RNA having a U-rich tail sequence was prepared according to Experimental Example 1-4. DNA sequences encoding the single guide RNA and primer sequences used to prepare an amplicon are shown in Tables 21 and 22.

**[Table 21]**

| | DNA sequences encoding sgRNA of Experimental Example 10 | | | | | |
|---|---|---|---|---|---|---|
| Label | | Target nucleic acid | sgRNA common sequence (5' to 3') | Protospacer sequence (5' to 3') | U-rich tail sequence (3' to 5') | SEQ ID NO |
| None | | | | | - | |
| T4AT6 | | | | | TTTTATT TTTT | |
| T4GT6 | | DYtarget2 | CTTCACTGAT AAAGTGGAG AACCGCTTC ACCAAAAGC TGTCCCTTAG GGGATTAGA ACTTGAGTG AAGGTGGGC TGCTTGCATC AGCCTAATGT CGAGAAGTG CTTTCTTCGG AAAGTAACC CTCGAAACA AATTCATTTT TCCTCTCCAA TTCTGCACAA gaaaGTTGCAG AACCCGAATA GACGAATGA AGGAATGCA AC (SEQ ID NO: 126) | CACACACACAGTGGGC TACC | TTTTGTT TTTT | |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 22]**

| Primer sequences used in Experimental Example 10 | | | | |
|---|---|---|---|---|
| Label | Target nucleic acid | Forward primer (5' to 3') | Reverse primer (5' to 3') | SEQ ID NO |
| None | DYtarget2 | GAGGGCC TATTTCCC ATGATT (SEQ ID NO: 183) | GGTAGCCCACTGTGTGTGTGGTTGCATTCCT TCATTCGTCTATTC | |
| T4AT6 | | | aaaaaataaaaGGTAGCCCACTGTGTGTGTGGTTG CATTCCTTCATTCGTCTATTC | |
| T4GT6 | | | aaaaaacaaaaGGTAGCCCACTGTGTGTGTGGTTG CATTCCTTCATTCGTCTATTC | |

3) HEK-293T cells cultured according to Experimental Example 1-8 were transfected with a plasmid vector including the nucleic acid sequence encoding the Cas14a1 sequence and an amplicon of the single guide RNA by the method disclosed in Experimental Example 1-9.

4) The indel efficiencies for the target nucleic acids of each example were analyzed by the methods disclosed in Experimental Examples 1-12 to 1-14. The indel efficiency analysis results are shown in FIG. 7.

As a result of the experiment, it was shown that when the engineered guide RNA has a modified continuous uridine sequence structure which can be represented by (UₐV)ₙU_{b} (in this regard, V is one of cytidine (C), guanosine (G), or adenosine (A)), the indel efficiency was remarkably higher in eukaryotic cells than the wild-type guide RNA having no U-rich tail sequence.

### Experimental Example 11 Comparison of indel efficiency according to length of spacer sequence

In order to compare the indel efficiency according to the length of the spacer sequence, the DYtarget2 and DYtarget10 were used as target nucleic acids, the U-rich tail sequence was fixed as U4AU6, and then the experiment was performed by changing the length of the spacer sequence. The protospacer sequences of the target nucleic acid and each PAM sequence are disclosed in Table 23.

**[Table 23]**

| Target nucleic acid sequences used in Experimental Example 11 | | |
|---|---|---|
| Target nicleic acid | Protospacer sequence (5' to 3') of target nucleic acid | PAM |
| DYtarget2 | CACACACACAGTGGGCTACC (SEQ ID NO: 63) | TTTG |
| DYtarget10 | CATCCCCAGGACACACACAC (SEQ ID NO: 65) | TTTG |

1) A plasmid vector including a nucleic acid sequence encoding a human codon-optimized Cas14a1 sequence was prepared according to Experimental Example 1-2.
2) An amplicon capable of expressing an engineered single guide RNA having a length of each different spacer sequence was prepared according to Experimental Example 1-4. DNA sequences encoding the single guide RNA, and primer sequences used to prepare an amplicon are shown in Tables 24 and 25.

**[Table 24]**

| DNA sequences encoding sgRNA used in Experimental Example 11 | | | | | |
|---|---|---|---|---|---|
| Label | Target nucleic acid | sgRNA common sequence (5' to 3') | Protospacer sequence (5' to 3') | U-rich tail sequence (3' to 5') | SEQ ID NO |
| 18mer | DYtarget2 | CTTCACTGAT AAAGTGGAG AACCGCTTC ACCAAAAGC TGTCCCTTAG GGGATTAGA ACTTGAGTG AAGGTGGGC TGCTTGCATC AGCCTAATGT CGAGAAGTG CTTTCTTCGG AAAGTAACC CTCGAAACA AATTCATTTT TCCTCTCCAA TTCTGCACAA gaaaGTTGCAG AACCCGAATA GACGAATGA AGGAATGCA AC (SEQ ID NO: 126) | CACACACACAGTGGGC TA | TTTTATT . TTTT | 151 |
| 19mer | | | CACACACACAGTGGGC TAC | | 152 |
| 20mer | | | CACACACACAGTGGGC TACC | | 142 |
| 21mer | | | 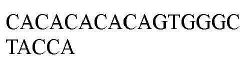 | | 153 |
| 25mer | | | | | 154 |
| 30mer | | | CACACACACAGTGGGC TACCATTTAAGGAG | | 155 |
| 17mer | DYtarget10 | | CATCCCCAGGACACAC A | | 165 |
| 18mer | | | CATCCCCAGGACACAC AC | | 166 |
| 19mer | | | CATCCCCAGGACACAC ACA | | 167 |
| 20mer | | | CATCCCCAGGACACAC ACAC | | 159 |
| 21mer | | | CATCCCCAGGACACAC ACACA | | 168 |
| 25mer | | | CATCCCCAGGACACAC ACACATTTC | | 169 |
| 30mer | | | CATCCCCAGGACACAC ACACATTTCAAAAG | | 170 |

In the above table, for the DNA sequence encoding sgRNA for each target nucleic acid, an sgRNA common sequence - a protospacer sequence - a U-rich tail sequence are linked in the 5'-to-3' direction, and a scaffold sequence in the sgRNA common sequence is a sequence portion of SEQ ID NO: 254. The amplicon sequence capable of expressing the (engineered) single guide RNA is an amplicon sequence in which a U6 promoter is operably linked to the DNA sequence encoding the sgRNA.

**[Table 25]**

| | Primer sequences used in Experimental Example 11 | | | |
|---|---|---|---|---|
| Label | Target nucleic acid | Forward primer (5' to 3') | Reverse primer (5' to 3') | SEQ ID NO |
| 18mer | DYtarget2 | GAGGGCC TATTTCCC ATGATT (SEQ ID NO: 183) | aaaaaataaaaTAGCCCACTGTGTGTGTGGTTGCA TTCCT | 209 |
| 19mer | | | aaaaaataaaaGTAGCCCACTGTGTGTGTGGTTGC ATTCCT | 210 |
| 20mer | | | aaaaaataaaaGGTAGCCCACTGTGTGTGTGGTTG CATTCCT | 200 |
| 21mer | | | aaaaaataaaaTGGTAGCCCACTGTGTGTGTGGTT GCATTCCT | 211 |
| 25mer | | | aaaaaataaaaTAAATGGTAGCCCACTGTGTGTGT GGTTGCATTCCT | 212 |
| 30mer | | | aaaaaataaaaCTCCTTAAATGGTAGCCCACTGTG TGTGTGGTTGCATTCCT | 213 |
| 17mer | DYtarget1 0 | | aaaaaataaaaTGTGTGTCCTGGGGATGGTTGCAT TCCT | 223 |
| 18mer | | | aaaaaataaaaGTGTGTGTCCTGGGGATGGTTGCA TTCCT | 224 |
| 19mer | | | aaaaaataaaaTGTGTGTGTCCTGGGATGGTTGC ATTCCT | 225 |
| 20mer | | | aaaaaataaaaGTGTGTGTGTCCTGGGATGGTTG CATTCCT | 217 |
| 21mer | | | aaaaaataaaaTGTGTGTGTGTCCTGGGGATGGTT GCATTCCT | 226 |
| 25mer | | | aaaaaataaaaGAAATGTGTGTGTGTCCTGGGGAT GGTTGCATTCCT | 227 |
| 30mer | | | aaaaaataaaaCTTTTGAAATGTGTGTGTGTCCTG GGGATGGTTGCATTCCT | 228 |

3) HEK-293T cells cultured according to Experimental Example 1-8 were transfected with a plasmid vector including the Cas14a1 sequence and an amplicon of the single guide RNA by the method disclosed in Experimental Example 1-9.

4) The indel efficiencies for the target nucleic acids of each example were analyzed by the methods disclosed in Experimental Examples 1-12 to 1-14. The indel efficiency analysis results are shown in FIG. 8.

### Experimental Example 12 Confirmation of intracellular gene cleavage activity of Type V CRISPR/Cas system having U-rich tail

In order to confirm whether the gene cleavage activity is enhanced when the U-rich tail sequence is introduced into the Type V CRISPR/Cas system other than the engineered CRISPR/Cas14a system used in the experimental example, the following experiment was conducted on the CRISPR/SpCas12f1 system and the CRISPR/AsCas12f1 system (Karvelis et al., Nucleic Acids Research, Vol. 48, No. 95017 (2020)), which are classified as CRISPR/Cas12f1 systems.

### Experimental Example 12-1. Confirmation of intracellular expression of SpCas12f1 and AsCas12f1 proteins

In order to confirm whether the SpCas12f1 and AsCas12f1 proteins are properly expressed in eukaryotic cells when a vector encoding the SpCas12f1 and AsCas12f1 proteins is introduced into eukaryotic cells, the expression of SpCas12f1 and AsCas12f1 proteins was confirmed by the method disclosed in Experimental Example 1-13. In this regard, amino acid sequences of the SpCas12f1 and AsCas12f1 proteins and human codon-optimized nucleic acid sequences encoding the amino acid sequences are shown in Table 26.

**[Table 26]**

| | Sequence information of SpCas12f1 and AsCas12f1 proteins | SEQ ID NO |
|---|---|---|
| Subject | Sequence information | |
| SpCas12 f1 (Amino acid) | MGESVKAIKLKILDMFLDPECTKQDDNWRKDLSTMSRFCAEAGNMCLR DLYNYFSMPKEDRISSKDLYNAMYHKTKLLHPELPGKVANQIVNHAKDV WKRNAKLIYRNQISMPTYKITTAPIRLQNNIYKLIKNKNKYIIDVQLYSKEY SKDSGKGTHRYFLVAVRDSSTRMIFDRIMSKDHIDSSKSYTQGQLQIKKDH QGKWYCIIPYTFPTHETVLDPDKVMGVDLGVAKAVYWAFNSSYKRGCID GGEIEHFRKMIRARRVSIQNQIKHSGDARKGHGRKRALKPIETLSEKEKNF RDTINHRYANRIVEAAIKQGCGTIQIENLEGIADTTGSKFLKNWPYYDLQT KIVNKAKEHGITVVAINPQYTSQRCSMCGYIEKTNRSSQAVFECKQCGYG SRTICINCRHVQVSGDVCEECGGIVKKENVNADYNAAKNISTPYIDQIIME KCLELGIPYRSITCKECGHIQASGNTCEVCGSTNILKPKKIRKAK | 282 |
| SpCas12 f1 (Human codon-optimize d nucleic acid) | ATGGGCGAGAGCGTTAAAGCAATCAAACTGAAGATCCTGGACATGTTC CTGGACCCGGAGTGCACTAAACAGGACGATAACTGGCGTAAAGACCTG TCTACCATGTCTCGTTTCTGCGCAGAGGCGGGTAACATGTGCCTGCGCG ACCTGTACAATTACTTTTCTATGCCGAAAGAAGACCGTATTAGCTCCAA GGATCTGTACAACGCCATGTACCACAAAACTAAACTGCTGCACCCGGA ACTGCCGGGTAAAGTGGCGAACCAGATCGTCAACCACGCTAAAGATGT GTGGAAACGCAACGCGAAGCTGATCTATCGTAATCAGATCTCTATGCCG ACTTACAAGATCACCACCGCACCGATTCGCCTGCAAAACAACATCTATA AACTGATCAAAAACAAGAACAAATATATCATCGATGTCCAGCTGTACAG CAAAGAATACTCTAAAGACAGCGGCAAAGGCACCCACCGCTACTTCCT GGTTGCAGTGCGCGATTCTAGCACGCGTATGATCTTCGACCGCATCATG AGCAAAGACCACATCGATAGCTCCAAATCCTACACCCAGGGTCAGCTG CAGATTAAAAAAGACCATCAGGGCAAATGGTACTGTATCATTCCGTACA CCTTCCCGACTCATGAGACCGTCCTGGATCCGGACAAAGTTATGGGTGT GGACCTGGGCGTGGCTAAAGCAGTGTATTGGGCGTTCAACTCCTCTTAC AAACGCGGTTGTATCGATGGTGGTGAAATTGAACACTTCCGCAAAATG ATCCGTGCTCGTCGTGTCTCCATTCAGAACCAGATTAAACATTCTGGCG | 284 |
| | ACGCGCGCAAGGGTCATGGTCGTAAACGTGCACTGAAGCCGATTGAAA CCCTGTCTGAAAAAGAAAAAAACTTCCGCGATACTATCAACCATCGTTA CGCAAACCGTATCGTGGAGGCAGCGATTAAGCAGGGCTGCGGCACCAT CCAGATCGAAAATCTGGAGGGTATCGCCGATACTACGGGTTCCAAATTT CTGAAAAACTGGCCATACTATGACCTGCAGACCAAAATCGTGAACAAA GCGAAGGAACATGGCATCACCGTCGTTGCGATCAACCCGCAGTATACTT CTCAACGTTGCTCCATGTGTGGTTACATTGAAAAAACCAACCGTTCCTC TCAGGCAGTGTTCGAGTGCAAACAGTGCGGCTATGGTTCCCGTACCATC TGCATCAACTGCCGCCACGTTCAGGTAAGCGGTGATGTCTGCGAAGAA TGCGGCGGCATCGTTAAAAAAGAAAACGTTAACGCGGACTATAACGCG GCGAAAAACATCTCCACCCCATATATCGATCAGATCATCATGGAGAAAT GTCTGGAGCTGGGTATCCCGTACCGTAGCATCACTTGCAAAGAATGCGG TCATATCCAGGCGTCTGGTAACACTTGTGAGGTGTGCGGTTCCACCAAC ATTCTGAAACCGAAAAAAATCCGTAAAGCTAAA | |
| AsCas1 2f1 (amino acid) | MIKVYRYEIVKPLDLDWKEFGTILRQLQQETRFALNKATQLAWEWMGFS SDYKDNHGEYPKSKDILGYTNVHGYAYHTIKTKAYRLNSGNLSQTIKRAT DRFKAYQKEILRGDMSIPSYKRDIPLDLIKENISVNRMNHGDYIASLSLLSN PAKQEMNVKRKISVIIIVRGAGKTIMDRILSGEYQVSASQIIHDDRKNKWY LNISYDFEPQTRVLDLNKIMGIDLGVAVAVYMAFQHTPARYKLEGGEIENF RRQVESRRISMLRQGKYAGGARGGHGRDKRIKPIEQLRDKIANFRDTTNH RYSRYIVDMAIKEGCGTIQMEDLTNIRDIGSRFLQNWTYYDLQQKIIYKAE EAGIKVIKIDPQYTSQRCSECGNIDSGNRIGQAIFKCRACGYEANADYNAA RNIAIPNIDKIIAESIK | 283 |
| AsCas1 2f1 (human codon-optimize d nucleic acid) | ATGATCAAGGTTTATCGCTATGAAATTGTGAAACCGCTGGACCTGGACT GGAAAGAGTTCGGTACTATCCTGCGTCAGCTGCAGCAGGAAACGCGTT TCGCACTGAACAAAGCGACGCAGCTGGCTTGGGAATGGATGGGCTTCA GCTCTGATTACAAAGACAACCACGGCGAATACCCTAAATCCAAAGATAT CCTGGGTTACACCAACGTGCACGGTTATGCGTACCACACCATCAAAACT AAAGCGTACCGCCTGAACTCCGGTAATCTGTCTCAGACCATCAAGCGT GCGACCGACCGTTTCAAAGCGTACCAGAAGGAAATCCTGCGCGGTGAC ATGAGCATCCCGTCCTATAAGCGTGACATCCCGCTGGATCTGATCAAAG AAAACATCAGCGTAAACCGCATGAACCACGGTGACTACATCGCCTCCC TGTCTCTGCTGTCTAACCCGGCAAAGCAAGAGATGAACGTTAAACGCA AAATCTCCGTTATCATTATCGTTCGTGGCGCAGGCAAAACCATCATGGAT CGTATTCTGTCCGGTGAATATCAAGTGTCCGCAAGCCAGATCATCCATG ACGATCGTAAAAATAAATGGTATCTGAACATCTCTTATGATTTCGAACCG CAGACCCGCGTACTGGACCTGAACAAAATCATGGGTATCGATCTGGGC GTTGCAGTAGCGGTGTACATGGCTTTCCAGCACACCCCGGCACGCTAC AAACTGGAAGGTGGTGAGATCGAAAACTTCCGTCGTCAAGTGGAATCT CGTCGCATCTCTATGCTGCGTCAGGGTAAATATGCAGGTGGTGCGCGTG GTGGTCATGGTCGTGACAAACGTATTAAACCAATCGAACAGCTGCGCG ATAAAATCGCCAACTTCCGTGATACCACCAACCACCGCTACTCTCGTTA TATCGTTGATATGGCAATCAAAGAGGGTTGTGGTACTATCCAGATGGAA GACCTGACGAACATCCGCGATATCGGTTCTCGTTTCCTGCAGAACTGGA CCTACTACGATCTGCAGCAGAAAATTATCTACAAAGCGGAGGAAGCAG GTATCAAAGTCATTAAAATCGATCCGCAGTATACTAGCCAGCGCTGCAG CGAATGTGGTAACATTGATAGCGGCAACCGCATTGGTCAGGCAATCTTT AAATGCCGCGCTTGTGGCTACGAAGCGAACGCAGACTACAACGCGGCG CGTAACATCGCCATCCCGAATATTGACAAAATCATTGCCGAATCTATTAA A | 285 |

As a result of the experiment, it was confirmed that the SpCas12f1 protein and the AsCas12f1 protein were properly expressed in HEK293T cells (FIG. 9).

### Experimental Example 12-2. Confirmation of intracellular gene cleavage activity of engineered CRISPR/SpCas12f1 and CRISPR/AsCas12f1 systems having U-rich tail

In order to confirm the gene cleavage activity of the engineered CRISPR/SpCas12f1 system and CRISPR/AsCas12f1 system having a U-rich tail sequence, experiments were conducted using various gene sequences as a target nucleic acid. The protospacer sequences of the target nucleic acid and each PAM sequence are disclosed in Table 27.

**[Table 27]**

| Target nucleic acids used in Experimental Example 12 | | |
|---|---|---|
| Target nucleic acid | Protospacer sequence (5' to 3') of target nucleic acid | PAM |
| DYtarget2 | CACACACACAGTGGGCTACC (SEQ ID NO: 63) | TTTG |
| VEGFA-3 | GGGGTGACCGCCGGAGCGCG (SEQ ID NO: 286) | TTTG |

1) Plasmid vector including human codon-optimized sequences encoding SpCas12f1 and AsCas12f1 proteins were prepared according to Experimental Example 1-2.
2) According to Experimental Example 1-2, a plasmid vector expressing tracrRNA, and a plasmid vector expressing a wild-type crRNA or a (engineered) crRNA having a U-rich tail sequence were prepared. DNA sequences encoding the tracrRNA and crRNA, and primer sequences used to prepare plasmid vectors are shown in Tables 28 to 30.

**[Table 28]**

| DNA sequence encoding tracrRNA of Experimental Example 12 | |
|---|---|
| tracrRNA sequence (5' to 3') | SEQ ID NO |
| CTTCACTGATAAAGTGGAGAACCGCTTCACCAAAAGCTGTCCCTTAG GGGATTAGAACTTGAGTGAAGGTGGGCTGCTTGCATCAGCCTAATGT CGAGAAGTGCTTTCTTCGGAAAGTAACCCTCGAAACAAATTCATTTT TCCTCTCCAATTCTGCACAA | 287 |

The CRISPR/SpCas12f1 and CRISPR/AsCas12f1 systems have the same tracrRNA sequence. The plasmid vector sequence which expresses the tracrRNA is a plasmid vector sequence in which a U6 promoter is operably linked to the DNA sequence encoding the tracrRNA.

**[Table 29]**

| DNA sequences encoding crRNA of Experimental Example 12 | | | | | |
|---|---|---|---|---|---|
| Label | Target nucleic acid | crRNA common sequence (5' to 3') | Protospacer sequence (5' to 3') | U-rich tail sequence (3'to5') | SEQ ID NO |
| SpCas12f1-DYtarget2(WT) | DYtargete | GTCGCATCTT GCGTAAGCG CGTGGATTGA AAC (SEQ ID NO: 288) | CACACACACAGTGGGC TACC | | 290 |
| SpCas12f1-DYtarget2(U6) | | | | TTTTTT | 291 |
| SpCas12f1-DYtarget2(U4AU6) | | | | TTTTATT TTTT | 292 |
| SpCas12f1-VEGFA-3(WT) | VEGFA-3 | | GGGGTGACCGCCGGAG CGCG | | 293 |
| SpCas12f1-VEGFA-3(U6) | | | | TTTTTT | 294 |
| SpCas12f1-VEGFA-3(U4AU6) | | | | TTTTATT TTTT | 295 |
| AsCas12f1-DYtarget2(WT) | DYtarget2 | GTTTGCGAG CTAGCTTGTG GAGTGTGAA C (SEQ ID NO: 289) | CACACACACAGTGGGC TACC | | 296 |
| AsCas12f1-DYtarget2(U6) | | | | TTTTTT | 297 |
| AsCas12f1-DYtarget2(U4AU6) | | | | TTTTATT TTTT | 298 |
| AsCas12f1-VEGFA-3(WT) | VEGFA-3 | | GGGGTGACCGCCGGAG CGCG | | 299 |
| AsCas12f1-VEGFA-3(U6) | | | | TTTTTT | 300 |
| AsCas12f1-VEGFA-3(U4AU6) | | | | TTTTATT TTTT | 301 |

In the above table, for the DNA sequence encoding crRNA for each target nucleic acid, a crRNA common sequence - a protospacer sequence - a U-rich tail sequence are linked in the 5'-to-3' direction. The plasmid vector sequence which expresses the crRNA is a plasmid vector sequence in which a U6 promoter is operably linked to the DNA sequence encoding the crRNA.

**[Table 30]**

| Primer sequences used in Experimental Example 12 | | | | |
|---|---|---|---|---|
| Label | Target nucleic acid | Forward primer (5' to 3') | Reverse primer (5' to 3') | SEQ ID NO |
| tracrRNA | - | | TTGTGCAGAATTGGAG AGGA | 302 |
| SpCas12f1-DYtarget2(WT) | DYtarget2 | | GGTAGCCCACTGTGTG TGTGgtttcaatcca | 303 |
| SpCas12f1-DYtarget2(U6) | | | aaaaaaGGTAGCCCACTG TGTGTGTGgtttcaatcca | 304 |
| SpCas12f1-DYtarget2(U4AU6) | | GAGGGCCTAT TTCCCATGAT T (SEQ ID NO: 183) | aaaaaataaaaGGTAGCCCA CTGTGTGTGTGgtttcaatcc a | 305 |
| SpCas12f1-VEGFA-3(WT) | VEGFA-3 | | CGCGCTCCGGCGGTCA CCCCgtttcaatcca | 306 |
| SpCas12f1-VEGFA-3(U6) | | | aaaaaaCGCGCTCCGGCG GTCACCCCgtttcaatcca | 307 |
| SpCas12f1-VEGFA-3(U4AU6) | | | aaaaaataaaaCGCGCTCCG GCGGTCACCCCgtttcaatc ca | 308 |
| AsCas12f1-DYtarget2(WT) | DYtarget2 | | GGTAGCCCACTGTGTG TGTGgttcacactcc | 309 |
| AsCas12f1-DYtarget2(U6) | | | aaaaaaGGTAGCCCACTG TGTGTGTGgttcacactcc | 310 |
| AsCas12f1-DYtarget2(U4AU6) | | | aaaaaataaaaGGTAGCCCA CTGTGTGTGTGgttcacact cc | 311 |
| AsCas12f1-VEGFA-3(WT) | VEGFA-3 | | CGCGCTCCGGCGGTCA CCCCgttcacactcc | 312 |
| AsCas12f1-VEGFA-3(U6) | | | aaaaaaCGCGCTCCGGCG GTCACCCCgttcacactcc | 313 |
| AsCas12f1-VEGFA-3(U4AU6) | | | aaaaaataaaaCGCGCTCCG GCGGTCACCCCgttcacact cc | 314 |

3) HEK-293T cells cultured according to Experimental Example 1-8 were transfected with plasmid vectors including nucleic acid sequences encoding the SpCas12f1 protein, the AsCas12f1 protein, a plasmid vector of the tracrRNA, and a plasmid vector of the crRNA by the method disclosed in Experimental Example 1-9.

4) The indel efficiencies for the target nucleic acids of each example were analyzed by the methods disclosed in Experimental Examples 1-12 to 1-14. The indel efficiency analysis results are shown in Table 31.

**[Table 31]**

| Indel efficiency analysis results of Experimental Example 12 | | |
|---|---|---|
| Label | Target nucleic acid | Indel efficiency (%) |
| SpCas12f1-DYtarget2(WT) | DYtarget2 | 0.00 |
| SpCas12f1-DYtarget2(U6) | | 0.06 |
| SpCas12f1-DYtarget2(U4AU6) | | 0.07 |
| SpCas 12f1-VEGFA-3(WT) | VEGFA-3 | 0.00 |
| SpCas12f1-VEGFA-3(U6) | | 0.00 |
| SpCas 12f1-VEGFA-3(U4AU6) | | 0.00 |
| AsCas12f1-DYtarget2(WT) | DYtarget2 | 0.00 |
| AsCas12f1-DYtarget2(U6) | | 0.06 |
| AsCas12f1-DYtarget2(U4AU6) | | 0.03 |
| AsCas12f1-VEGFA-3(WT) | VEGFA-3 | 0.00 |
| AsCas12f1-VEGFA-3(U6) | | 0.01 |
| AsCas12f1-VEGFA-3(U4AU6) | | 0.00 |

As a result of the experiment, no significant indel efficiency was observed in either the engineered CRISPR/SpCas12f1 system into which the U-rich tail sequence has been introduced or the engineered CRISPR/AsCas12f1 system. In conclusion, although it was confirmed that SpCas12f1 and AsCas12f1 proteins are expressed in eukaryotic cells (FIG. 9), no significant indel efficiency has been shown, so that it can be seen that even when a U-rich tail is introduced into the CRISPR/SpCas12f1 system and the CRISPR/AsCas12f1 system, gene cleavage activity is not affected.

### [Industrial Applicability]

The present disclosure provides a CRISPR/Cas12f1 system which can be used for a gene editing technique, and particularly, an engineered CRISPR/Cas12f1 system of which gene editing efficiency is improved by the introduction of a U-rich tail sequence.

When used for gene editing, the engineered CRISPR/Cas12f1 system having the U-rich tail sequence according to the present disclosure exhibits higher gene editing efficiency than a wild-type CRISPR/Cas12f1 system.

## Claims

1. An engineered CRISPR RNA (crRNA) for a CRISPR/Cas12f1 system capable of editing a nucleic acid including a target sequence, the engineered crRNA comprises;
a spacer sequence which is complementary to the target sequence; and
a U-rich tail sequence which is linked to 3'-terminus of the spacer sequence,
wherein the U-rich tail sequence is represented by (UₐN)ₙU_{b}, N is one of adenosine (A), uracil (U), cytidine (C), and guanosine (G),
a is an integer between 1 to 4 inclusive, n is an integer selected from 0, 1, and 2, and b is an integer between 1 to 10 inclusive, and
the sequence of the engineered crRNA includes the crRNA repeat sequence, the spacer sequence, and the U-rich tail sequence, which are linked sequentially in a 5'-to-3' direction thereof.

2. A DNA having a sequence encoding the engineered crRNA of claim 1.

3. The engineered crRNA of claim 1, wherein the U-rich tail sequence is UUUUUU, UUUUAUUUUUU, or UUUUGUUUUUU.

4. The engineered crRNA of claim 1, wherein the crRNA repeat sequence has a sequence of SEQ ID NO:58.

5. An engineered guide RNA for CRISPR/Cas12f1 system capable of editing a nucleic acid including a target sequence, having a sequence comprising:
a tracrRNA sequence;
a crRNA sequence including a CRISPR RNA repeat sequence and a spacer sequence which is complementary to the target sequence; and
a U-rich tail sequence which is linked to 3'-terminus of the crRNA sequence wherein the U-rich tail sequence is represented by (UₐN)ₙU_{b}, N is one of adenosine (A), uracil (U), cytidine (C), and guanosine (G), and a is an integer between 1 to 4 inclusive, n is an integer selected from 0, 1, and 2, and b is an integer between 1 to 10 inclusive.

6. The engineered guide RNA of claim 5, wherein the engineered guide RNA further comprises a linker sequence, wherein the tracrRNA sequence and the crRNA sequence are linked via the linker sequence.

7. The engineered guide RNA of claim 6, wherein the linker sequence is 5'-gaaa-3'.

8. The engineered guide RNA of claim 6, wherein the U-rich tail sequence is UUUUUU, UUUUAUUUUUU, or UUUUGUUUUUU

9. A DNA having a sequence encoding the engineered guide RNA of claim 5 or claim 6.

10. An engineered CRISPR/Cas12f1 complex capable of editing a nucleic acid including a target sequence, the engineered CRISPR/Cas12f1 complex comprising:
a Cas12f1 protein belonging to a Cas14 family; and
an engineered guide RNA comprising
a scaffold sequence which interacts with the Cas12f1 protein,
a spacer sequence which is complementary to the target sequence, and
a U-rich tail sequence, wherein
the U-rich tail sequence is represented by (UₐN)ₙU_{b},
N is one of adenosine (A), uracil (U), cytidine (C), and guanosine (G), and
a is an integer between 1 to 4 inclusive, n is an integer selected from 0, 1, and 2, and b is an integer between 1 to 10 inclusive.

11. A vector for expressing an engineered CRISPR/Cas12f1 complex capable of editing a nucleic acid including a target sequence, the vector comprising:
a first sequence including a sequence that encodes a Cas12f1 protein;
a first promoter sequence operably linked to the first sequence;
a second sequence including a sequence that encodes an engineered guide RNA, wherein the engineered guide RNA has a scaffold sequence which interacts with the Cas12f1 protein, a spacer sequence which is complementary to the target sequence, and a U-rich tail sequence which is linked to a 3'-terminus of the spacer sequence, the U-rich tail sequence is represented by (UₐN)ₙU_{b}, and N is one of adenosine (A), uracil (U), cytidine (C), and guanosine (G), and a is an integer between 1 to 4 inclusive, n is an integer selected from 0, 1, and 2, and b is an integer between 1 to 10 inclusive; and
a second promoter sequence operably linked to the second sequence,
wherein the vector is constructed to express the Cas12f1 protein and the engineered guide RNA, so that the Cas12f1 protein and the engineered guide sequence forms the CRISPR/Cas12f1 complex in the cell, and a nucleic acid including the target sequence is edited by the CRISPR/Cas12f1 complex.

12. The vector of claim 11, wherein the second promoter sequence is a U6 promoter sequence.

13. The vector of claim 11, wherein the vector is a viral vector.

14. The vector of claim 11, wherein the vector is a plasmid vector.

15. The vector of claim 13, wherein the vector is one or more selected from the group consisting of a retrovirus, a lentivirus, an adenovirus, an adeno-associated virus (AAV), a vaccinia virus, a poxvirus and a herpes simplex virus.

16. The vector of claim 11, wherein the vector is linear PCR amplicon.

17. A vector for expressing an engineered CRISPR/Cas12f1 complex capable of editing a nucleic acid including a first target sequence and a second target sequence, the vector comprising:
a first sequence including a sequence that encodes a Cas12f1 protein;
a first promoter sequence operably linked to the first sequence;
a second sequence including a sequence that encodes a first engineered guide RNA, wherein the first engineered guide RNA has a first scaffold sequence which interacts with the Cas12f1 protein, a first spacer sequence which is complementary to the first target sequence, and a first U-rich tail sequence, wherein the first U-rich tail sequence is represented by (UₐN)ₙU_{b}, N is one of adenosine (A), uracil (U), cytidine (C), and guanosine (G), and a is an integer between 1 to 4 inclusive, n is an integer selected from 0, 1, and 2, and b is an integer between 1 to 10 inclusive;
a second promoter sequence operably linked to the second sequence;
a third sequence including a sequence that encodes a second engineered guide RNA, wherein the second engineered guide RNA has a second scaffold sequence which interacts with the Cas12f1 protein, a second spacer sequence which is complementary to the second target sequence, and a second U-rich tail sequence, wherein the second U-rich tail sequence is represented by (UₐN)ₙU_{b}, N is one of adenosine (A), uracil (U), cytidine (C), and guanosine (G), and a is an integer between 1 to 4 inclusive, n is an integer selected from 0, 1, and 2, and b is an integer between 1 to 10 inclusive; and a third promoter sequence operably linked to the third sequence.

18. The vector of claim 17, wherein the second promoter sequence and the third promoter sequence are the same promoter sequence.

19. The vector of claim 17, wherein the second promoter sequence is a HI promoter sequence, and the third promoter sequence is a U6 promoter sequence.

20. A method for editing a nucleic acid including a target sequence, in the cell, the method comprises:
delivering a Cas12f1 protein or a nucleic acid encoding the Cas12f1 protein, and an engineered guide RNA or a nucleic acid encoding the engineered guide RNA, into a cell, so that
a CRISPR/Cas12f1 complex is formed in the cell, and
the nucleic acid including the target sequence is edited by the CRISPR/Cas12f1 complex, wherein the engineered guide RNA comprises a scaffold sequence which interacts with the Cas12f1 protein, a spacer sequence which is complementary to the target sequence, and a U-rich tail sequence which is linked to 3'-terminus of the spacer sequence, wherein the U-rich tail sequence is represented by (UₐN)ₙU_{b}, N is one of adenosine (A), uracil (U), cytidine (C), and guanosine (G), and a is an integer between 1 to 4 inclusive, n is an integer selected from 0, 1, and 2, and b is an integer between 1 to 10 inclusive.

21. The method of claim 20, wherein in the delivering, the Cas12f1 protein and the engineered guide RNA are introduced in the form of a CRISPR/Cas12f1 complex into the cell.

22. The method of claim 20, wherein in the delivering, a vector including the nucleic acid encoding the Cas12f1 protein and the nucleic acid encoding the engineered guide RNA is introduced into the cell.

23. The method of claim 20, wherein the vector is one or more selected from the group consisting of a retrovirus, a lentivirus, an adenovirus, an adeno-associated virus (AAV), a vaccinia virus, a poxvirus, and a herpes simplex virus.

24. A method for editing a nucleic acid including a target sequence, in a cell, the method comprises:
bringing a CRISPR/Cas12f1 complex in contact with the nucleic acid including the target sequence, wherein
the CRISPR/Cas12f1 complex comprises a Cas12f1 protein and an engineered guide RNA,
the engineered guide RNA comprises a scaffold sequence which interacts with the Cas12f1 protein, a spacer sequence which is complementary to the target sequence, and a U-rich tail sequence, wherein the U-rich tail sequence is represented by (UₐN)ₙU_{b}, N is one of adenosine (A), uracil (U), cytidine (C), and guanosine (G), and a is an integer between 1 to 4 inclusive, n is an integer selected from 0, 1, and 2, and b is an integer between 1 to 10 inclusive.


<110>     GenKOre

<120>     An engineered guide RNA for CRISPR/Cas12f1 system and use thereof

<130>     CP20-201

<160>     325

<170>     KoPatentIn 3.0

<210>     1
<211>     1680
<212>     DNA
<213>     Artificial Sequence

<220>
<223>     Human codon-optimized Cas14a1


<400>     1
ccaaagaaga agcggaaggt cggtatccac ggagtcccag cagccatggc caagaacaca          60

attacaaaga cactgaagct gaggatcgtg agaccataca acagcgctga ggtcgagaag         120

attgtggctg atgaaaagaa caacagggaa aagatcgccc tcgagaagaa caaggataag         180

gtgaaggagg cctgctctaa gcacctgaaa gtggccgcct actgcaccac acaggtggag         240

aggaacgcct gtctgttttg taaagctcgg aagctggatg ataagtttta ccagaagctg         300

cggggccagt tccccgatgc cgtcttttgg caggagatta gcgagatctt cagacagctg         360

cagaagcagg ccgccgagat ctacaaccag agcctgatcg agctctacta cgagatcttc         420

atcaagggca agggcattgc caacgcctcc tccgtggagc actacctgag cgacgtgtgc         480

tacacaagag ccgccgagct ctttaagaac gccgctatcg cttccgggct gaggagcaag         540

attaagagta acttccggct caaggagctg aagaacatga agagcggcct gcccactaca         600

aagagcgaca acttcccaat tccactggtg aagcagaagg ggggccagta cacagggttc         660

gagatttcca accacaacag cgactttatt attaagatcc cctttggcag gtggcaggtc         720

aagaaggaga ttgacaagta caggccctgg gagaagtttg atttcgagca ggtgcagaag         780

agccccaagc ctatttccct gctgctgtcc acacagcggc ggaagaggaa caaggggtgg         840

tctaaggatg aggggaccga ggccgagatt aagaaagtga tgaacggcga ctaccagaca         900

agctacatcg aggtcaagcg gggcagtaag attggcgaga agagcgcctg gatgctgaac         960

ctgagcattg acgtgccaaa gattgataag ggcgtggatc ccagcatcat cggagggatc       1020

gatgtggggg tcaagagccc cctcgtgtgc gccatcaaca acgccttcag caggtacagc        1080

atctccgata acgacctgtt ccactttaac aagaagatgt tcgcccggcg gaggattttg        1140

ctcaagaaga accggcacaa gcgggccgga cacggggcca agaacaagct caagcccatc        1200

actatcctga ccgagaagag cgagaggttc aggaagaagc tcatcgagag atgggcctgc        1260

gagatcgccg atttctttat taagaacaag gtcggaacag tgcagatgga gaacctcgag        1320

agcatgaaga ggaaggagga ttcctacttc aacattcggc tgagggggtt ctggccctac 1380

gctgagatgc agaacaagat tgagtttaag ctgaagcagt acgggattga gatccggaag 1440

gtggccccca acaacaccag caagacctgc agcaagtgcg ggcacctcaa caactacttc 1500

aacttcgagt accggaagaa gaacaagttc ccacacttca agtgcgagaa gtgcaacttt 1560

aaggagaacg ccgattacaa cgccgccctg aacatcagca accctaagct gaagagcact 1620

aaggaggagc ccaaaaggcc ggcggccacg aaaaaggccg gccaggcaaa aaagaaaaag 1680

1680

<210> 2
<211> 3009
<212> DNA
<213> Artificial Sequence

<220>
<223> Deaminase fusion Cas14a1

<400> 2
ccaaagaaga agcggaaagt ctcctcagag actgggcctg tcgccgtcga tccaaccctg 60

cgccgccgga ttgaacctca cgagtttgaa gtgttctttg acccccggga gctgagaaag 120

gagacatgcc tgctgtacga gatcaactgg ggaggcaggc actccatctg gaggcacacc 180

tctcagaaca caaataagca cgtggaggtg aacttcatcg agaagtttac cacagagcgg 240

tacttctgcc ccaataccag atgtagcatc acatggtttc tgagctggtc cccttgcgga 300

gagtgtagca gggccatcac cgagttcctg tccagatatc cacacgtgac actgtttatc 360

tacatcgcca ggctgtatca ccacgcagac ccaaggaata ggcagggcct gcgcgatctg 420

atcagctccg gcgtgaccat ccagatcatg acagagcagg agtccggcta ctgctggcgg 480

aacttcgtga attattctcc tagcaacgag gcccactggc ctaggtaccc acacctgtgg 540

gtgcgcctgt acgtgctgga gctgtattgc atcatcctgg gcctgccccc ttgtctgaat 600

atcctgcgga gaaagcagcc ccagctgacc ttctttacaa tcgccctgca gtcttgtcac 660

tatcagaggc tgccacccca catcctgtgg gccacaggcc tgaagtctgg aggatctagc 720

ggaggatcct ctggcagcga gacaccagga acaagcgagt cagcaacacc agagagcagt 780

ggcggcagca gcggcggcag catggccaag aacacaatta caaagacact gaagctgagg 840

atcgtgagac catacaacag cgctgaggtc gagaagattg tggctgatga aaagaacaac 900

agggaaaaga tcgccctcga gaagaacaag gataaggtga aggaggcctg ctctaagcac 960

ctgaaagtgg ccgcctactg caccacacag gtggagagga acgcctgtct gttttgtaaa 1020

gctcggaagc tggatgataa gttttaccag aagctgcggg gccagttccc cgatgccgtc 1080

ttttggcagg agattagcga gatcttcaga cagctgcaga agcaggccgc cgagatctac 1140

aaccagagcc tgatcgagct ctactacgag atcttcatca agggcaaggg cattgccaac 1200

gcctcctccg tggagcacta cctgagcgac gtgtgctaca caagagccgc cgagctcttt 1260

aagaacgccg ctatcgcttc cgggctgagg agcaagatta agagtaactt ccggctcaag 1320

gagctgaaga acatgaagag cggcctgccc actacaaaga gcgacaactt cccaattcca 1380

ctggtgaagc agaagggggg ccagtacaca gggttcgaga tttccaacca caacagcgac 1440

tttattatta agatcccctt tggcaggtgg caggtcaaga aggagattga caagtacagg 1500

ccctgggaga agtttgattt cgagcaggtg cagaagagcc ccaagcctat ttccctgctg 1560

ctgtccacac agcggcggaa gaggaacaag gggtggtcta aggatgaggg gaccgaggcc 1620

gagattaaga aagtgatgaa cggcgactac cagacaagct acatcgaggt caagcggggc 1680

agtaagattg gcgagaagag cgcctggatg ctgaacctga gcattgacgt gccaaagatt 1740

gataagggcg tggatcccag catcatcgga gggatcgatg tgggggtcaa gagccccctc 1800

gtgtgcgcca tcaacaacgc cttcagcagg tacagcatct ccgataacga cctgttccac 1860

tttaacaaga agatgttcgc ccggcggagg attttgctca agaagaaccg gcacaagcgg 1920

gccggacacg gggccaagaa caagctcaag cccatcacta tcctgaccga gaagagcgag 1980

aggttcagga agaagctcat cgagagatgg gcctgcgaga tcgccgattt ctttattaag 2040

aacaaggtcg gaacagtgca gatggagaac ctcgagagca tgaagaggaa ggaggattcc 2100

tacttcaaca ttcggctgag ggggttctgg ccctacgctg agatgcagaa caagattgag 2160

tttaagctga agcagtacgg gattgagatc cggaaggtgg cccccaacaa caccagcaag 2220

acctgcagca agtgcgggca cctcaacaac tacttcaact tcgagtaccg gaagaagaac 2280

aagttcccac acttcaagtg cgagaagtgc aactttaagg agaacgccga ttacaacgcc 2340

gccctgaaca tcagcaaccc taagctgaag agcactaagg aggagcccag cggcgggagc 2400

ggcgggagcg gggggagcac taatctgagc gacatcattg agaaggagac tgggaaacag 2460

ctggtcattc aggagtccat cctgatgctg cctgaggagg tggaggaagt gatcggcaac 2520

aagccagagt ctgacatcct ggtgcacacc gcctacgacg agtccacaga tgagaatgtg 2580

atgctgctga cctctgacgc ccccgagtat aagccttggg ccctggtcat ccaggattct 2640

aacggcgaga ataagatcaa gatgctgagc ggaggatccg gaggatctgg aggcagcacc 2700

aacctgtctg acatcatcga gaaggagaca ggcaagcagc tggtcatcca ggagagcatc 2760

ctgatgctgc ccgaagaagt cgaagaagtg atcggaaaca agcctgagag cgatatcctg 2820

gtccataccg cctacgacga gagtaccgac gaaaatgtga tgctgctgac atccgacgcc 2880

ccagagtata agccctgggc tctggtcatc caggattcca acggagagaa caaaatcaaa 2940

atgctgtctg gcggctcaaa aagaaccgcc gacggcagcg aattcgagcc caagaagaag 3000

aggaaagtc                                                                 3009

<210>    3
<211>    10
<212>    RNA
<213>    Artificial Sequence

<220>
<223>    U-rich tail sequence

<400>    3
uuuuuuuuuu                                                                10

<210>    4
<211>    10
<212>    RNA
<213>    Artificial Sequence

<220>
<223>    U-rich tail sequence

<400>    4
uuuuauuuuu                                                                10

<210>    5
<211>    11
<212>    RNA
<213>    Artificial Sequence

<220>
<223>    U-rich tail sequence

<400>    5
uuuuauuuuu u                                                              11

<210>    6
<211>    12
<212>    RNA
<213>    Artificial Sequence

<220>
<223>    U-rich tail sequence

<400>    6
uuuuauuuuu uu                                                             12

<210>    7
<211>    13
<212>    RNA
<213>    Artificial Sequence

<220>
<223>    U-rich tail sequence

<400>    7
uuuuauuuuu uuu                                                          13


<210>    8
<211>    14
<212>    RNA
<213>    Artificial Sequence

<220>
<223>    U-rich tail sequence


<400>    8
uuuuauuuuu uuuu                                                         14


<210>    9
<211>    15
<212>    RNA
<213>    Artificial Sequence

<220>
<223>    U-rich tail sequence


<400>    9
uuuuauuuuu uuuuu                                                        15


<210>    10
<211>    10
<212>    RNA
<213>    Artificial Sequence

<220>
<223>    U-rich tail sequence


<400>    10
uuuauuuauu                                                              10


<210>    11
<211>    11
<212>    RNA
<213>    Artificial Sequence

<220>
<223>    U-rich tail sequence


<400>    11
uuuauuuauu u                                                            11


<210>    12
<211>    12
<212>    RNA
<213>    Artificial Sequence

<220>

<223>    U-rich tail sequence

<400>    12
uuuauuuauu uu                                                          12

<210>    13
<211>    13
<212>    RNA
<213>    Artificial Sequence

<220>
<223>    U-rich tail sequence

<400>    13
uuuauuuauu uuu                                                         13

<210>    14
<211>    14
<212>    RNA
<213>    Artificial Sequence

<220>
<223>    U-rich tail sequence

<400>    14
uuuauuuauu uuuu                                                        14

<210>    15
<211>    10
<212>    RNA
<213>    Artificial Sequence

<220>
<223>    U-rich tail sequence

<400>    15
uuuuauuuua                                                             10

<210>    16
<211>    11
<212>    RNA
<213>    Artificial Sequence

<220>
<223>    U-rich tail sequence

<400>    16
uuuuauuuua u                                                           11

<210>    17
<211>    12
<212>    RNA
<213>    Artificial Sequence

<220>
<223>    U-rich tail sequence


<400>    17
uuuuauuuua uu                                                          12


<210>    18
<211>    13
<212>    RNA
<213>    Artificial Sequence

<220>
<223>    U-rich tail sequence


<400>    18
uuuuauuuua uuu                                                         13


<210>    19
<211>    14
<212>    RNA
<213>    Artificial Sequence

<220>
<223>    U-rich tail sequence


<400>    19
uuuuauuuua uuuu                                                        14


<210>    20
<211>    15
<212>    RNA
<213>    Artificial Sequence

<220>
<223>    U-rich tail sequence


<400>    20
uuuuauuuua uuuuu                                                       15


<210>    21
<211>    16
<212>    RNA
<213>    Artificial Sequence

<220>
<223>    U-rich tail sequence


<400>    21
uuuuauuuua uuuuuu                                                      16


<210>    22
<211>    10

<212>    RNA
<213>    Artificial Sequence

<220>
<223>    U-rich tail sequence


<400>    22
uuuuguuuuu                                                              10


<210>    23
<211>    11
<212>    RNA
<213>    Artificial Sequence

<220>
<223>    U-rich tail sequence


<400>    23
uuuuguuuuu u                                                            11


<210>    24
<211>    12
<212>    RNA
<213>    Artificial Sequence

<220>
<223>    U-rich tail sequence


<400>    24
uuuuguuuuu uu                                                           12


<210>    25
<211>    13
<212>    RNA
<213>    Artificial Sequence

<220>
<223>    U-rich tail sequence


<400>    25
uuuuguuuuu uuu                                                          13


<210>    26
<211>    14
<212>    RNA
<213>    Artificial Sequence

<220>
<223>    U-rich tail sequence


<400>    26
uuuuguuuuu uuuu                                                         14

<210>    27
<211>    15
<212>    RNA
<213>    Artificial Sequence

<220>
<223>    U-rich tail sequence


<400>    27
uuuuguuuuu uuuuu                                                        15


<210>    28
<211>    10
<212>    RNA
<213>    Artificial Sequence

<220>
<223>    U-rich tail sequence


<400>    28
uuuguuuguu                                                              10


<210>    29
<211>    11
<212>    RNA
<213>    Artificial Sequence

<220>
<223>    U-rich tail sequence


<400>    29
uuuguuuguu u                                                            11


<210>    30
<211>    12
<212>    RNA
<213>    Artificial Sequence

<220>
<223>    U-rich tail sequence


<400>    30
uuuguuuguu uu                                                           12


<210>    31
<211>    13
<212>    RNA
<213>    Artificial Sequence

<220>
<223>    U-rich tail sequence


<400>    31
uuuguuuguu uuu                                                          13

<210>    32
<211>    14
<212>    RNA
<213>    Artificial Sequence

<220>
<223>    U-rich tail sequence


<400>    32
uuuguuuguu uuuu                                                          14


<210>    33
<211>    10
<212>    RNA
<213>    Artificial Sequence

<220>
<223>    U-rich tail sequence


<400>    33
uuuuguuuug                                                               10


<210>    34
<211>    11
<212>    RNA
<213>    Artificial Sequence

<220>
<223>    U-rich tail sequence


<400>    34
uuuuguuuug u                                                             11


<210>    35
<211>    12
<212>    RNA
<213>    Artificial Sequence

<220>
<223>    U-rich tail sequence


<400>    35
uuuuguuuug uu                                                            12


<210>    36
<211>    13
<212>    RNA
<213>    Artificial Sequence

<220>
<223>    U-rich tail sequence

<400>    36
uuuuguuuug uuu                                                          13


<210>    37
<211>    14
<212>    RNA
<213>    Artificial Sequence

<220>
<223>    U-rich tail sequence


<400>    37
uuuuguuuug uuuu                                                         14


<210>    38
<211>    15
<212>    RNA
<213>    Artificial Sequence

<220>
<223>    U-rich tail sequence


<400>    38
uuuuguuuug uuuuu                                                        15


<210>    39
<211>    16
<212>    RNA
<213>    Artificial Sequence

<220>
<223>    U-rich tail sequence


<400>    39
uuuuguuuug uuuuuu                                                       16


<210>    40
<211>    10
<212>    RNA
<213>    Artificial Sequence

<220>
<223>    U-rich tail sequence


<400>    40
   uuuucuuuuu                                                               10


<210>    41
<211>    11
<212>    RNA
<213>    Artificial Sequence

<220>
<223>    U-rich tail sequence

<400>    41
uuuucuuuuu u                                                            11


<210>    42
<211>    12
<212>    RNA
<213>    Artificial Sequence

<220>
<223>    U-rich tail sequence


<400>    42
uuuucuuuuu uu                                                           12


<210>    43
<211>    13
<212>    RNA
<213>    Artificial Sequence

<220>
<223>    U-rich tail sequence


<400>    43
uuuucuuuuu uuu                                                          13


<210>    44
<211>    14
<212>    RNA
<213>    Artificial Sequence

<220>
<223>    U-rich tail sequence


<400>    44
uuuucuuuuu uuuu                                                         14


<210>    45
<211>    15
<212>    RNA
<213>    Artificial Sequence

<220>
<223>    U-rich tail sequence


<400>    45
uuuucuuuuu uuuuu                                                        15


<210>    46
<211>    10
<212>    RNA
<213>    Artificial Sequence

<220>
<223>    U-rich tail sequence

<400>    46
uuucuuucuu                                                              10

<210>    47
<211>    11
<212>    RNA
<213>    Artificial Sequence

<220>
<223>    U-rich tail sequence

<400>    47
uuucuuucuu u                                                            11

<210>    48
<211>    12
<212>    RNA
<213>    Artificial Sequence

<220>
<223>    U-rich tail sequence

<400>    48
uuucuuucuu uu                                                           12

<210>    49
<211>    13
<212>    RNA
<213>    Artificial Sequence

<220>
<223>    U-rich tail sequence

<400>    49
uuucuuucuu uuu                                                          13

<210>    50
<211>    14
<212>    RNA
<213>    Artificial Sequence

<220>
<223>    U-rich tail sequence

<400>    50
uuucuuucuu uuuu                                                         14

<210>    51
<211>    10
<212>    RNA

<213>    Artificial Sequence

<220>
<223>    U-rich tail sequence


<400>    51
uuuucuuuuc                                                              10


<210>    52
<211>    11
<212>    RNA
<213>    Artificial Sequence

<220>
<223>    U-rich tail sequence


<400>    52
uuuucuuuuc u                                                            11


<210>    53
<211>    12
<212>    RNA
<213>    Artificial Sequence

<220>
<223>    U-rich tail sequence


<400>    53
uuuucuuuuc uu                                                           12


<210>    54
<211>    13
<212>    RNA
<213>    Artificial Sequence

<220>
<223>    U-rich tail sequence


<400>    54
uuuucuuuuc uuu                                                          13


<210>    55
<211>    14
<212>    RNA
<213>    Artificial Sequence

<220>
<223>    U-rich tail sequence


<400>    55
uuuucuuuuc uuuu                                                         14


<210>    56

<211>	15
<212>	RNA
<213>	Artificial Sequence

<220>
<223>	U-rich tail sequence


<400>	56
uuuucuuuuc uuuuu	15


<210>	57
<211>	16
<212>	RNA
<213>	Artificial Sequence

<220>
<223>	U-rich tail sequence


<400>	57
uuuucuuuuc uuuuuu	16


<210>	58
<211>	37
<212>	RNA
<213>	unidentified archaeon

<400>	58
guugcagaac ccgaauagac gaaugaagga augcaac	37


<210>	59
<211>	37
<212>	DNA
<213>	Artificial Sequence

<220>
<223>	CRISPR RNA Repeat sequence encoding DNA sequence for
	CRISPR/Cas14a1 system


<400>	59
gttgcagaac ccgaatagac gaatgaagga atgcaac	37


<210>	60
<211>	161
<212>	RNA
<213>	unidentified archaeon

<400>	60
cuucacugau aaaguggaga accgcuucac caaaagcugu cccuuagggg auuagaacuu	60

gagugaaggu gggcugcuug caucagccua augucgagaa gugcuuucuu cggaaaguaa	120

cccucgaaac aaauucauuu uuccucucca auucugcaca a	161


<210>	61
<211>	161

<212>    DNA
<213>    Artificial Sequence

<220>
<223>    tracrRNA sequence encoding DNA sequence for CRISPR/Cas14a1 system


<400>    61
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca a                           161


<210>    62
<211>    20
<212>    DNA
<213>    Homo sapiens

<400>    62
cacgtctcat atgccccttg                                                    20


<210>    63
<211>    20
<212>    DNA
<213>    Homo sapiens

<400>    63
cacacacaca gtgggctacc                                                    20


<210>    64
<211>    20
<212>    DNA
<213>    Homo sapiens

<400>    64
gacgtgggag acacacacac                                                    20


<210>    65
<211>    20
<212>    DNA
<213>    Homo sapiens

<400>    65
catccccagg acacacacac                                                    20


<210>    66
<211>    20
<212>    DNA
<213>    Homo sapiens

<400>    66
agcacatgtg cacacacaca                                                    20


<210>    67
<211>    20
<212>    DNA

<213>    Homo sapiens

<400>    67
agaacacata ccccctgggcc                                                20

<210>    68
<211>    20
<212>    RNA
<213>    Artificial Sequence

<220>
<223>    spacer sequence for RNF2

<400>    68
cacgucucau augccccuug                                                20

<210>    69
<211>    20
<212>    RNA
<213>    Artificial Sequence

<220>
<223>    spacer sequence for DYtarget2

<400>    69
cacacacaca gugggcuacc                                                20

<210>    70
<211>    20
<212>    RNA
<213>    Artificial Sequence

<220>
<223>    spacer sequence for DYtarget9

<400>    70
gacgugggag acacacacac                                                20

<210>    71
<211>    20
<212>    RNA
<213>    Artificial Sequence

<220>
<223>    spacer sequence for DYtarget10

<400>    71
cauccccagg acacacacac                                                20

<210>    72
<211>    20
<212>    RNA
<213>    Artificial Sequence

<220>
<223>    spacer sequence for DYtarget13

<400>    72
agcacaugug cacacacaca                                                    20

<210>    73
<211>    20
<212>    RNA
<213>    Artificial Sequence

<220>
<223>    spacer sequence for Intergenic-22

<400>    73
agaacacaua ccccugggcc                                                    20

<210>    74
<211>    202
<212>    RNA
<213>    Artificial Sequence

<220>
<223>    common sgRNA sequence except spacer sequence and U-rich tail
         sequence

<400>    74
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca ac                                                202

<210>    75
<211>    222
<212>    RNA
<213>    Artificial Sequence

<220>
<223>    sgRNA for RNF2(WT)

<400>    75
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacgucuc auaugccccu ug                          222

<210>    76
<211>    233
<212>    RNA

<213>    Artificial Sequence

<220>
<223>    sgRNA for RNF2(U4AU6)


<400>    76
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacgucuc auaugccccu uguuuuauuu uuu            233


<210>    77
<211>    222
<212>    RNA
<213>    Artificial Sequence

<220>
<223>    sgRNA for DYtarget2(WT)


<400>    77
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua cc                         222


<210>    78
<211>    223
<212>    RNA
<213>    Artificial Sequence

<220>
<223>    sgRNA for DYtarget2(U)


<400>    78
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccu                        223


<210>    79
<211>    224
<212>    RNA
<213>    Artificial Sequence

<220>
<223>    sgRNA for DYtarget2(U2)

<400>    79
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accacacaca cagugggcua ccuu                     224


<210>    80
<211>    225
<212>    RNA
<213>    Artificial Sequence

<220>
<223>    sgRNA for DYtarget2(U3)


<400>    80
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accacacaca cagugggcua ccuuu                    225


<210>    81
<211>    226
<212>    RNA
<213>    Artificial Sequence

<220>
<223>    sgRNA for DYtarget2(U4)


<400>    81
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accacacaca cagugggcua ccuuuu                   226


<210>    82
<211>    227
<212>    RNA
<213>    Artificial Sequence

<220>
<223>    sgRNA for DYtarget2(U5)


<400>    82
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accacacaca cagugggcua ccuuuuu                    227

<210>    83
<211>    228
<212>    RNA
<213>    Artificial Sequence

<220>
<223>    sgRNA for DYtarget2(U6)

<400>    83
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuuuu                   228

<210>    84
<211>    227
<212>    RNA
<213>    Artificial Sequence

<220>
<223>    sgRNA for DYtarget2(U4A)

<400>    84
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuua                    227

<210>    85
<211>    228
<212>    RNA
<213>    Artificial Sequence

<220>
<223>    sgRNA for DYtarget2(U4AU)

<400>    85
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuuau                   228

<210>    86
<211>    229

<212>    RNA
<213>    Artificial Sequence

<220>
<223>    sgRNA for DYtarget2(U4AU2)


<400>    86
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccuuuuauu                   229


<210>    87
<211>    230
<212>    RNA
<213>    Artificial Sequence

<220>
<223>    sgRNA for DYtarget2(U4AU3)


<400>    87
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccuuuuauuu                  230


<210>    88
<211>    231
<212>    RNA
<213>    Artificial Sequence

<220>
<223>    sgRNA for DYtarget2(U4AU4)


<400>    88
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccuuuuauuu u                231


<210>    89
<211>    232
<212>    RNA
<213>    Artificial Sequence

<220>
<223>    sgRNA for DYtarget2(U4AU5)

<400>    89
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuuauuu uu              232


<210>    90
<211>    233
<212>    RNA
<213>    Artificial Sequence

<220>
<223>    sgRNA for DYtarget2(U4AU6)


<400>    90
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuuauuu uuu             233


<210>    91
<211>    234
<212>    RNA
<213>    Artificial Sequence

<220>
<223>    sgRNA for DYtarget2(U4AU7)


<400>    91
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccuuuuauuu uuuu            234


<210>    92
<211>    235
<212>    RNA
<213>    Artificial Sequence

<220>
<223>    sgRNA for DYtarget2(U4AU8)


<400>    92
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa 180

uagacgaaug aaggaaugca accacacaca cagugggcua ccuuuuauuu uuuuu 235

<210> 93
<211> 236
<212> RNA
<213> Artificial Sequence

<220>
<223> sgRNA for DYtarget2(U4AU9)

<400> 93
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accacacaca cagugggcua ccuuuuauuu uuuuuu 236

<210> 94
<211> 237
<212> RNA
<213> Artificial Sequence

<220>
<223> sgRNA for DYtarget2(U4AU10)

<400> 94
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accacacaca cagugggcua ccuuuuauuu uuuuuuu 237

<210> 95
<211> 232
<212> RNA
<213> Artificial Sequence

<220>
<223> sgRNA for DYtarget2(U3AU6)

<400> 95
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accacacaca cagugggcua ccuuuauuuu uu 232

<210> 96

<211>    236
<212>    RNA
<213>    Artificial Sequence

<220>
<223>    sgRNA for DYtarget2(U3AU3AU6)


<400>    96
cuucacugau aaaguggaga accgcuucac caaaagcugu cccuuagggg auuagaacuu      60

gagugaaggu gggcugcuug caucagccua augucgagaa gugcuuucuu cggaaaguaa      120

cccucgaaac aaauucauuu uuccucucca auucugcaca agaaaguugc agaacccgaa      180

uagacgaaug aaggaaugca accacacaca cagugggcua ccuuuauuua uuuuuu      236


<210>    97
<211>    57
<212>    RNA
<213>    Artificial Sequence

<220>
<223>    crRNA for DYtarget2(WT)


<400>    97
guugcagaac ccgaauagac gaaugaagga augcaaccac acacacagug ggcuacc      57


<210>    98
<211>    68
<212>    RNA
<213>    Artificial Sequence

<220>
<223>    crRNA for DYtarget2(U4AU6)


<400>    98
guugcagaac ccgaauagac gaaugaagga augcaaccac acacacagug ggcuaccuuu      60

uauuuuuu      68


<210>    99
<211>    231
<212>    RNA
<213>    Artificial Sequence

<220>
<223>    sgRNA for DYtarget2(spacer 18nt, U4AU6)


<400>    99
cuucacugau aaaguggaga accgcuucac caaaagcugu cccuuagggg auuagaacuu      60

gagugaaggu gggcugcuug caucagccua augucgagaa gugcuuucuu cggaaaguaa      120

cccucgaaac aaauucauuu uuccucucca auucugcaca agaaaguugc agaacccgaa      180

uagacgaaug aaggaaugca accacacaca cagugggcua uuuuauuuuu u      231

<210>    100
<211>    232
<212>    RNA
<213>    Artificial Sequence

<220>
<223>    sgRNA for DYtarget2(spacer 19nt, U4AU6)


<400>    100
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua cuuuuauuuu uu                232


<210>    101
<211>    234
<212>    RNA
<213>    Artificial Sequence

<220>
<223>    sgRNA for DYtarget2(spacer 21nt, U4AU6)


<400>    101
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccauuuuauu uuuu              234


<210>    102
<211>    238
<212>    RNA
<213>    Artificial Sequence

<220>
<223>    sgRNA for DYtarget2(spacer 25nt, U4AU6)


<400>    102
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccauuuauuu uauuuuuu          238


<210>    103
<211>    243
<212>    RNA
<213>    Artificial Sequence

<220>
<223>    sgRNA for DYtarget2(spacer 30nt, U4AU6)


<400>    103
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accacacaca cagugggcua ccauuuaagg aguuuuauuu     240

uuu                                                                   243


<210>    104
<211>    222
<212>    RNA
<213>    Artificial Sequence

<220>
<223>    sgRNA for DYtarget9(WT)


<400>    104
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgacguggg agacacacac ac                        222


<210>    105
<211>    233
<212>    RNA
<213>    Artificial Sequence

<220>
<223>    sgRNA for DYtarget9(U4AU6)


<400>    105
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgacguggg agacacacac acuuuuauuu uuu            233


<210>    106
<211>    222
<212>    RNA
<213>    Artificial Sequence

<220>
<223>    sgRNA for DYtarget10(WT)

<400>    106
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accaucccca ggacacacac ac                        222


<210>    107
<211>    233
<212>    RNA
<213>    Artificial Sequence

<220>
<223>    sgRNA for DYtarget10(U4AU6)


<400>    107
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accaucccca ggacacacac acuuuuauuu uuu            233


<210>    108
<211>    228
<212>    RNA
<213>    Artificial Sequence

<220>
<223>    sgRNA for DYtarget10(U6)


<400>    108
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca accaucccca ggacacacac acuuuuuu                  228


<210>    109
<211>    232
<212>    RNA
<213>    Artificial Sequence

<220>
<223>    sgRNA for DYtarget10(U3AU6)


<400>    109
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa 180

uagacgaaug aaggaaugca accaucccca ggacacacac acuuuauuuu uu 232

<210> 110
<211> 236
<212> RNA
<213> Artificial Sequence

<220>
<223> sgRNA for DYtarget10(U3AU3AU6)

<400> 110
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accaucccca ggacacacac acuuuauuua uuuuuu 236

<210> 111
<211> 57
<212> RNA
<213> Artificial Sequence

<220>
<223> crRNA for DYtarget10(WT)

<400> 111
guugcagaac ccgaauagac gaaugaagga augcaaccau ccccaggaca cacacac 57

<210> 112
<211> 68
<212> RNA
<213> Artificial Sequence

<220>
<223> crRNA for DYtarget10(U4AU6)

<400> 112
guugcagaac ccgaauagac gaaugaagga augcaaccau ccccaggaca cacacacuuu 60

uauuuuuu 68

<210> 113
<211> 230
<212> RNA
<213> Artificial Sequence

<220>
<223> sgRNA for DYtarget10(spacer 17nt, U4AU6)

<400> 113
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accaucccca ggacacacau uuuauuuuuu 230

<210> 114
<211> 230
<212> RNA
<213> Artificial Sequence

<220>
<223> sgRNA for DYtarget10(spacer 18nt, U4AU6)

<400> 114
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accaucccca ggacacacau uuuauuuuuu 230

<210> 115
<211> 232
<212> RNA
<213> Artificial Sequence

<220>
<223> sgRNA for DYtarget10(spacer 19nt, U4AU6)

<400> 115
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accaucccca ggacacacac auuuuauuuu uu 232

<210> 116
<211> 234
<212> RNA
<213> Artificial Sequence

<220>
<223> sgRNA for DYtarget10(spacer 21nt, U4AU6)

<400> 116
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accaucccca ggacacacac acauuuuauu uuuu 234

<210>    117
<211>    238
<212>    RNA
<213>    Artificial Sequence

<220>
<223>    sgRNA for DYtarget10(spacer 25nt, U4AU6)


<400>    117
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accaucccca ggacacacac acauuucuuu uauuuuuu    238


<210>    118
<211>    243
<212>    RNA
<213>    Artificial Sequence

<220>
<223>    sgRNA for DYtarget10(spacer 30nt, U4AU6)


<400>    118
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca accaucccca ggacacacac acauuucaaa aguuuuauuu    240

uuu                                                                  243


<210>    119
<211>    222
<212>    RNA
<213>    Artificial Sequence

<220>
<223>    sgRNA for DYtarget13(WT)


<400>    119
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca acagcacaug ugcacacaca ca                       222


<210>    120
<211>    233

<212>    RNA
<213>    Artificial Sequence

<220>
<223>    sgRNA for DYtarget13(U4AU6)


<400>    120
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca acagcacaug ugcacacaca cauuuuauuu uuu              233


<210>    121
<211>    222
<212>    RNA
<213>    Artificial Sequence

<220>
<223>    sgRNA for Intergenic-22(WT)


<400>    121
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca acagaacaca uaccccuggg cc                          222


<210>    122
<211>    228
<212>    RNA
<213>    Artificial Sequence

<220>
<223>    sgRNA for Intergenic-22(U6)


<400>    122
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca acagaacaca uaccccuggg ccuuuuuu                    228


<210>    123
<211>    233
<212>    RNA
<213>    Artificial Sequence

<220>
<223>    sgRNA for Intergenic-22(U4AU6)

<400>    123
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acagaacaca uaccccuggg ccuuuuauuu uuu            233


<210>    124
<211>    57
<212>    RNA
<213>    Artificial Sequence

<220>
<223>    crRNA for Intergenic-22(WT)


<400>    124
guugcagaac ccgaauagac gaaugaagga augcaacaga acacauaccc cugggcc         57


<210>    125
<211>    68
<212>    RNA
<213>    Artificial Sequence

<220>
<223>    crRNA for Intergenic-22(U4AU6)


<400>    125
guugcagaac ccgaauagac gaaugaagga augcaacaga acacauaccc cugggccuuu      60

uauuuuuu                                                               68


<210>    126
<211>    202
<212>    DNA
<213>    Artificial Sequence

<220>
<223>    common sgRNA encoding DNA sequence except spacer sequence and
         U-rich tail sequence


<400>    126
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca ac                                              202


<210>    127
<211>    222
<212>    DNA
<213>    Artificial Sequence

<220>
<223>    sgRNA encoding DNA sequence, targeting RNF2(WT)


<400>    127
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacgtctc atatgcccct tg                        222


<210>    128
<211>    233
<212>    DNA
<213>    Artificial Sequence

<220>
<223>    sgRNA encoding DNA sequence, targeting RNF2(U4AU6)


<400>    128
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacgtctc atatgcccct tgttttattt ttt            233


<210>    129
<211>    222
<212>    DNA
<213>    Artificial Sequence

<220>
<223>    sgRNA encoding DNA sequence, targeting DYtarget2(WT)


<400>    129
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta cc                        222


<210>    130
<211>    223
<212>    DNA
<213>    Artificial Sequence

<220>
<223>    sgRNA encoding DNA sequence, targeting DYtarget2(U)


<400>    130

cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta cct 223

<210> 131
<211> 224
<212> DNA
<213> Artificial Sequence

<220>
<223> sgRNA encoding DNA sequence, targeting DYtarget2(U2)

<400> 131
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta cctt 224

<210> 132
<211> 225
<212> DNA
<213> Artificial Sequence

<220>
<223> sgRNA encoding DNA sequence, targeting DYtarget2(U3)

<400> 132
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta ccttt 225

<210> 133
<211> 226
<212> DNA
<213> Artificial Sequence

<220>
<223> sgRNA encoding DNA sequence, targeting DYtarget2(U4)

<400> 133
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta cctttt 226

<210> 134
<211> 227
<212> DNA
<213> Artificial Sequence

<220>
<223> sgRNA encoding DNA sequence, targeting DYtarget2(U5)

<400> 134
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta ccttttt 227

<210> 135
<211> 228
<212> DNA
<213> Artificial Sequence

<220>
<223> sgRNA encoding DNA sequence, targeting DYtarget2(U6)

<400> 135
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta cctttttt 228

<210> 136
<211> 227
<212> DNA
<213> Artificial Sequence

<220>
<223> sgRNA encoding DNA sequence, targeting DYtarget2(U4A)

<400> 136
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta cctttta 227

<210> 137
<211> 228
<212> DNA

<213>    Artificial Sequence

<220>
<223>    sgRNA encoding DNA sequence, targeting DYtarget2(U4AU)


<400>    137
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccttttat                  228


<210>    138
<211>    229
<212>    DNA
<213>    Artificial Sequence

<220>
<223>    sgRNA encoding DNA sequence, targeting DYtarget2(U4AU2)


<400>    138
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccttttatt                 229


<210>    139
<211>    230
<212>    DNA
<213>    Artificial Sequence

<220>
<223>    sgRNA encoding DNA sequence, targeting DYtarget2(U4AU3)


<400>    139
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccttttattt                230


<210>    140
<211>    231
<212>    DNA
<213>    Artificial Sequence

<220>
<223>    sgRNA encoding DNA sequence, targeting DYtarget2(U4AU4)

<400>    140
cttcactgat aaagtggaga accgcttcac caaaagctgt cccttagggg attagaactt    60

gagtgaaggt gggctgcttg catcagccta atgtcgagaa gtgctttctt cggaaagtaa    120

ccctcgaaac aaattcattt ttcctctcca attctgcaca agaaagttgc agaacccgaa    180

tagacgaatg aaggaatgca accacacaca cagtgggcta ccttttattt t    231


<210>    141
<211>    232
<212>    DNA
<213>    Artificial Sequence

<220>
<223>    sgRNA encoding DNA sequence, targeting DYtarget2(U4AU5)


<400>    141
cttcactgat aaagtggaga accgcttcac caaaagctgt cccttagggg attagaactt    60

gagtgaaggt gggctgcttg catcagccta atgtcgagaa gtgctttctt cggaaagtaa    120

ccctcgaaac aaattcattt ttcctctcca attctgcaca agaaagttgc agaacccgaa    180

tagacgaatg aaggaatgca accacacaca cagtgggcta ccttttattt tt    232


<210>    142
<211>    233
<212>    DNA
<213>    Artificial Sequence

<220>
<223>    sgRNA encoding DNA sequence, targeting DYtarget2(U4AU6)


<400>    142
cttcactgat aaagtggaga accgcttcac caaaagctgt cccttagggg attagaactt    60

gagtgaaggt gggctgcttg catcagccta atgtcgagaa gtgctttctt cggaaagtaa    120

ccctcgaaac aaattcattt ttcctctcca attctgcaca agaaagttgc agaacccgaa    180

tagacgaatg aaggaatgca accacacaca cagtgggcta ccttttattt ttt    233


<210>    143
<211>    234
<212>    DNA
<213>    Artificial Sequence

<220>
<223>    sgRNA encoding DNA sequence, targeting DYtarget2(U4AU7)


<400>    143
cttcactgat aaagtggaga accgcttcac caaaagctgt cccttagggg attagaactt    60

gagtgaaggt gggctgcttg catcagccta atgtcgagaa gtgctttctt cggaaagtaa    120

ccctcgaaac aaattcattt ttcctctcca attctgcaca agaaagttgc agaacccgaa    180

tagacgaatg aaggaatgca accacacaca cagtgggcta ccttttattt tttt 234

<210> 144
<211> 235
<212> DNA
<213> Artificial Sequence

<220>
<223> sgRNA encoding DNA sequence, targeting DYtarget2(U4AU8)

<400> 144
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta ccttttattt ttttt 235

<210> 145
<211> 236
<212> DNA
<213> Artificial Sequence

<220>
<223> sgRNA encoding DNA sequence, targeting DYtarget2(U4AU9)

<400> 145
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta ccttttattt tttttt 236

<210> 146
<211> 237
<212> DNA
<213> Artificial Sequence

<220>
<223> sgRNA encoding DNA sequence, targeting DYtarget2(U4AU10)

<400> 146
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accacacaca cagtgggcta ccttttattt ttttttt 237

<210> 147
<211> 232

<212>    DNA
<213>    Artificial Sequence

<220>
<223>    sgRNA encoding DNA sequence, targeting DYtarget2(U3AU6)


<400>    147
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accacacaca cagtgggcta cctttatttt tt               232


<210>    148
<211>    236
<212>    DNA
<213>    Artificial Sequence

<220>
<223>    sgRNA encoding DNA sequence, targeting DYtarget2(U3AU3AU6)


<400>    148
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accacacaca cagtgggcta cctttattta tttttt           236


<210>    149
<211>    57
<212>    DNA
<213>    Artificial Sequence

<220>
<223>    crRNA encoding DNA sequence, targeting DYtarget2(WT)


<400>    149
gttgcagaac ccgaatagac gaatgaagga atgcaaccac acacacagtg ggctacc           57


<210>    150
<211>    68
<212>    DNA
<213>    Artificial Sequence

<220>
<223>    crRNA encoding DNA sequence, targeting DYtarget2(U4AU6)


<400>    150
gttgcagaac ccgaatagac gaatgaagga atgcaaccac acacacagtg ggctaccttt        60

tatttttt                                                                 68

<210>    151
<211>    231
<212>    DNA
<213>    Artificial Sequence

<220>
<223>    sgRNA encoding DNA sequence, targeting DYtarget2(spacer 18nt,
         U4AU6)


<400>    151
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ttttattttt t              231


<210>    152
<211>    232
<212>    DNA
<213>    Artificial Sequence

<220>
<223>    sgRNA encoding DNA sequence, targeting DYtarget2(spacer 19nt,
         U4AU6)


<400>    152
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta cttttatttt tt             232


<210>    153
<211>    234
<212>    DNA
<213>    Artificial Sequence

<220>
<223>    sgRNA encoding DNA sequence, targeting DYtarget2(spacer 21nt,
         U4AU6)


<400>    153
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccattttatt tttt           234


<210>    154
<211>    238

<212>    DNA
<213>    Artificial Sequence

<220>
<223>    sgRNA encoding DNA sequence, targeting DYtarget2(spacer 25nt,
         U4AU6)


<400>    154
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accacacaca cagtgggcta ccatttattt tatttttt         238


<210>    155
<211>    243
<212>    DNA
<213>    Artificial Sequence

<220>
<223>    sgRNA encoding DNA sequence, targeting DYtarget2(spacer 30nt,
         U4AU6)


<400>    155
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accacacaca cagtgggcta ccatttaagg agttttattt       240

ttt                                                                     243


<210>    156
<211>    222
<212>    DNA
<213>    Artificial Sequence

<220>
<223>    sgRNA encoding DNA sequence, targeting DYtarget9(WT)


<400>    156
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca acgacgtggg agacacacac ac                          222


<210>    157
<211>    233
<212>    DNA
<213>    Artificial Sequence

<220>
<223>    sgRNA encoding DNA sequence, targeting DYtarget9(U4AU6)


<400>    157
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca acgacgtggg agacacacac acttttattt ttt            233


<210>    158
<211>    222
<212>    DNA
<213>    Artificial Sequence

<220>
<223>    sgRNA encoding DNA sequence, targeting DYtarget10(WT)


<400>    158
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accatcccca ggacacacac ac                        222


<210>    159
<211>    233
<212>    DNA
<213>    Artificial Sequence

<220>
<223>    sgRNA encoding DNA sequence, targeting DYtarget10(U4AU6)


<400>    159
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accatcccca ggacacacac acttttattt ttt            233


<210>    160
<211>    228
<212>    DNA
<213>    Artificial Sequence

<220>
<223>    sgRNA encoding DNA sequence, targeting DYtarget10(U6)


<400>    160

cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac actttttt 228

<210> 161
<211> 232
<212> DNA
<213> Artificial Sequence

<220>
<223> sgRNA encoding DNA sequence, targeting DYtarget10(U3AU6)

<400> 161
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac actttatttt tt 232

<210> 162
<211> 236
<212> DNA
<213> Artificial Sequence

<220>
<223> sgRNA encoding DNA sequence, targeting DYtarget10(U3AU3AU6)

<400> 162
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac actttattta tttttt 236

<210> 163
<211> 57
<212> DNA
<213> Artificial Sequence

<220>
<223> crRNA encoding DNA sequence, targeting DYtarget10(WT)

<400> 163
gttgcagaac ccgaatagac gaatgaagga atgcaaccat ccccaggaca cacacac 57

<210> 164
<211> 68
<212> DNA

<213>    Artificial Sequence

<220>
<223>    crRNA encoding DNA sequence, targeting DYtarget10(U4AU6)


<400>    164
gttgcagaac ccgaatagac gaatgaagga atgcaaccat ccccaggaca cacacacttt        60

tatttttt                                                                  68


<210>    165
<211>    230
<212>    DNA
<213>    Artificial Sequence

<220>
<223>    sgRNA encoding DNA sequence, targeting DYtarget10(spacer 17nt,
         U4AU6)


<400>    165
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accatcccca ggacacacat tttatttttt                  230


<210>    166
<211>    231
<212>    DNA
<213>    Artificial Sequence

<220>
<223>    sgRNA encoding DNA sequence, targeting DYtarget10(spacer 18nt,
         U4AU6)


<400>    166
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accatcccca ggacacacac ttttattttt t                231


<210>    167
<211>    232
<212>    DNA
<213>    Artificial Sequence

<220>
<223>    sgRNA encoding DNA sequence, targeting DYtarget10(spacer 19nt,
         U4AU6)


<400>    167

cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac attttatttt tt 232

<210> 168
<211> 234
<212> DNA
<213> Artificial Sequence

<220>
<223> sgRNA encoding DNA sequence, targeting DYtarget10(spacer 21nt,
      U4AU6)

<400> 168
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac acattttatt tttt 234

<210> 169
<211> 238
<212> DNA
<213> Artificial Sequence

<220>
<223> sgRNA encoding DNA sequence, targeting DYtarget10(spacer 25nt,
      U4AU6)

<400> 169
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac acatttcttt tatttttt 238

<210> 170
<211> 243
<212> DNA
<213> Artificial Sequence

<220>
<223> sgRNA encoding DNA sequence, targeting DYtarget10(spacer 30nt,
      U4AU6)

<400> 170
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca accatcccca ggacacacac acatttcaaa agttttattt 240

ttt 243

<210> 171
<211> 222
<212> DNA
<213> Artificial Sequence

<220>
<223> sgRNA encoding DNA sequence, targeting DYtarget13(WT)

<400> 171
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca acagcacatg tgcacacaca ca 222

<210> 172
<211> 233
<212> DNA
<213> Artificial Sequence

<220>
<223> sgRNA encoding DNA sequence, targeting DYtarget13(U4AU6)

<400> 172
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca acagcacatg tgcacacaca catttatttt ttt 233

<210> 173
<211> 222
<212> DNA
<213> Artificial Sequence

<220>
<223> sgRNA encoding DNA sequence, targeting Intergenic-22(WT)

<400> 173
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca agaaagttgc agaacccgaa 180

tagacgaatg aaggaatgca acagaacaca tacccctggg cc 222

<210>    174
<211>    228
<212>    DNA
<213>    Artificial Sequence

<220>
<223>    sgRNA encoding DNA sequence, targeting Intergenic-22(U6)


<400>    174
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca acagaacaca tacccctggg cctttttt                   228


<210>    175
<211>    233
<212>    DNA
<213>    Artificial Sequence

<220>
<223>    sgRNA encoding DNA sequence, targeting Intergenic-22(U4AU6)


<400>    175
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca acagaacaca tacccctggg ccttttattt ttt             233


<210>    176
<211>    57
<212>    DNA
<213>    Artificial Sequence

<220>
<223>    crRNA encoding DNA sequence, targeting Intergenic-22(WT)


<400>    176
gttgcagaac ccgaatagac gaatgaagga atgcaacaga acacataccc ctgggcc          57


<210>    177
<211>    68
<212>    DNA
<213>    Artificial Sequence

<220>
<223>    crRNA encoding DNA sequence, targeting Intergenic-22(U4AU6)


<400>    177

gttgcagaac ccgaatagac gaatgaagga atgcaacaga acacataccc ctgggccttt 60

tatttttt 68

<210> 178
<211> 22
<212> DNA
<213> Artificial Sequence

<220>
<223> forward primer to synthesize sgRNA for RNF2

<400> 178
gccaacatac agaagtcagg aa 22

<210> 179
<211> 20
<212> DNA
<213> Artificial Sequence

<220>
<223> forward primer to synthesize sgRNA amplicon for DYtarget2

<400> 179
ccaggccatt tctcagggtt 20

<210> 180
<211> 20
<212> DNA
<213> Artificial Sequence

<220>
<223> forward primer to synthesize sgRNA amplicon for DYtarget9

<400> 180
gtacagcttc ccagactccg 20

<210> 181
<211> 20
<212> DNA
<213> Artificial Sequence

<220>
<223> forward primer to synthesize sgRNA amplicon for DYtarget10

<400> 181
tgctccagga acctcaccta 20

<210> 182
<211> 20
<212> DNA
<213> Artificial Sequence

<220>

<223>    forward primer to synthesize sgRNA amplicon for DYtarget13

<400>    182
gccttccccc acctttcttt                                                    20

<210>    183
<211>    21
<212>    DNA
<213>    Artificial Sequence

<220>
<223>    U6 forward primer to synthesize sgRNA

<400>    183
gagggcctat ttcccatgat t                                                  21

<210>    184
<211>    21
<212>    DNA
<213>    Artificial Sequence

<220>
<223>    forward primer to synthesize tracrRNA

<400>    184
gagggcctat ttcccatgat t                                                  21

<210>    185
<211>    20
<212>    DNA
<213>    Artificial Sequence

<220>
<223>    reverse primer to synthesize sgRNA amplicon for RNF2(WT)

<400>    185
tgtgcagaca aacggaactc                                                    20

<210>    186
<211>    31
<212>    DNA
<213>    Artificial Sequence

<220>
<223>    reverse primer to synthesize sgRNA amplicon for RNF2(U4AU6)

<400>    186
aaaaaataaa atgtgcagac aaacggaact c                                       31

<210>    187
<211>    45
<212>    DNA
<213>    Artificial Sequence

<220>
<223>    reverse primer to synthesize sgRNA amplicon for DYtarget2(WT)


<400>    187
ggtagcccac tgtgtgtgtg gttgcattcc ttcattcgtc tattc                45


<210>    188
<211>    46
<212>    DNA
<213>    Artificial Sequence

<220>
<223>    reverse primer to synthesize sgRNA amplicon for DYtarget2(U)


<400>    188
aggtagccca ctgtgtgtgt ggttgcattc cttcattcgt ctattc               46


<210>    189
<211>    47
<212>    DNA
<213>    Artificial Sequence

<220>
<223>    reverse primer to synthesize sgRNA amplicon for DYtarget2(U2)


<400>    189
aaggtagccc actgtgtgtg tggttgcatt ccttcattcg tctattc              47


<210>    190
<211>    48
<212>    DNA
<213>    Artificial Sequence

<220>
<223>    reverse primer to synthesize sgRNA amplicon for DYtarget2(U3)


<400>    190
aaaggtagcc cactgtgtgt gtggttgcat tccttcattc gtctattc             48


<210>    191
<211>    49
<212>    DNA
<213>    Artificial Sequence

<220>
<223>    reverse primer to synthesize sgRNA amplicon for DYtarget2(U4)


<400>    191
aaaaggtagc ccactgtgtg tgtggttgca ttccttcatt cgtctattc            49


<210>    192
<211>    50

<212>    DNA
<213>    Artificial Sequence

<220>
<223>    reverse primer to synthesize sgRNA amplicon for DYtarget2(U5)


<400>    192
aaaaaggtag cccactgtgt gtgtggttgc attccttcat tcgtctattc                50


<210>    193
<211>    51
<212>    DNA
<213>    Artificial Sequence

<220>
<223>    reverse primer to synthesize sgRNA amplicon for DYtarget2(U6)


<400>    193
aaaaaaggta gcccactgtg tgtgtggttg cattccttca ttcgtctatt c              51


<210>    194
<211>    50
<212>    DNA
<213>    Artificial Sequence

<220>
<223>    reverse primer to synthesize sgRNA amplicon for DYtarget2(U4A)


<400>    194
taaaaggtag cccactgtgt gtgtggttgc attccttcat tcgtctattc                50


<210>    195
<211>    51
<212>    DNA
<213>    Artificial Sequence

<220>
<223>    reverse primer to synthesize sgRNA amplicon for DYtarget2(U4AU)


<400>    195
ataaaaggta gcccactgtg tgtgtggttg cattccttca ttcgtctatt c              51


<210>    196
<211>    52
<212>    DNA
<213>    Artificial Sequence

<220>
<223>    reverse primer to synthesize sgRNA amplicon for DYtarget2(U4AU2)


<400>    196
aataaaaggt agcccactgt gtgtgtggtt gcattccttc attcgtctat tc             52

<210>    197
<211>    53
<212>    DNA
<213>    Artificial Sequence

<220>
<223>    reverse primer to synthesize sgRNA amplicon for DYtarget2(U4AU3)


<400>    197
aaataaaagg tagcccactg tgtgtgtggt tgcattcctt cattcgtcta ttc          53


<210>    198
<211>    54
<212>    DNA
<213>    Artificial Sequence

<220>
<223>    reverse primer to synthesize sgRNA amplicon for DYtarget2(U4AU4)


<400>    198
aaaataaaag gtagcccact gtgtgtgtgg ttgcattcct tcattcgtct attc          54


<210>    199
<211>    55
<212>    DNA
<213>    Artificial Sequence

<220>
<223>    reverse primer to synthesize sgRNA amplicon for DYtarget2(U4AU5)


<400>    199
aaaaataaaa ggtagcccac tgtgtgtgtg gttgcattcc ttcattcgtc tattc          55


<210>    200
<211>    56
<212>    DNA
<213>    Artificial Sequence

<220>
<223>    reverse primer to synthesize sgRNA amplicon for DYtarget2(U4AU6)


<400>    200
aaaaaataaa aggtagccca ctgtgtgtgt ggttgcattc cttcattcgt ctattc          56


<210>    201
<211>    57
<212>    DNA
<213>    Artificial Sequence

<220>
<223>    reverse primer to synthesize sgRNA amplicon for DYtarget2(U4AU7)


<400>    201
aaaaaaataa aaggtagccc actgtgtgtg tggttgcatt ccttcattcg tctattc          57

<210>    202
<211>    58
<212>    DNA
<213>    Artificial Sequence

<220>
<223>    reverse primer to synthesize sgRNA amplicon for DYtarget2(U4AU8)


<400>    202
aaaaaaaata aaaggtagcc cactgtgtgt gtggttgcat tccttcattc gtctattc         58


<210>    203
<211>    59
<212>    DNA
<213>    Artificial Sequence

<220>
<223>    reverse primer to synthesize sgRNA amplicon for DYtarget2(U4AU9)


<400>    203
aaaaaaaaat aaaaggtagc ccactgtgtg tgtggttgca ttccttcatt cgtctattc        59


<210>    204
<211>    60
<212>    DNA
<213>    Artificial Sequence

<220>
<223>    reverse primer to synthesize sgRNA amplicon for DYtarget2(U4AU10)


<400>    204
aaaaaaaaaa taaaaggtag cccactgtgt gtgtggttgc attccttcat tcgtctattc       60

                                                                        60


<210>    205
<211>    55
<212>    DNA
<213>    Artificial Sequence

<220>
<223>    reverse primer to synthesize sgRNA amplicon for DYtarget2(U3AU6)


<400>    205
aaaaaataaa ggtagcccac tgtgtgtgtg gttgcattcc ttcattcgtc tattc            55


<210>    206
<211>    59
<212>    DNA
<213>    Artificial Sequence

<220>
<223>    reverse primer to synthesize sgRNA amplicon for

DYtarget2(U3AU3AU6)

<400>    206
aaaaaataaa taaaggtagc ccactgtgtg tgtggttgca ttccttcatt cgtctattc         59

<210>    207
<211>    45
<212>    DNA
<213>    Artificial Sequence

<220>
<223>    reverse primer to synthesize crRNA amplicon for DYtarget2(WT)

<400>    207
ggtagcccac tgtgtgtgtg gttgcattcc ttcattcgtc tattc                        45

<210>    208
<211>    56
<212>    DNA
<213>    Artificial Sequence

<220>
<223>    reverse primer to synthesize crRNA amplicon for DYtarget2(U4AU6)

<400>    208
aaaaaataaa aggtagccca ctgtgtgtgt ggttgcattc cttcattcgt ctattc            56

<210>    209
<211>    40
<212>    DNA
<213>    Artificial Sequence

<220>
<223>    reverse primer to synthesize sgRNA amplicon for DYtarget2(spacer
         18nt, U4AU6)

<400>    209
aaaaaataaa atagcccact gtgtgtgtgg ttgcattcct                              40

<210>    210
<211>    41
<212>    DNA
<213>    Artificial Sequence

<220>
<223>    reverse primer to synthesize sgRNA amplicon for DYtarget2(spacer
         19nt, U4AU6)

<400>    210
aaaaaataaa agtagcccac tgtgtgtgtg gttgcattcc t                            41

<210>    211
<211>    43

<212>    DNA
<213>    Artificial Sequence

<220>
<223>    reverse primer to synthesize sgRNA amplicon for DYtarget2(spacer
         21nt, U4AU6)


<400>    211
aaaaaataaa atggtagccc actgtgtgtg tggttgcatt cct                        43


<210>    212
<211>    47
<212>    DNA
<213>    Artificial Sequence

<220>
<223>    reverse primer to synthesize sgRNA amplicon for DYtarget2(spacer
         25nt, U4AU6)


<400>    212
aaaaaataaa ataaatggta gcccactgtg tgtgtggttg cattcct                    47


<210>    213
<211>    52
<212>    DNA
<213>    Artificial Sequence

<220>
<223>    reverse primer to synthesize sgRNA amplicon for DYtarget2(spacer
         30nt, U4AU6)


<400>    213
aaaaaataaa actccttaaa tggtagccca ctgtgtgtgt ggttgcattc ct              52


<210>    214
<211>    45
<212>    DNA
<213>    Artificial Sequence

<220>
<223>    reverse primer to synthesize sgRNA amplicon for DYtarget9(WT)


<400>    214
gtgtgtgtgt ctcccacgtc gttgcattcc ttcattcgtc tattc                      45


<210>    215
<211>    55
<212>    DNA
<213>    Artificial Sequence

<220>
<223>    reverse primer to synthesize sgRNA amplicon for DYtarget9(U4AU6)


<400>    215

aaaaaataaa atgtgtgtgt ctcccacgtc gttgcattcc ttcattcgtc tattc          55

<210>    216
<211>    45
<212>    DNA
<213>    Artificial Sequence

<220>
<223>    reverse primer to synthesize sgRNA amplicon for DYtarget10(WT)

<400>    216
gtgtgtgtgt cctggggatg gttgcattcc ttcattcgtc tattc          45

<210>    217
<211>    55
<212>    DNA
<213>    Artificial Sequence

<220>
<223>    reverse primer to synthesize sgRNA amplicon for DYtarget10(U4AU6)

<400>    217
aaaaaataaa atgtgtgtgt cctggggatg gttgcattcc ttcattcgtc tattc          55

<210>    218
<211>    50
<212>    DNA
<213>    Artificial Sequence

<220>
<223>    reverse primer to synthesize sgRNA amplicon for DYtarget10(U6)

<400>    218
aaaaaatgtg tgtgtcctgg ggatggttgc attccttcat tcgtctattc          50

<210>    219
<211>    54
<212>    DNA
<213>    Artificial Sequence

<220>
<223>    reverse primer to synthesize sgRNA amplicon for DYtarget10(U3AU6)

<400>    219
aaaaaataaa tgtgtgtgtc ctggggatgg ttgcattcct tcattcgtct attc          54

<210>    220
<211>    58
<212>    DNA
<213>    Artificial Sequence

<220>
<223>    reverse primer to synthesize sgRNA amplicon for
         DYtarget10(U3AU3AU6)

<400>    220
aaaaaataaa taaatgtgtg tgtcctgggg atggttgcat tccttcattc gtctattc         58


<210>    221
<211>    45
<212>    DNA
<213>    Artificial Sequence

<220>
<223>    reverse primer to synthesize crRNA amplicon for DYtarget10(WT)


<400>    221
gtgtgtgtgt cctggggatg gttgcattcc ttcattcgtc tattc                       45


<210>    222
<211>    56
<212>    DNA
<213>    Artificial Sequence

<220>
<223>    reverse primer to synthesize crRNA amplicon for DYtarget10(U4AU6)


<400>    222
aaaaataaaa gtgtgtgtgt cctggggatg gttgcattcc ttcattcgtc atattc           56


<210>    223
<211>    39
<212>    DNA
<213>    Artificial Sequence

<220>
<223>    reverse primer to synthesize sgRNA amplicon for DYtarget10(spacer
         17nt, U4AU6)


<400>    223
aaaaaataaa atgtgtgtcc tggggatggt tgcattcct                              39


<210>    224
<211>    40
<212>    DNA
<213>    Artificial Sequence

<220>
<223>    reverse primer to synthesize sgRNA amplicon for DYtarget10(spacer
         18nt, U4AU6)


<400>    224
aaaaaataaa agtgtgtgtc ctggggatgg ttgcattcct                             40


<210>    225
<211>    41
<212>    DNA

<213>    Artificial Sequence

<220>
<223>    reverse primer to synthesize sgRNA amplicon for DYtarget10(spacer
         19nt, U4AU6)


<400>    225
aaaaaataaa atgtgtgtgt cctggggatg gttgcattcc t                          41


<210>    226
<211>    43
<212>    DNA
<213>    Artificial Sequence

<220>
<223>    reverse primer to synthesize sgRNA amplicon for DYtarget10(spacer
         21nt, U4AU6)


<400>    226
aaaaaataaa atgtgtgtgt gtcctgggga tggttgcatt cct                        43


<210>    227
<211>    47
<212>    DNA
<213>    Artificial Sequence

<220>
<223>    reverse primer to synthesize sgRNA amplicon for DYtarget10(spacer
         25nt, U4AU6)


<400>    227
aaaaaataaa agaaatgtgt gtgtgtcctg gggatggttg cattcct                    47


<210>    228
<211>    52
<212>    DNA
<213>    Artificial Sequence

<220>
<223>    reverse primer to synthesize sgRNA amplicon for DYtarget10(spacer
         30nt, U4AU6)


<400>    228
aaaaaataaa acttttgaaa tgtgtgtgtg tcctggggat ggttgcattc ct              52


<210>    229
<211>    45
<212>    DNA
<213>    Artificial Sequence

<220>
<223>    reverse primer to synthesize sgRNA amplicon for DYtarget13(WT)


<400>    229

tgtgtgtgtg cacatgtgct gttgcattcc ttcattcgtc tattc                45

<210>    230
<211>    55
<212>    DNA
<213>    Artificial Sequence

<220>
<223>    reverse primer to synthesize sgRNA amplicon for DYtarget13(U4AU6)

<400>    230
aaaaaataaa agtgtgtgtg cacatgtgct gttgcattcc ttcattcgtc tattc     55

<210>    231
<211>    31
<212>    DNA
<213>    Artificial Sequence

<220>
<223>    reverse primer to synthesize sgRNA amplicon for Intergenic-22(WT)

<400>    231
ggcccagggg tatgtgttct gttgcattcc t                               31

<210>    232
<211>    37
<212>    DNA
<213>    Artificial Sequence

<220>
<223>    reverse primer to synthesize sgRNA amplicon for Intergenic-22(U6)

<400>    232
aaaaaaggcc caggggtatg tgttctgttg cattcct                         37

<210>    233
<211>    42
<212>    DNA
<213>    Artificial Sequence

<220>
<223>    reverse primer to synthesize sgRNA amplicon for
         Intergenic-22(U4AU6)

<400>    233
aaaaaataaa aggcccaggg gtatgtgttc tgttgcattc ct                   42

<210>    234
<211>    31
<212>    DNA
<213>    Artificial Sequence

<220>
<223>    reverse primer to synthesize crRNA amplicon for Intergenic-22(WT)

<400>    234
ggcccagggg tatgtgttct gttgcattcc t                                    31


<210>    235
<211>    42
<212>    DNA
<213>    Artificial Sequence

<220>
<223>    reverse primer to synthesize crRNA amplicon for
         Intergenic-22(U4AU6)


<400>    235
aaaaaataaa aggcccaggg gtatgtgttc tgttgcattc ct                        42


<210>    236
<211>    20
<212>    DNA
<213>    Artificial Sequence

<220>
<223>    reverse primer to synthesize tracrRNA amplicon


<400>    236
ttgtgcagaa ttggagagga                                                 20


<210>    237
<211>    7
<212>    PRT
<213>    simian virus 40

<400>    237
Pro Lys Lys Lys Arg Lys Val
1               5


<210>    238
<211>    16
<212>    PRT
<213>    mus musculus

<400>    238
Lys Arg Pro Ala Ala Thr Lys Lys Ala Gly Gln Ala Lys Lys Lys Lys
1               5                   10                  15



<210>    239
<211>    9
<212>    PRT
<213>    homo sapiens

<400>    239
Pro Ala Ala Lys Arg Val Lys Leu Asp
1               5

<210>    240
<211>    11
<212>    PRT
<213>    Homo sapiens

<400>    240
Arg Gln Arg Arg Asn Glu Leu Lys Arg Ser Pro
1               5                   10

<210>    241
<211>    38
<212>    PRT
<213>    mus musculus

<400>    241
Asn Gln Ser Ser Asn Phe Gly Pro Met Lys Gly Gly Asn Phe Gly Gly
1               5                   10                  15

Arg Ser Ser Gly Pro Tyr Gly Gly Gly Gly Gln Tyr Phe Ala Lys Pro
            20                  25                  30

Arg Asn Gln Gly Gly Tyr
        35

<210>    242
<211>    42
<212>    PRT
<213>    Homo sapiens

<400>    242
Arg Met Arg Ile Glx Phe Lys Asn Lys Gly Lys Asp Thr Ala Glu Leu
1               5                   10                  15

Arg Arg Arg Arg Val Glu Val Ser Val Glu Leu Arg Lys Ala Lys Lys
            20                  25                  30

Asp Glu Gln Ile Leu Lys Arg Arg Asn Val
        35                  40

<210>    243
<211>    8
<212>    PRT
<213>    Homo sapiens

<400>    243
Val Ser Arg Lys Arg Pro Arg Pro
1               5

<210>    244
<211>    8
<212>    PRT
<213>    homo sapiens

<400>    244
Pro Pro Lys Lys Ala Arg Glu Asp
1               5

<210>    245
<211>    8
<212>    PRT
<213>    homo sapiens

<400>    245
Pro Gln Pro Lys Lys Lys Pro Leu
1               5



<210>    246
<211>    12
<212>    PRT
<213>    mus musculus

<400>    246
Ser Ala Leu Ile Lys Lys Lys Lys Lys Met Ala Pro
1               5                   10



<210>    247
<211>    5
<212>    PRT
<213>    Influenza virus

<400>    247
Asp Arg Leu Arg Arg
1               5



<210>    248
<211>    7
<212>    PRT
<213>    influenza virus

<400>    248
Pro Lys Gln Lys Lys Arg Lys
1               5



<210>    249
<211>    10
<212>    PRT
<213>    Hepatitis D virus

<400>    249
Arg Lys Leu Lys Lys Lys Ile Lys Lys Leu
1               5                   10



<210>    250
<211>    10
<212>    PRT
<213>    mus musculus

<400>    250
Arg Glu Lys Lys Lys Phe Leu Lys Arg Arg
1               5                   10



<210>    251
<211>    20
<212>    PRT

<213>    homo sapiens

<400>    251
Lys Arg Lys Gly Asp Glu Val Asp Gly Val Asp Glu Val Ala Lys Lys
1               5                   10                  15

Lys Ser Lys Lys
            20


<210>    252
<211>    17
<212>    PRT
<213>    Homo sapiens

<400>    252
Arg Lys Cys Leu Gln Ala Gly Met Asn Leu Glu Ala Arg Lys Thr Lys
1               5                   10                  15

Lys




<210>    253
<211>    69
<212>    RNA
<213>    Artificial Sequence

<220>
<223>    scaffold sequence for sgRNA


<400>    253
uucauuuuuc cucuccaauu cugcacaaga aaguugcaga acccgaauag acgaaugaag    60

gaaugcaac                                                            69


<210>    254
<211>    69
<212>    DNA
<213>    Artificial Sequence

<220>
<223>    scaffold sequence encoding DNA sequence, for sgRNA


<400>    254
ttcatttttc ctctccaatt ctgcacaaga aaguugcaga acccgaauag acgaaugaag    60

gaaugcaac                                                            69


<210>    255
<211>    37
<212>    DNA
<213>    Artificial Sequence

<220>
<223>    common crRNA sequence


<400>    255

gttgcagaac ccgaatagac gaatgaagga atgcaac                                37

<210>    256
<211>    233
<212>    DNA
<213>    Artificial Sequence

<220>
<223>    sgRNA encoding DNA sequence for DYtarget2(T4AT4AT)

<400>    256
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccttttattt tat            233

<210>    257
<211>    234
<212>    DNA
<213>    Artificial Sequence

<220>
<223>    sgRNA encoding DNA sequence for DYtarget2(T4AT4AT2)

<400>    257
cttcactgat aaagtggaga accgcttcac caaaagctgt cccttagggg attagaactt      60

gagtgaaggt gggctgcttg catcagccta atgtcgagaa gtgctttctt cggaaagtaa     120

ccctcgaaac aaattcattt ttcctctcca attctgcaca agaaagttgc agaacccgaa     180

tagacgaatg aaggaatgca accacacaca cagtgggcta ccttttattt tatt           234

<210>    258
<211>    56
<212>    DNA
<213>    Artificial Sequence

<220>
<223>    reverse primer for sgRNA amplicon for DYtarget2(T4AT4AT)

<400>    258
ataaaataaa aggtagccca ctgtgtgtgt ggttgcattc cttcattcgt ctattc          56

<210>    259
<211>    57
<212>    DNA
<213>    Artificial Sequence

<220>
<223>    Reverse primer for sgRNA amplicon for DYtarget2(T4AT4AT2)

<400>    259
aataaaataa aaggtagccc actgtgtgtg tggttgcatt ccttcattcg tctattc          57


<210>    260
<211>    20
<212>    DNA
<213>    Artificial Sequence

<220>
<223>    protospacer sequence for CSMD1


<400>    260
gaataccccc attcttcagg                                                   20


<210>    261
<211>    222
<212>    RNA
<213>    Artificial Sequence

<220>
<223>    sgRNA sequence for CSMD1(WT)


<400>    261
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gg                        222


<210>    262
<211>    223
<212>    RNA
<213>    Artificial Sequence

<220>
<223>    sgRNA for CSMD1(U)


<400>    262
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca ggu                       223


<210>    263
<211>    224
<212>    RNA
<213>    Artificial Sequence

<220>
<223>    sgRNA sequence for CSMD1(U2)

<400>    263
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguu                      224


<210>    264
<211>    225
<212>    RNA
<213>    Artificial Sequence

<220>
<223>    sgRNA sequence for CSMD1(U3)


<400>    264
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguuu                     225


<210>    265
<211>    226
<212>    RNA
<213>    Artificial Sequence

<220>
<223>    sgRNA sequence for CSMD1(U4)


<400>    265
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguuuu                    226


<210>    266
<211>    227
<212>    RNA
<213>    Artificial Sequence

<220>
<223>    sgRNA sequence for CSMD1(U5)


<400>    266
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa 180

uagacgaaug aaggaaugca acgaauaccc ccauucuuca gguuuuu 227

<210> 267
<211> 228
<212> RNA
<213> Artificial Sequence

<220>
<223> sgRNA sequence for CSMD1(U6)

<400> 267
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca acgaauaccc ccauucuuca gguuuuuu 228

<210> 268
<211> 227
<212> RNA
<213> Artificial Sequence

<220>
<223> sgRNA sequence for CSMD1(U4A)

<400> 268
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca acgaauaccc ccauucuuca gguuuua 227

<210> 269
<211> 228
<212> RNA
<213> Artificial Sequence

<220>
<223> sgRNA sequence for CSMD1(U4AU)

<400> 269
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca acgaauaccc ccauucuuca gguuuuau 228

<210> 270

<211>    229
<212>    RNA
<213>    Artificial Sequence

<220>
<223>    sgRNA sequence for CSMD1(U4AU)


<400>    270
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguuuuauu                 229


<210>    271
<211>    230
<212>    RNA
<213>    Artificial Sequence

<220>
<223>    sgRNA sequence for CSMD1(U4AU3)


<400>    271
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguuuuauuu                230


<210>    272
<211>    231
<212>    RNA
<213>    Artificial Sequence

<220>
<223>    sgRNA sequence for CSMD1(U4AU5)


<400>    272
cuucacugau aaaguggaga accgcuucac caaaagcugu cccuuagggg auuagaacuu      60

gagugaaggu gggcugcuug caucagccua augucgagaa gugcuuucuu cggaaaguaa     120

cccucgaaac aaauucauuu uuccucucca auucugcaca agaaaguugc agaacccgaa     180

uagacgaaug aaggaaugca acgaauaccc ccauucuuca gguuuuauuu u              231


<210>    273
<211>    232
<212>    RNA
<213>    Artificial Sequence

<220>
<223>    sgRNA sequence for CSMD1(U4AU5)

<400>    273
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca acgaauaccc ccauucuuca gguuuuauuu uu    232


<210>    274
<211>    233
<212>    RNA
<213>    Artificial Sequence

<220>
<223>    sgRNA sequence for CSMD1(U4AU6)


<400>    274
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca acgaauaccc ccauucuuca gguuuuauuu uuu    233


<210>    275
<211>    234
<212>    RNA
<213>    Artificial Sequence

<220>
<223>    sgRNA sequence for CSMD1(U4AU7)


<400>    275
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa    180

uagacgaaug aaggaaugca acgaauaccc ccauucuuca gguuuuauuu uuuu    234


<210>    276
<211>    235
<212>    RNA
<213>    Artificial Sequence

<220>
<223>    sgRNA sequence for CSMD1(U4AU8)


<400>    276
cuucacugau aaaguggaga accgcuucac caaaagcugu cccuuagggg auuagaacuu    60

gagugaaggu gggcugcuug caucagccua augucgagaa gugcuuucuu cggaaaguaa    120

cccucgaaac aaauucauuu uuccucucca auucugcaca agaaaguugc agaacccgaa 180

uagacgaaug aaggaaugca acgaauaccc ccauucuuca gguuuuauuu uuuuu 235

<210> 277
<211> 236
<212> RNA
<213> Artificial Sequence

<220>
<223> sgRNA sequence for CSMD1(U4AU9)

<400> 277
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca acgaauaccc ccauucuuca gguuuuauuu uuuuuu 236

<210> 278
<211> 237
<212> RNA
<213> Artificial Sequence

<220>
<223> sgRNA sequence for CSMD1(U4AU10)

<400> 278
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca acgaauaccc ccauucuuca gguuuuauuu uuuuuuu 237

<210> 279
<211> 233
<212> RNA
<213> Artificial Sequence

<220>
<223> sgRNA sequence for DYtarget2(U4AU4AU)

<400> 279
cuucacugau aaaguggaga accgcuucac caaaagcugu cccuuagggg auuagaacuu 60

gagugaaggu gggcugcuug caucagccua augucgagaa gugcuuucuu cggaaaguaa 120

cccucgaaac aaauucauuu uuccucucca auucugcaca agaaaguugc agaacccgaa 180

uagacgaaug aaggaaugca accacacaca cagugggcua ccuuuuauuu uau 233

<210>    280
<211>    234
<212>    RNA
<213>    Artificial Sequence

<220>
<223>    sgRNA sequence for DYtarget2(U4AU4AU2)


<400>    280
cuucacugau aaaguggaga accgcuucac caaaagcugu cccuuagggg auuagaacuu        60

gagugaaggu gggcugcuug caucagccua augucgagaa gugcuuucuu cggaaaguaa       120

cccucgaaac aaauucauuu uuccucucca auucugcaca agaaaguugc agaacccgaa       180

uagacgaaug aaggaaugca accacacaca cagugggcua ccuuuuauuu uauu             234


<210>    281
<211>    560
<212>    PRT
<213>    unidentified archaeon ICHT

<400>    281
Pro Lys Lys Lys Arg Lys Val Gly Ile His Gly Val Pro Ala Ala Met
1               5                   10                  15

Ala Lys Asn Thr Ile Thr Lys Thr Leu Lys Leu Arg Ile Val Arg Pro
            20                  25                  30

Tyr Asn Ser Ala Glu Val Glu Lys Ile Val Ala Asp Glu Lys Asn Asn
        35                  40                  45

Arg Glu Lys Ile Ala Leu Glu Lys Asn Lys Asp Lys Val Lys Glu Ala
    50                  55                  60

Cys Ser Lys His Leu Lys Val Ala Ala Tyr Cys Thr Thr Gln Val Glu
65                  70                  75                  80

Arg Asn Ala Cys Leu Phe Cys Lys Ala Arg Lys Leu Asp Asp Lys Phe
                85                  90                  95

Tyr Gln Lys Leu Arg Gly Gln Phe Pro Asp Ala Val Phe Trp Gln Glu
            100                 105                 110

Ile Ser Glu Ile Phe Arg Gln Leu Gln Lys Gln Ala Ala Glu Ile Tyr
        115                 120                 125

Asn Gln Ser Leu Ile Glu Leu Tyr Tyr Glu Ile Phe Ile Lys Gly Lys
    130                 135                 140

Gly Ile Ala Asn Ala Ser Ser Val Glu His Tyr Leu Ser Asp Val Cys
145                 150                 155                 160

Tyr Thr Arg Ala Ala Glu Leu Phe Lys Asn Ala Ala Ile Ala Ser Gly
                165                 170                 175

Leu Arg Ser Lys Ile Lys Ser Asn Phe Arg Leu Lys Glu Leu Lys Asn
            180                 185                 190

Met Lys Ser Gly Leu Pro Thr Thr Lys Ser Asp Asn Phe Pro Ile Pro
        195                 200                 205

Leu Val Lys Gln Lys Gly Gly Gln Tyr Thr Gly Phe Glu Ile Ser Asn
    210                 215                 220

His Asn Ser Asp Phe Ile Ile Lys Ile Pro Phe Gly Arg Trp Gln Val
225                 230                 235                 240

Lys Lys Glu Ile Asp Lys Tyr Arg Pro Trp Glu Lys Phe Asp Phe Glu
                245                 250                 255

Gln Val Gln Lys Ser Pro Lys Pro Ile Ser Leu Leu Leu Ser Thr Gln
            260                 265                 270

Arg Arg Lys Arg Asn Lys Gly Trp Ser Lys Asp Glu Gly Thr Glu Ala
        275                 280                 285

Glu Ile Lys Lys Val Met Asn Gly Asp Tyr Gln Thr Ser Tyr Ile Glu
    290                 295                 300

Val Lys Arg Gly Ser Lys Ile Gly Glu Lys Ser Ala Trp Met Leu Asn
305                 310                 315                 320

Leu Ser Ile Asp Val Pro Lys Ile Asp Lys Gly Val Asp Pro Ser Ile
                325                 330                 335

Ile Gly Gly Ile Asp Val Gly Val Lys Ser Pro Leu Val Cys Ala Ile
            340                 345                 350

Asn Asn Ala Phe Ser Arg Tyr Ser Ile Ser Asp Asn Asp Leu Phe His
        355                 360                 365

Phe Asn Lys Lys Met Phe Ala Arg Arg Arg Ile Leu Leu Lys Lys Asn
    370                 375                 380

Arg His Lys Arg Ala Gly His Gly Ala Lys Asn Lys Leu Lys Pro Ile
385                 390                 395                 400

Thr Ile Leu Thr Glu Lys Ser Glu Arg Phe Arg Lys Lys Leu Ile Glu
                405                 410                 415

Arg Trp Ala Cys Glu Ile Ala Asp Phe Phe Ile Lys Asn Lys Val Gly
            420                 425                 430

Thr Val Gln Met Glu Asn Leu Glu Ser Met Lys Arg Lys Glu Asp Ser
        435                 440                 445

Tyr Phe Asn Ile Arg Leu Arg Gly Phe Trp Pro Tyr Ala Glu Met Gln
    450                 455                 460

Asn Lys Ile Glu Phe Lys Leu Lys Gln Tyr Gly Ile Glu Ile Arg Lys
465                 470                 475                 480

Val Ala Pro Asn Asn Thr Ser Lys Thr Cys Ser Lys Cys Gly His Leu
                485                 490                 495

Asn Asn Tyr Phe Asn Phe Glu Tyr Arg Lys Lys Asn Lys Phe Pro His
            500                 505                 510

Phe Lys Cys Glu Lys Cys Asn Phe Lys Glu Asn Ala Asp Tyr Asn Ala
        515                 520                 525

Ala Leu Asn Ile Ser Asn Pro Lys Leu Lys Ser Thr Lys Glu Glu Pro
    530                 535                 540

Lys Arg Pro Ala Ala Thr Lys Lys Ala Gly Gln Ala Lys Lys Lys Lys
545                 550                 555                 560

<210>   282
<211>   497
<212>   PRT
<213>   Artificial Sequence

<220>
<223>   polypeptide sequence for effector protein of Syntrophomonas
        palmitatica

<400>   282
Met Gly Glu Ser Val Lys Ala Ile Lys Leu Lys Ile Leu Asp Met Phe
1               5                   10                  15

Leu Asp Pro Glu Cys Thr Lys Gln Asp Asp Asn Trp Arg Lys Asp Leu
            20                  25                  30

Ser Thr Met Ser Arg Phe Cys Ala Glu Ala Gly Asn Met Cys Leu Arg
        35                  40                  45

Asp Leu Tyr Asn Tyr Phe Ser Met Pro Lys Glu Asp Arg Ile Ser Ser
    50                  55                  60

Lys Asp Leu Tyr Asn Ala Met Tyr His Lys Thr Lys Leu Leu His Pro
65                  70                  75                  80

Glu Leu Pro Gly Lys Val Ala Asn Gln Ile Val Asn His Ala Lys Asp
                85                  90                  95

Val Trp Lys Arg Asn Ala Lys Leu Ile Tyr Arg Asn Gln Ile Ser Met
            100                 105                 110

Pro Thr Tyr Lys Ile Thr Thr Ala Pro Ile Arg Leu Gln Asn Asn Ile
        115                 120                 125

Tyr Lys Leu Ile Lys Asn Lys Asn Lys Tyr Ile Ile Asp Val Gln Leu
    130                 135                 140

Tyr Ser Lys Glu Tyr Ser Lys Asp Ser Gly Lys Gly Thr His Arg Tyr
145                 150                 155                 160

Phe Leu Val Ala Val Arg Asp Ser Ser Thr Arg Met Ile Phe Asp Arg
                165                 170                 175

Ile Met Ser Lys Asp His Ile Asp Ser Ser Lys Ser Tyr Thr Gln Gly
            180                 185                 190

Gln Leu Gln Ile Lys Lys Asp His Gln Gly Lys Trp Tyr Cys Ile Ile
        195                 200                 205

Pro Tyr Thr Phe Pro Thr His Glu Thr Val Leu Asp Pro Asp Lys Val
    210                 215                 220

Met Gly Val Asp Leu Gly Val Ala Lys Ala Val Tyr Trp Ala Phe Asn
225                 230                 235                 240

Ser Ser Tyr Lys Arg Gly Cys Ile Asp Gly Gly Glu Ile Glu His Phe
                245                 250                 255

Arg Lys Met Ile Arg Ala Arg Arg Val Ser Ile Gln Asn Gln Ile Lys
            260                 265                 270

His Ser Gly Asp Ala Arg Lys Gly His Gly Arg Lys Arg Ala Leu Lys
        275                 280                 285

Pro Ile Glu Thr Leu Ser Glu Lys Glu Lys Asn Phe Arg Asp Thr Ile
    290                 295                 300

Asn His Arg Tyr Ala Asn Arg Ile Val Glu Ala Ala Ile Lys Gln Gly
305                 310                 315                 320

Cys Gly Thr Ile Gln Ile Glu Asn Leu Glu Gly Ile Ala Asp Thr Thr
                325                 330                 335

Gly Ser Lys Phe Leu Lys Asn Trp Pro Tyr Tyr Asp Leu Gln Thr Lys
            340                 345                 350

Ile Val Asn Lys Ala Lys Glu His Gly Ile Thr Val Val Ala Ile Asn
        355                 360                 365

Pro Gln Tyr Thr Ser Gln Arg Cys Ser Met Cys Gly Tyr Ile Glu Lys
    370                 375                 380

Thr Asn Arg Ser Ser Gln Ala Val Phe Glu Cys Lys Gln Cys Gly Tyr
385                 390                 395                 400

Gly Ser Arg Thr Ile Cys Ile Asn Cys Arg His Val Gln Val Ser Gly
                405                 410                 415

Asp Val Cys Glu Glu Cys Gly Gly Ile Val Lys Lys Glu Asn Val Asn
            420                 425                 430

Ala Asp Tyr Asn Ala Ala Lys Asn Ile Ser Thr Pro Tyr Ile Asp Gln
        435                 440                 445

Ile Ile Met Glu Lys Cys Leu Glu Leu Gly Ile Pro Tyr Arg Ser Ile
    450                 455                 460

Thr Cys Lys Glu Cys Gly His Ile Gln Ala Ser Gly Asn Thr Cys Glu
465                 470                 475                 480

Val Cys Gly Ser Thr Asn Ile Leu Lys Pro Lys Lys Ile Arg Lys Ala
                485                 490                 495

Lys

<210>    283
<211>    422
<212>    PRT
<213>    Artificial Sequence

<220>
<223>    polypeptide sequence for effector protein of acidibacillus
         sulfuroxidans

<400>    283
Met Ile Lys Val Tyr Arg Tyr Glu Ile Val Lys Pro Leu Asp Leu Asp
1               5                   10                  15

Trp Lys Glu Phe Gly Thr Ile Leu Arg Gln Leu Gln Gln Glu Thr Arg
            20                  25                  30

Phe Ala Leu Asn Lys Ala Thr Gln Leu Ala Trp Glu Trp Met Gly Phe
        35                  40                  45

Ser Ser Asp Tyr Lys Asp Asn His Gly Glu Tyr Pro Lys Ser Lys Asp
    50                  55                  60

Ile Leu Gly Tyr Thr Asn Val His Gly Tyr Ala Tyr His Thr Ile Lys
65                  70                  75                  80

Thr Lys Ala Tyr Arg Leu Asn Ser Gly Asn Leu Ser Gln Thr Ile Lys
                85                  90                  95

Arg Ala Thr Asp Arg Phe Lys Ala Tyr Gln Lys Glu Ile Leu Arg Gly
            100                 105                 110

Asp Met Ser Ile Pro Ser Tyr Lys Arg Asp Ile Pro Leu Asp Leu Ile
        115                 120                 125

Lys Glu Asn Ile Ser Val Asn Arg Met Asn His Gly Asp Tyr Ile Ala
    130                 135                 140

Ser Leu Ser Leu Leu Ser Asn Pro Ala Lys Gln Glu Met Asn Val Lys
145                 150                 155                 160

Arg Lys Ile Ser Val Ile Ile Ile Val Arg Gly Ala Gly Lys Thr Ile
                165                 170                 175

Met Asp Arg Ile Leu Ser Gly Glu Tyr Gln Val Ser Ala Ser Gln Ile
            180                 185                 190

Ile His Asp Asp Arg Lys Asn Lys Trp Tyr Leu Asn Ile Ser Tyr Asp
        195                 200                 205

Phe Glu Pro Gln Thr Arg Val Leu Asp Leu Asn Lys Ile Met Gly Ile
    210                 215                 220

Asp Leu Gly Val Ala Val Ala Val Tyr Met Ala Phe Gln His Thr Pro
225                 230                 235                 240

Ala Arg Tyr Lys Leu Glu Gly Gly Glu Ile Glu Asn Phe Arg Arg Gln
                245                 250                 255

Val Glu Ser Arg Arg Ile Ser Met Leu Arg Gln Gly Lys Tyr Ala Gly
            260                 265                 270

Gly Ala Arg Gly Gly His Gly Arg Asp Lys Arg Ile Lys Pro Ile Glu
        275                 280                 285

Gln Leu Arg Asp Lys Ile Ala Asn Phe Arg Asp Thr Thr Asn His Arg
    290                 295                 300

Tyr Ser Arg Tyr Ile Val Asp Met Ala Ile Lys Glu Gly Cys Gly Thr
305                 310                 315                 320

Ile Gln Met Glu Asp Leu Thr Asn Ile Arg Asp Ile Gly Ser Arg Phe
                325                 330                 335

Leu Gln Asn Trp Thr Tyr Tyr Asp Leu Gln Gln Lys Ile Ile Tyr Lys
            340                 345                 350

Ala Glu Glu Ala Gly Ile Lys Val Ile Lys Ile Asp Pro Gln Tyr Thr
        355                 360                 365

Ser Gln Arg Cys Ser Glu Cys Gly Asn Ile Asp Ser Gly Asn Arg Ile
    370                 375                 380

Gly Gln Ala Ile Phe Lys Cys Arg Ala Cys Gly Tyr Glu Ala Asn Ala
385                 390                 395                 400

Asp Tyr Asn Ala Ala Arg Asn Ile Ala Ile Pro Asn Ile Asp Lys Ile
                405                 410                 415

Ile Ala Glu Ser Ile Lys
            420


<210>    284
<211>    1491
<212>    DNA
<213>    Artificial Sequence

<220>
<223>    human codon-optimized, SpCas12f1 encoding DNA sequence


<400>    284
atgggcgaga gcgttaaagc aatcaaactg aagatcctgg acatgttcct ggacccggag      60

tgcactaaac aggacgataa ctggcgtaaa gacctgtcta ccatgtctcg tttctgcgca     120

gaggcgggta acatgtgcct gcgcgacctg tacaattact tttctatgcc gaaagaagac     180

cgtattagct ccaaggatct gtacaacgcc atgtaccaca aaactaaact gctgcacccg     240

gaactgccgg gtaaagtggc gaaccagatc gtcaaccacg ctaaagatgt gtggaaacgc     300

aacgcgaagc tgatctatcg taatcagatc tctatgccga cttacaagat caccaccgca     360

ccgattcgcc tgcaaaacaa catctataaa ctgatcaaaa acaagaacaa atatatcatc     420

gatgtccagc tgtacagcaa agaatactct aaagacagcg gcaaaggcac ccaccgctac     480

ttcctggttg cagtgcgcga ttctagcacg cgtatgatct tcgaccgcat catgagcaaa     540

gaccacatcg atagctccaa atcctacacc cagggtcagc tgcagattaa aaaagaccat      600

cagggcaaat ggtactgtat cattccgtac accttcccga ctcatgagac cgtcctggat     660

ccggacaaag ttatgggtgt ggacctgggc gtggctaaag cagtgtattg ggcgttcaac     720

tcctcttaca aacgcggttg tatcgatggt ggtgaaattg aacacttccg caaaatgatc     780

cgtgctcgtc gtgtctccat tcagaaccag attaaacatt ctggcgacgc gcgcaagggt     840

catggtcgta aacgtgcact gaagccgatt gaaaccctgt ctgaaaaaga aaaaaacttc      900

cgcgatacta tcaaccatcg ttacgcaaac cgtatcgtgg aggcagcgat taagcagggc     960

tgcggcacca tccagatcga aaatctggag ggtatcgccg atactacggg ttccaaattt    1020

ctgaaaaact ggccatacta tgacctgcag accaaaatcg tgaacaaagc gaaggaacat    1080

ggcatcaccg tcgttgcgat caacccgcag tatacttctc aacgttgctc catgtgtggt 1140

tacattgaaa aaaccaaccg ttcctctcag gcagtgttcg agtgcaaaca gtgcggctat 1200

ggttcccgta ccatctgcat caactgccgc cacgttcagg taagcggtga tgtctgcgaa 1260

gaatgcggcg gcatcgttaa aaaagaaaac gttaacgcgg actataacgc ggcgaaaaac 1320

atctccaccc catatatcga tcagatcatc atggagaaat gtctggagct gggtatcccg 1380

taccgtagca tcacttgcaa agaatgcggt catatccagg cgtctggtaa cacttgtgag 1440

gtgtgcggtt ccaccaacat tctgaaaccg aaaaaaatcc gtaaagctaa a 1491

<210> 285
<211> 1266
<212> DNA
<213> Artificial Sequence

<220>
<223> human codon-optimized, AsCas12f1 encoding DNA sequence

<400> 285
atgatcaagg tttatcgcta tgaaattgtg aaaccgctgg acctggactg gaaagagttc 60

ggtactatcc tgcgtcagct gcagcaggaa acgcgtttcg cactgaacaa agcgacgcag 120

ctggcttggg aatggatggg cttcagctct gattacaaag acaaccacgg cgaataccct 180

aaatccaaag atatcctggg ttacaccaac gtgcacggtt atgcgtacca caccatcaaa 240

actaaagcgt accgcctgaa ctccggtaat ctgtctcaga ccatcaagcg tgcgaccgac 300

cgtttcaaag cgtaccagaa ggaaatcctg cgcggtgaca tgagcatccc gtcctataag 360

cgtgacatcc cgctggatct gatcaaagaa aacatcagcg taaaccgcat gaaccacggt 420

gactacatcg cctccctgtc tctgctgtct aacccggcaa agcaagagat gaacgttaaa 480

cgcaaaatct ccgttatcat tatcgttcgt ggcgcaggca aaaccatcat ggatcgtatt 540

ctgtccggtg aatatcaagt gtccgcaagc cagatcatcc atgacgatcg taaaaataaa 600

tggtatctga acatctctta tgatttcgaa ccgcagaccc gcgtactgga cctgaacaaa 660

atcatgggta tcgatctggg cgttgcagta gcggtgtaca tggctttcca gcacaccccg 720

gcacgctaca aactggaagg tggtgagatc gaaaacttcc gtcgtcaagt ggaatctcgt 780

cgcatctcta tgctgcgtca gggtaaatat gcaggtggtg cgcgtggtgg tcatggtcgt 840

gacaaacgta ttaaaccaat cgaacagctg cgcgataaaa tcgccaactt ccgtgatacc 900

accaaccacc gctactctcg ttatatcgtt gatatggcaa tcaaagaggg ttgtggtact 960

atccagatgg aagacctgac gaacatccgc gatatcggtt ctcgtttcct gcagaactgg 1020

acctactacg atctgcagca gaaaattatc tacaaagcgg aggaagcagg tatcaaagtc 1080

attaaaatcg atccgcagta tactagccag cgctgcagcg aatgtggtaa cattgatagc 1140

ggcaaccgca ttggtcaggc aatctttaaa tgccgcgctt gtggctacga agcgaacgca 1200

gactacaacg cggcgcgtaa catcgccatc ccgaatattg acaaaatcat tgccgaatct 1260

attaaa 1266

<210> 286
<211> 20
<212> DNA
<213> Artificial Sequence

<220>
<223> protospacer sequence of VEGFA-3 gene

<400> 286
ggggtgaccg ccggagcgcg 20

<210> 287
<211> 161
<212> DNA
<213> Artificial Sequence

<220>
<223> tracrRNA encoding DNA sequence for SpCas12f1 and AsCas12f1

<400> 287
cttcactgat aaagtggaga accgcttcac caaaagctgt cccttagggg attagaactt 60

gagtgaaggt gggctgcttg catcagccta atgtcgagaa gtgctttctt cggaaagtaa 120

ccctcgaaac aaattcattt ttcctctcca attctgcaca a 161

<210> 288
<211> 32
<212> DNA
<213> Artificial Sequence

<220>
<223> crRNA repeat sequence encoding DNA sequence for SpCas12f1

<400> 288
gtcgcatctt gcgtaagcgc gtggattgaa ac 32

<210> 289
<211> 29
<212> DNA
<213> Artificial Sequence

<220>
<223> crRNA repeat sequence encoding DNA sequence for AsCas12f1

<400> 289
gtttgcgagc tagcttgtgg agtgtgaac 29

<210>    290
<211>    52
<212>    DNA
<213>    Artificial Sequence

<220>
<223>    SpCas12f1, crRNA encoding DNA for DYtarget2(WT)


<400>    290
gtcgcatctt gcgtaagcgc gtggattgaa accacacaca cagtgggcta cc                52


<210>    291
<211>    58
<212>    DNA
<213>    Artificial Sequence

<220>
<223>    SpCas12f1, crRNA encoding DNA for DYtarget2(U6)


<400>    291
gtcgcatctt gcgtaagcgc gtggattgaa accacacaca cagtgggcta cctttttt          58


<210>    292
<211>    63
<212>    DNA
<213>    Artificial Sequence

<220>
<223>    SpCas12f1, crRNA encoding DNA for DYtarget2(U4AU6)


<400>    292
gtcgcatctt gcgtaagcgc gtggattgaa accacacaca cagtgggcta ccttttattt      60

ttt                                                                      63


<210>    293
<211>    52
<212>    DNA
<213>    Artificial Sequence

<220>
<223>    SpCas12f1, crRNA encoding DNA for VEGFA-3(WT)


<400>    293
gtcgcatctt gcgtaagcgc gtggattgaa acggggtgac cgccggagcg cg                52


<210>    294
<211>    58
<212>    DNA
<213>    Artificial Sequence

<220>
<223>    SpCas12f1, crRNA encoding DNA for VEGFA-3(U6)

<400>    294
gtcgcatctt gcgtaagcgc gtggattgaa acggggtgac cgccggagcg cgtttttt          58

<210>    295
<211>    63
<212>    DNA
<213>    Artificial Sequence

<220>
<223>    SpCas12f1, crRNA encoding DNA for VEGFA-3(U4AU6)

<400>    295
gtcgcatctt gcgtaagcgc gtggattgaa acggggtgac cgccggagcg cgttttattt          60

ttt                                                                        63

<210>    296
<211>    49
<212>    DNA
<213>    Artificial Sequence

<220>
<223>    AsCas12f1, crRNA encoding DNA for DYtarget2(WT)

<400>    296
gtttgcgagc tagcttgtgg agtgtgaacc acacacacag tgggctacc                      49

<210>    297
<211>    55
<212>    DNA
<213>    Artificial Sequence

<220>
<223>    AsCas12f1, crRNA encoding DNA sequence for DYtarget2(U6)

<400>    297
gtttgcgagc tagcttgtgg agtgtgaacc acacacacag tgggctacct ttttt               55

<210>    298
<211>    60
<212>    DNA
<213>    Artificial Sequence

<220>
<223>    AsCas12f1, crRNA encoding DNA for DYtarget2(U4AU6)

<400>    298
gtttgcgagc tagcttgtgg agtgtgaacc acacacacag tgggctacct tttatttttt          60

                                                                           60

<210>    299
<211>    49
<212>    DNA

<213>    Artificial Sequence

<220>
<223>    AsCas12f1, crRNA encoding DNA for VEGFA-3(WT)


<400>    299
gtttgcgagc tagcttgtgg agtgtgaacg gggtgaccgc cggagcgcg                    49


<210>    300
<211>    55
<212>    DNA
<213>    Artificial Sequence

<220>
<223>    AsCas12f1, crRNA encoding DNA for VEGFA-3(U6)


<400>    300
gtttgcgagc tagcttgtgg agtgtgaacg gggtgaccgc cggagcgcgt ttttt             55


<210>    301
<211>    60
<212>    DNA
<213>    Artificial Sequence

<220>
<223>    AsCas12f1, crRNA encoding DNA for VEGFA-3(U4AU6)


<400>    301
gtttgcgagc tagcttgtgg agtgtgaacg gggtgaccgc cggagcgcgt tttatttttt        60

                                                                          60


<210>    302
<211>    20
<212>    DNA
<213>    Artificial Sequence

<220>
<223>    reverse primer for synthesizing tracrRNA amplicon


<400>    302
ttgtgcagaa ttggagagga                                                    20


<210>    303
<211>    31
<212>    DNA
<213>    Artificial Sequence

<220>
<223>    Reverse primer for crRNA of SpCas12f1(DYtarget2, WT)


<400>    303
ggtagcccac tgtgtgtgtg gtttcaatcc a                                       31

<210>    304
<211>    37
<212>    DNA
<213>    Artificial Sequence

<220>
<223>    Reverse primer for crRNA of SpCas12f1(DYtarget2, U6)


<400>    304
aaaaaaggta gcccactgtg tgtgtggttt caatcca                                37


<210>    305
<211>    42
<212>    DNA
<213>    Artificial Sequence

<220>
<223>    Reverse primer for crRNA of SpCas12f1(DYtarget2, U4AU6)


<400>    305
aaaaaataaa aggtagccca ctgtgtgtgt ggtttcaatc ca                          42


<210>    306
<211>    31
<212>    DNA
<213>    Artificial Sequence

<220>
<223>    Reverse primer for crRNA of SpCas12f1(VEGFA-3, WT)


<400>    306
cgcgctccgg cggtcacccc gtttcaatcc a                                      31


<210>    307
<211>    37
<212>    DNA
<213>    Artificial Sequence

<220>
<223>    Reverse primer for crRNA of SpCas12f1(VEGFA-3, U6)


<400>    307
aaaaaacgcg ctccggcggt caccccgttt caatcca                                37


<210>    308
<211>    42
<212>    DNA
<213>    Artificial Sequence

<220>
<223>    Reverse primer for crRNA of SpCas12f1(VEGFA-3, U4AU6)


<400>    308

aaaaaataaa acgcgctccg gcggtcaccc cgtttcaatc ca                    42

<210>    309
<211>    31
<212>    DNA
<213>    Artificial Sequence

<220>
<223>    Reverse primer for crRNA of AsCas12f1(DYtarget2, WT)

<400>    309
ggtagcccac tgtgtgtgtg gttcacactc c                                31

<210>    310
<211>    37
<212>    DNA
<213>    Artificial Sequence

<220>
<223>    Reverse primer for crRNA of AsCas12f1(DYtarget2, U6)

<400>    310
aaaaaaggta gcccactgtg tgtgtggttc acactcc                          37

<210>    311
<211>    42
<212>    DNA
<213>    Artificial Sequence

<220>
<223>    Reverse primer for crRNA of AsCas12f1(DYtarget2, U4AU6)

<400>    311
aaaaaataaa aggtagccca ctgtgtgtgt ggttcacact cc                    42

<210>    312
<211>    31
<212>    DNA
<213>    Artificial Sequence

<220>
<223>    Reverse primer for crRNA of AsCas12f1(VEGFA-3, WT)

<400>    312
cgcgctccgg cggtcacccc gttcacactc c                                31

<210>    313
<211>    37
<212>    DNA
<213>    Artificial Sequence

<220>
<223>    Reverse primer for crRNA of AsCas12f1(VEGFA-3, U6)

<400>    313
aaaaaacgcg ctccggcggt caccccgttc acactcc                                37

<210>    314
<211>    42
<212>    DNA
<213>    Artificial Sequence

<220>
<223>    Reverse primer for crRNA of AsCas12f1(VEGFA-3, U4AU6)

<400>    314
aaaaaataaa acgcgctccg gcggtcaccc cgttcacact cc                          42

<210>    315
<211>    81
<212>    DNA
<213>    Artificial Sequence

<220>
<223>    HA tag sequence

<400>    315
tacccatacg atgttccaga ttacgcttat ccctacgacg tgcctgatta tgcataccca       60

tatgatgtcc ccgactatgc c                                                 81

<210>    316
<211>    3009
<212>    DNA
<213>    Artificial Sequence

<220>
<223>    Cas14a1 and cytidine deaminase fused protein

<400>    316
ccaaagaaga agcggaaagt ctcctcagag actgggcctg tcgccgtcga tccaaccctg       60

cgccgccgga ttgaacctca cgagtttgaa gtgttctttg accccgggga gctgagaaag      120

gagacatgcc tgctgtacga gatcaactgg ggaggcaggc actccatctg gaggcacacc      180

tctcagaaca caaataagca cgtggaggtg aacttcatcg agaagtttac cacagagcgg      240

tacttctgcc ccaataccag atgtagcatc acatggtttc tgagctggtc cccttgcgga      300

gagtgtagca gggccatcac cgagttcctg tccagatatc cacacgtgac actgtttatc      360

tacatcgcca ggctgtatca ccacgcagac ccaaggaata ggcagggcct gcgcgatctg      420

atcagctccg gcgtgaccat ccagatcatg acagagcagg agtccggcta ctgctggcgg      480

aacttcgtga attattctcc tagcaacgag gcccactggc ctaggtaccc acacctgtgg      540

gtgcgcctgt acgtgctgga gctgtattgc atcatcctgg gcctgccccc ttgtctgaat      600

atcctgcgga gaaagcagcc ccagctgacc ttctttacaa tcgccctgca gtcttgtcac 660

tatcagaggc tgccacccca catcctgtgg gccacaggcc tgaagtctgg aggatctagc 720

ggaggatcct ctggcagcga gacaccagga acaagcgagt cagcaacacc agagagcagt 780

ggcggcagca gcggcggcag catggccaag aacacaatta caaagacact gaagctgagg 840

atcgtgagac catacaacag cgctgaggtc gagaagattg tggctgatga aaagaacaac 900

agggaaaaga tcgccctcga gaagaacaag gataaggtga aggaggcctg ctctaagcac 960

ctgaaagtgg ccgcctactg caccacacag gtggagagga acgcctgtct gttttgtaaa 1020

gctcggaagc tggatgataa gttttaccag aagctgcggg gccagttccc cgatgccgtc 1080

ttttggcagg agattagcga gatcttcaga cagctgcaga agcaggccgc cgagatctac 1140

aaccagagcc tgatcgagct ctactacgag atcttcatca agggcaaggg cattgccaac 1200

gcctcctccg tggagcacta cctgagcgac gtgtgctaca caagagccgc cgagctcttt 1260

aagaacgccg ctatcgcttc cggctgaggg agcaagatta agagtaactt ccggctcaag 1320

gagctgaaga acatgaagag cggcctgccc actacaaaga gcgacaactt cccaattcca 1380

ctggtgaagc agaagggggg ccagtacaca gggttcgaga tttccaacca caacagcgac 1440

tttattatta agatcccctt tggcaggtgg caggtcaaga aggagattga caagtacagg 1500

ccctgggaga agtttgattt cgagcaggtg cagaagagcc ccaagcctat ttccctgctg 1560

ctgtccacac agcggcggaa gaggaacaag gggtggtcta aggatgaggg gaccgaggcc 1620

gagattaaga aagtgatgaa cggcgactac cagacaagct acatcgaggt caagcggggc 1680

agtaagattg gcgagaagag cgcctggatg ctgaacctga gcattgacgt gccaaagatt 1740

gataagggcg tggatcccag catcatcgga gggatcgatg tgggggtcaa gagccccctc 1800

gtgtgcgcca tcaacaacgc cttcagcagg tacagcatct ccgataacga cctgttccac 1860

tttaacaaga agatgttcgc ccggcggagg attttgctca agaagaaccg gcacaagcgg 1920

gccggacacg gggccaagaa caagctcaag cccatcacta tcctgaccga gaagagcgag 1980

aggttcagga agaagctcat cgagagatgg gcctgcgaga tcgccgattt ctttattaag 2040

aacaaggtcg gaacagtgca gatggagaac ctcgagagca tgaagaggaa ggaggattcc 2100

tacttcaaca ttcggctgag ggggttctgg ccctacgctg agatgcagaa caagattgag 2160

tttaagctga agcagtacgg gattgagatc cggaaggtgg cccccaacaa caccagcaag 2220

acctgcagca agtgcgggca cctcaacaac tacttcaact tcgagtaccg gaagaagaac 2280

aagttcccac acttcaagtg cgagaagtgc aactttaagg agaacgccga ttacaacgcc 2340

gccctgaaca tcagcaaccc taagctgaag agcactaagg aggagcccag cggcgggagc 2400

ggcgggagcg gggggagcac taatctgagc gacatcattg agaaggagac tgggaaacag 2460

ctggtcattc aggagtccat cctgatgctg cctgaggagg tggaggaagt gatcggcaac 2520

aagccagagt ctgacatcct ggtgcacacc gcctacgacg agtccacaga tgagaatgtg 2580

atgctgctga cctctgacgc ccccgagtat aagccttggg ccctggtcat ccaggattct 2640

aacggcgaga ataagatcaa gatgctgagc ggaggatccg gaggatctgg aggcagcacc 2700

aacctgtctg acatcatcga gaaggagaca ggcaagcagc tggtcatcca ggagagcatc 2760

ctgatgctgc ccgaagaagt cgaagaagtg atcggaaaca agcctgagag cgatatcctg 2820

gtccataccg cctacgacga gagtaccgac gaaaatgtga tgctgctgac atccgacgcc 2880

ccagagtata agccctgggc tctggtcatc caggattcca acggagagaa caaaatcaaa 2940

atgctgtctg gcggctcaaa aagaaccgcc gacggcagcg aattcgagcc caagaagaag 3000

aggaaagtc 3009

<210> 317
<211> 1003
<212> PRT
<213> Artificial Sequence

<220>
<223> Cas14a1 with CBE fusion protein(N terminal -Cytidine deaminase)

<400> 317
Pro Lys Lys Lys Arg Lys Val Ser Ser Glu Thr Gly Pro Val Ala Val
1               5                   10                  15

Asp Pro Thr Leu Arg Arg Arg Ile Glu Pro His Glu Phe Glu Val Phe
            20                  25                  30

Phe Asp Pro Arg Glu Leu Arg Lys Glu Thr Cys Leu Leu Tyr Glu Ile
        35                  40                  45

Asn Trp Gly Gly Arg His Ser Ile Trp Arg His Thr Ser Gln Asn Thr
    50                  55                  60

Asn Lys His Val Glu Val Asn Phe Ile Glu Lys Phe Thr Thr Glu Arg
65                  70                  75                  80

Tyr Phe Cys Pro Asn Thr Arg Cys Ser Ile Thr Trp Phe Leu Ser Trp
                85                  90                  95

Ser Pro Cys Gly Glu Cys Ser Arg Ala Ile Thr Glu Phe Leu Ser Arg
            100                 105                 110

Tyr Pro His Val Thr Leu Phe Ile Tyr Ile Ala Arg Leu Tyr His His
        115                 120                 125

Ala Asp Pro Arg Asn Arg Gln Gly Leu Arg Asp Leu Ile Ser Ser Gly
    130                 135                 140

Val Thr Ile Gln Ile Met Thr Glu Gln Glu Ser Gly Tyr Cys Trp Arg
145                 150                 155                 160

Asn Phe Val Asn Tyr Ser Pro Ser Asn Glu Ala His Trp Pro Arg Tyr
                165                 170                 175

Pro His Leu Trp Val Arg Leu Tyr Val Leu Glu Leu Tyr Cys Ile Ile
        180                 185                 190

Leu Gly Leu Pro Pro Cys Leu Asn Ile Leu Arg Arg Lys Gln Pro Gln
    195                 200                 205

Leu Thr Phe Phe Thr Ile Ala Leu Gln Ser Cys His Tyr Gln Arg Leu
    210                 215                 220

Pro Pro His Ile Leu Trp Ala Thr Gly Leu Lys Ser Gly Gly Ser Ser
225                 230                 235                 240

Gly Gly Ser Ser Gly Ser Glu Thr Pro Gly Thr Ser Glu Ser Ala Thr
                245                 250                 255

Pro Glu Ser Ser Gly Gly Ser Ser Gly Gly Ser Met Ala Lys Asn Thr
            260                 265                 270

Ile Thr Lys Thr Leu Lys Leu Arg Ile Val Arg Pro Tyr Asn Ser Ala
        275                 280                 285

Glu Val Glu Lys Ile Val Ala Asp Glu Lys Asn Asn Arg Glu Lys Ile
    290                 295                 300

Ala Leu Glu Lys Asn Lys Asp Lys Val Lys Glu Ala Cys Ser Lys His
305                 310                 315                 320

Leu Lys Val Ala Ala Tyr Cys Thr Thr Gln Val Glu Arg Asn Ala Cys
                325                 330                 335

Leu Phe Cys Lys Ala Arg Lys Leu Asp Asp Lys Phe Tyr Gln Lys Leu
            340                 345                 350

Arg Gly Gln Phe Pro Asp Ala Val Phe Trp Gln Glu Ile Ser Glu Ile
        355                 360                 365

Phe Arg Gln Leu Gln Lys Gln Ala Ala Glu Ile Tyr Asn Gln Ser Leu
    370                 375                 380

Ile Glu Leu Tyr Tyr Glu Ile Phe Ile Lys Gly Lys Gly Ile Ala Asn
385                 390                 395                 400

Ala Ser Ser Val Glu His Tyr Leu Ser Asp Val Cys Tyr Thr Arg Ala
                405                 410                 415

Ala Glu Leu Phe Lys Asn Ala Ala Ile Ala Ser Gly Leu Arg Ser Lys
            420                 425                 430

Ile Lys Ser Asn Phe Arg Leu Lys Glu Leu Lys Asn Met Lys Ser Gly
        435                 440                 445

Leu Pro Thr Thr Lys Ser Asp Asn Phe Pro Ile Pro Leu Val Lys Gln
    450                 455                 460

Lys Gly Gly Gln Tyr Thr Gly Phe Glu Ile Ser Asn His Asn Ser Asp
465                 470                 475                 480

Phe Ile Ile Lys Ile Pro Phe Gly Arg Trp Gln Val Lys Lys Glu Ile
                485                 490                 495

Asp Lys Tyr Arg Pro Trp Glu Lys Phe Asp Phe Glu Gln Val Gln Lys
            500                 505                 510

Ser Pro Lys Pro Ile Ser Leu Leu Leu Ser Thr Gln Arg Arg Lys Arg
        515                 520                 525

Asn Lys Gly Trp Ser Lys Asp Glu Gly Thr Glu Ala Glu Ile Lys Lys
    530                 535                 540

Val Met Asn Gly Asp Tyr Gln Thr Ser Tyr Ile Glu Val Lys Arg Gly
545                 550                 555                 560

Ser Lys Ile Gly Glu Lys Ser Ala Trp Met Leu Asn Leu Ser Ile Asp
                565                 570                 575

Val Pro Lys Ile Asp Lys Gly Val Asp Pro Ser Ile Ile Gly Gly Ile
            580                 585                 590

Asp Val Gly Val Lys Ser Pro Leu Val Cys Ala Ile Asn Asn Ala Phe
        595                 600                 605

Ser Arg Tyr Ser Ile Ser Asp Asn Asp Leu Phe His Phe Asn Lys Lys
    610                 615                 620

Met Phe Ala Arg Arg Arg Ile Leu Leu Lys Lys Asn Arg His Lys Arg
625                 630                 635                 640

Ala Gly His Gly Ala Lys Asn Lys Leu Lys Pro Ile Thr Ile Leu Thr
                645                 650                 655

Glu Lys Ser Glu Arg Phe Arg Lys Lys Leu Ile Glu Arg Trp Ala Cys
            660                 665                 670

Glu Ile Ala Asp Phe Phe Ile Lys Asn Lys Val Gly Thr Val Gln Met
        675                 680                 685

Glu Asn Leu Glu Ser Met Lys Arg Lys Glu Asp Ser Tyr Phe Asn Ile
    690                 695                 700

Arg Leu Arg Gly Phe Trp Pro Tyr Ala Glu Met Gln Asn Lys Ile Glu
705                 710                 715                 720

Phe Lys Leu Lys Gln Tyr Gly Ile Glu Ile Arg Lys Val Ala Pro Asn
                725                 730                 735

Asn Thr Ser Lys Thr Cys Ser Lys Cys Gly His Leu Asn Asn Tyr Phe
            740                 745                 750

Asn Phe Glu Tyr Arg Lys Lys Asn Lys Phe Pro His Phe Lys Cys Glu
        755                 760                 765

Lys Cys Asn Phe Lys Glu Asn Ala Asp Tyr Asn Ala Ala Leu Asn Ile
    770                 775                 780

Ser Asn Pro Lys Leu Lys Ser Thr Lys Glu Glu Pro Ser Gly Gly Ser
785                 790                 795                 800

Gly Gly Ser Gly Gly Ser Thr Asn Leu Ser Asp Ile Ile Glu Lys Glu
                805                 810                 815

Thr Gly Lys Gln Leu Val Ile Gln Glu Ser Ile Leu Met Leu Pro Glu
            820                 825                 830

Glu Val Glu Glu Val Ile Gly Asn Lys Pro Glu Ser Asp Ile Leu Val
        835                 840                 845

His Thr Ala Tyr Asp Glu Ser Thr Asp Glu Asn Val Met Leu Leu Thr
    850                 855                 860

Ser Asp Ala Pro Glu Tyr Lys Pro Trp Ala Leu Val Ile Gln Asp Ser
865                 870                 875                 880

Asn Gly Glu Asn Lys Ile Lys Met Leu Ser Gly Gly Ser Gly Gly Ser
                885                 890                 895

Gly Gly Ser Thr Asn Leu Ser Asp Ile Ile Glu Lys Glu Thr Gly Lys
            900                 905                 910

Gln Leu Val Ile Gln Glu Ser Ile Leu Met Leu Pro Glu Glu Val Glu
        915                 920                 925

Glu Val Ile Gly Asn Lys Pro Glu Ser Asp Ile Leu Val His Thr Ala
    930                 935                 940

Tyr Asp Glu Ser Thr Asp Glu Asn Val Met Leu Leu Thr Ser Asp Ala
945                 950                 955                 960

Pro Glu Tyr Lys Pro Trp Ala Leu Val Ile Gln Asp Ser Asn Gly Glu
                965                 970                 975

Asn Lys Ile Lys Met Leu Ser Gly Gly Ser Lys Arg Thr Ala Asp Gly
            980                 985                 990

Ser Glu Phe Glu Pro Lys Lys Lys Arg Lys Val
        995                 1000

<210>    318
<211>    3009
<212>    DNA
<213>    Artificial Sequence

<220>
<223>    Cas14a1 with CBE fusion protein(C terminal -Cytidine deaminase)
         encoding DNA

<400>    318
ccaaagaaga agcggaaagt catggccaag aacacaatta caaagacact gaagctgagg      60

atcgtgagac catacaacag cgctgaggtc gagaagattg tggctgatga aaagaacaac     120

agggaaaaga tcgccctcga gaagaacaag gataaggtga aggaggcctg ctctaagcac     180

ctgaaagtgg ccgcctactg caccacacag gtggagagga acgcctgtct gttttgtaaa     240

gctcggaagc tggatgataa gttttaccag aagctgcggg gccagttccc cgatgccgtc     300

ttttggcagg agattagcga gatcttcaga cagctgcaga agcaggccgc cgagatctac     360

aaccagagcc tgatcgagct ctactacgag atcttcatca agggcaaggg cattgccaac     420

gcctcctccg tggagcacta cctgagcgac gtgtgctaca caagagccgc cgagctcttt     480

aagaacgccg ctatcgcttc cgggctgagg agcaagatta agagtaactt ccggctcaag     540

gagctgaaga acatgaagag cggcctgccc actacaaaga gcgacaactt cccaattcca     600

ctggtgaagc agaagggggg ccagtacaca gggttcgaga tttccaacca caacagcgac     660

tttattatta agatcccctt tggcaggtgg caggtcaaga aggagattga caagtacagg 720

ccctgggaga agtttgattt cgagcaggtg cagaagagcc ccaagcctat ttccctgctg 780

ctgtccacac agcggcggaa gaggaacaag gggtggtcta aggatgaggg gaccgaggcc 840

gagattaaga aagtgatgaa cggcgactac cagacaagct acatcgaggt caagcggggc 900

agtaagattg gcgagaagag cgcctggatg ctgaacctga gcattgacgt gccaaagatt 960

gataagggcg tggatcccag catcatcgga gggatcgatg tgggggtcaa gagccccctc 1020

gtgtgcgcca tcaacaacgc cttcagcagg tacagcatct ccgataacga cctgttccac 1080

tttaacaaga agatgttcgc ccggcggagg attttgctca agaagaaccg gcacaagcgg 1140

gccggacacg gggccaagaa caagctcaag cccatcacta tcctgaccga gaagagcgag 1200

aggttcagga agaagctcat cgagagatgg gcctgcgaga tcgccgattt ctttattaag 1260

aacaaggtcg gaacagtgca gatggagaac ctcgagagca tgaagaggaa ggaggattcc 1320

tacttcaaca ttcggctgag ggggttctgg ccctacgctg agatgcagaa caagattgag 1380

tttaagctga agcagtacgg gattgagatc cggaaggtgg cccccaacaa caccagcaag 1440

acctgcagca agtgcgggca cctcaacaac tacttcaact tcgagtaccg gaagaagaac 1500

aagttcccac acttcaagtg cgagaagtgc aactttaagg agaacgccga ttacaacgcc 1560

gccctgaaca tcagcaaccc taagctgaag agcactaagg aggagccctc tggaggatct 1620

agcggaggat cctctggcag cgagacacca ggaacaagcg agtcagcaac accagagagc 1680

agtggcggca gcagcggcgg cagctcctca gagactgggc ctgtcgccgt cgatccaacc 1740

ctgcgccgcc ggattgaacc tcacgagttt gaagtgttct ttgacccccg ggagctgaga 1800

aaggagacat gcctgctgta cgagatcaac tggggaggca ggcactccat ctggaggcac 1860

acctctcaga acacaaataa gcacgtggag gtgaacttca tcgagaagtt taccacagag 1920

cggtacttct gccccaatac cagatgtagc atcacatggt ttctgagctg gtccccttgc 1980

ggagagtgta gcagggccat caccgagttc ctgtccagat atccacacgt gacactgttt 2040

atctacatcg ccaggctgta tcaccacgca gacccaagga ataggcaggg cctgcgcgat 2100

ctgatcagct ccggcgtgac catccagatc atgacagagc aggagtccgg ctactgctgg 2160

cggaacttcg tgaattattc tcctagcaac gaggcccact ggcctaggta cccacacctg 2220

tgggtgcgcc tgtacgtgct ggagctgtat tgcatcatcc tgggcctgcc cccttgtctg 2280

aatatcctgc ggagaaagca gccccagctg accttcttta caatcgccct gcagtcttgt 2340

cactatcaga ggctgccacc ccacatcctg tgggccacag gcctgaagag cggcgggagc 2400

ggcgggagcg gggggagcac taatctgagc gacatcattg agaaggagac tgggaaacag 2460

ctggtcattc aggagtccat cctgatgctg cctgaggagg tggaggaagt gatcggcaac 2520

aagccagagt ctgacatcct ggtgcacacc gcctacgacg agtccacaga tgagaatgtg 2580

atgctgctga cctctgacgc ccccgagtat aagccttggg ccctggtcat ccaggattct 2640

aacggcgaga ataagatcaa gatgctgagc ggaggatccg gaggatctgg aggcagcacc 2700

aacctgtctg acatcatcga gaaggagaca ggcaagcagc tggtcatcca ggagagcatc 2760

ctgatgctgc ccgaagaagt cgaagaagtg atcggaaaca agcctgagag cgatatcctg 2820

gtccataccg cctacgacga gagtaccgac gaaaatgtga tgctgctgac atccgacgcc 2880

ccagagtata agccctgggc tctggtcatc caggattcca acggagagaa caaaatcaaa 2940

atgctgtctg gcggctcaaa aagaaccgcc gacggcagcg aattcgagcc caagaagaag 3000

aggaaagtc 3009

<210> 319
<211> 1003
<212> PRT
<213> Artificial Sequence

<220>
<223> Cas14a1 with CBE fusion protein(C terminal -Cytidine deaminase)

<400> 319
Pro Lys Lys Lys Arg Lys Val Met Ala Lys Asn Thr Ile Thr Lys Thr
1               5                   10                  15

Leu Lys Leu Arg Ile Val Arg Pro Tyr Asn Ser Ala Glu Val Glu Lys
            20                  25                  30

Ile Val Ala Asp Glu Lys Asn Asn Arg Glu Lys Ile Ala Leu Glu Lys
        35                  40                  45

Asn Lys Asp Lys Val Lys Glu Ala Cys Ser Lys His Leu Lys Val Ala
    50                  55                  60

Ala Tyr Cys Thr Thr Gln Val Glu Arg Asn Ala Cys Leu Phe Cys Lys
65                  70                  75                  80

Ala Arg Lys Leu Asp Asp Lys Phe Tyr Gln Lys Leu Arg Gly Gln Phe
                85                  90                  95

Pro Asp Ala Val Phe Trp Gln Glu Ile Ser Glu Ile Phe Arg Gln Leu
            100                 105                 110

Gln Lys Gln Ala Ala Glu Ile Tyr Asn Gln Ser Leu Ile Glu Leu Tyr
        115                 120                 125

Tyr Glu Ile Phe Ile Lys Gly Lys Gly Ile Ala Asn Ala Ser Ser Val
    130                 135                 140

Glu His Tyr Leu Ser Asp Val Cys Tyr Thr Arg Ala Ala Glu Leu Phe
145                 150                 155                 160

Lys Asn Ala Ala Ile Ala Ser Gly Leu Arg Ser Lys Ile Lys Ser Asn
                165                 170                 175

Phe Arg Leu Lys Glu Leu Lys Asn Met Lys Ser Gly Leu Pro Thr Thr

180                 185                 190

Lys Ser Asp Asn Phe Pro Ile Pro Leu Val Lys Gln Lys Gly Gly Gln
        195                 200                 205

Tyr Thr Gly Phe Glu Ile Ser Asn His Asn Ser Asp Phe Ile Ile Lys
    210                 215                 220

Ile Pro Phe Gly Arg Trp Gln Val Lys Lys Glu Ile Asp Lys Tyr Arg
225                 230                 235                 240

Pro Trp Glu Lys Phe Asp Phe Glu Gln Val Gln Lys Ser Pro Lys Pro
                245                 250                 255

Ile Ser Leu Leu Leu Ser Thr Gln Arg Arg Lys Arg Asn Lys Gly Trp
            260                 265                 270

Ser Lys Asp Glu Gly Thr Glu Ala Glu Ile Lys Lys Val Met Asn Gly
        275                 280                 285

Asp Tyr Gln Thr Ser Tyr Ile Glu Val Lys Arg Gly Ser Lys Ile Gly
    290                 295                 300

Glu Lys Ser Ala Trp Met Leu Asn Leu Ser Ile Asp Val Pro Lys Ile
305                 310                 315                 320

Asp Lys Gly Val Asp Pro Ser Ile Ile Gly Gly Ile Asp Val Gly Val
                325                 330                 335

Lys Ser Pro Leu Val Cys Ala Ile Asn Asn Ala Phe Ser Arg Tyr Ser
            340                 345                 350

Ile Ser Asp Asn Asp Leu Phe His Phe Asn Lys Lys Met Phe Ala Arg
        355                 360                 365

Arg Arg Ile Leu Leu Lys Lys Asn Arg His Lys Arg Ala Gly His Gly
    370                 375                 380

Ala Lys Asn Lys Leu Lys Pro Ile Thr Ile Leu Thr Glu Lys Ser Glu
385                 390                 395                 400

Arg Phe Arg Lys Lys Leu Ile Glu Arg Trp Ala Cys Glu Ile Ala Asp
                405                 410                 415

Phe Phe Ile Lys Asn Lys Val Gly Thr Val Gln Met Glu Asn Leu Glu
            420                 425                 430

Ser Met Lys Arg Lys Glu Asp Ser Tyr Phe Asn Ile Arg Leu Arg Gly
        435                 440                 445

Phe Trp Pro Tyr Ala Glu Met Gln Asn Lys Ile Glu Phe Lys Leu Lys
    450                 455                 460

Gln Tyr Gly Ile Glu Ile Arg Lys Val Ala Pro Asn Asn Thr Ser Lys
465                 470                 475                 480

Thr Cys Ser Lys Cys Gly His Leu Asn Asn Tyr Phe Asn Phe Glu Tyr
                485                 490                 495

Arg Lys Lys Asn Lys Phe Pro His Phe Lys Cys Glu Lys Cys Asn Phe
            500                 505                 510

Lys Glu Asn Ala Asp Tyr Asn Ala Ala Leu Asn Ile Ser Asn Pro Lys

515                 520                 525

Leu Lys Ser Thr Lys Glu Glu Pro Ser Gly Gly Ser Ser Gly Gly Ser
    530                 535                 540

Ser Gly Ser Glu Thr Pro Gly Thr Ser Glu Ser Ala Thr Pro Glu Ser
545                 550                 555                 560

Ser Gly Gly Ser Ser Gly Gly Ser Ser Ser Glu Thr Gly Pro Val Ala
                565                 570                 575

Val Asp Pro Thr Leu Arg Arg Arg Ile Glu Pro His Glu Phe Glu Val
            580                 585                 590

Phe Phe Asp Pro Arg Glu Leu Arg Lys Glu Thr Cys Leu Leu Tyr Glu
        595                 600                 605

Ile Asn Trp Gly Gly Arg His Ser Ile Trp Arg His Thr Ser Gln Asn
    610                 615                 620

Thr Asn Lys His Val Glu Val Asn Phe Ile Glu Lys Phe Thr Thr Glu
625                 630                 635                 640

Arg Tyr Phe Cys Pro Asn Thr Arg Cys Ser Ile Thr Trp Phe Leu Ser
                645                 650                 655

Trp Ser Pro Cys Gly Glu Cys Ser Arg Ala Ile Thr Glu Phe Leu Ser
            660                 665                 670

Arg Tyr Pro His Val Thr Leu Phe Ile Tyr Ile Ala Arg Leu Tyr His
        675                 680                 685

His Ala Asp Pro Arg Asn Arg Gln Gly Leu Arg Asp Leu Ile Ser Ser
    690                 695                 700

Gly Val Thr Ile Gln Ile Met Thr Glu Gln Glu Ser Gly Tyr Cys Trp
705                 710                 715                 720

Arg Asn Phe Val Asn Tyr Ser Pro Ser Asn Glu Ala His Trp Pro Arg
                725                 730                 735

Tyr Pro His Leu Trp Val Arg Leu Tyr Val Leu Glu Leu Tyr Cys Ile
            740                 745                 750

Ile Leu Gly Leu Pro Pro Cys Leu Asn Ile Leu Arg Arg Lys Gln Pro
        755                 760                 765

Gln Leu Thr Phe Phe Thr Ile Ala Leu Gln Ser Cys His Tyr Gln Arg
    770                 775                 780

Leu Pro Pro His Ile Leu Trp Ala Thr Gly Leu Lys Ser Gly Gly Ser
785                 790                 795                 800

Gly Gly Ser Gly Gly Ser Thr Asn Leu Ser Asp Ile Ile Glu Lys Glu
                805                 810                 815

Thr Gly Lys Gln Leu Val Ile Gln Glu Ser Ile Leu Met Leu Pro Glu
            820                 825                 830

Glu Val Glu Glu Val Ile Gly Asn Lys Pro Glu Ser Asp Ile Leu Val
        835                 840                 845

His Thr Ala Tyr Asp Glu Ser Thr Asp Glu Asn Val Met Leu Leu Thr

850                 855                 860

Ser Asp Ala Pro Glu Tyr Lys Pro Trp Ala Leu Val Ile Gln Asp Ser
865                 870                 875                 880

Asn Gly Glu Asn Lys Ile Lys Met Leu Ser Gly Gly Ser Gly Gly Ser
                885                 890                 895

Gly Gly Ser Thr Asn Leu Ser Asp Ile Ile Glu Lys Glu Thr Gly Lys
            900                 905                 910

Gln Leu Val Ile Gln Glu Ser Ile Leu Met Leu Pro Glu Glu Val Glu
        915                 920                 925

Glu Val Ile Gly Asn Lys Pro Glu Ser Asp Ile Leu Val His Thr Ala
    930                 935                 940

Tyr Asp Glu Ser Thr Asp Glu Asn Val Met Leu Leu Thr Ser Asp Ala
945                 950                 955                 960

Pro Glu Tyr Lys Pro Trp Ala Leu Val Ile Gln Asp Ser Asn Gly Glu
                965                 970                 975

Asn Lys Ile Lys Met Leu Ser Gly Gly Ser Lys Arg Thr Ala Asp Gly
            980                 985                 990

Ser Glu Phe Glu Pro Lys Lys Lys Arg Lys Val
        995                 1000

<210>    320
<211>    2823
<212>    DNA
<213>    Artificial Sequence

<220>
<223>    Cas14a1 with ABE fusion protein(N terminal -Adenine deaminase)
         encoding DNA

<400>    320
atgtccgaag tcgagttttc ccatgagtac tggatgagac acgcattgac tctcgcaaag      60

agggcttggg atgaacgcga ggtgcccgtg ggggcagtac tcgtgcataa caatcgcgta     120

atcggcgaag gttggaatag gccgatcgga cgccacgacc ccactgcaca tgcggaaatc     180

atggcccttc gacagggagg gcttgtgatg cagaattatc gacttatcga tgcgacgctg     240

tacgtcacgc ttgaaccttg cgtaatgtgc gcgggagcta tgattcactc ccgcattgga     300

cgagttgtat tcggtgcccg cgacgccaag acgggtgccg caggttcact gatggacgtg     360

ctgcatcacc caggcatgaa ccaccgggta gaaatcacag aaggcatatt ggcggacgaa     420

tgtgcggcgc tgttgtccga cttttttcgc atgcggaggc aggagatcaa ggcccagaaa     480

aaagcacaat cctctactga ctctggtggt tcttctggtg gttctagcgg cagcgagact     540

cccgggacct cagagtccgc cacacccgaa agttctggtg gttcttctgg tggttcttcc     600

gaagtcgagt tttcccatga gtactggatg agacacgcat tgactctcgc aaagagggct     660

cgagatgaac gcgaggtgcc cgtgggggca gtactcgtgc tcaacaatcg cgtaatcggc 720

gaaggttgga atagggcaat cggactccac gaccccactg cacatgcgga aatcatggcc 780

cttcgacagg gagggcttgt gatgcagaat tatcgactta tcgatgcgac gctgtacgtc 840

acgtttgaac cttgcgtaat gtgcgcggga gctatgattc actcccgcat tggacgagtt 900

gtattcggtg ttcgcaacgc caagacgggt gccgcaggtt cactgatgga cgtgctgcat 960

tacccaggca tgaaccaccg ggtagaaatc acagaaggca tattggcgga cgaatgtgcg 1020

gcgctgttgt gttacttttt tcgcatgccc aggcaggtct ttaacgccca gaaaaaagca 1080

caatcctcta ctgactctgg tggttcttct ggtggttcta gcggcagcga gactcccggg 1140

acctcagagt ccgccacacc cgaaagttct ggtggttctt ctggtggttc tgccaagaac 1200

acaattacaa agacactgaa gctgaggatc gtgagaccat acaacagcgc tgaggtcgag 1260

aagattgtgg ctgatgaaaa gaacaacagg gaaaagatcg ccctcgagaa gaacaaggat 1320

aaggtgaagg aggcctgctc taagcacctg aaagtggccg cctactgcac cacacaggtg 1380

gagaggaacg cctgtctgtt ttgtaaagct cggaagctgg atgataagtt ttaccagaag 1440

ctgcggggcc agttccccga tgccgtcttt tggcaggaga ttagcgagat cttcagacag 1500

ctgcagaagc aggccgccga gatctacaac cagagcctga tcgagctcta ctacgagatc 1560

ttcatcaagg gcaagggcat tgccaacgcc tcctccgtgg agcactacct gagcgacgtg 1620

tgctacacaa gagccgccga gctctttaag aacgccgcta tcgcttccgg gctgaggagc 1680

aagattaaga gtaacttccg gctcaaggag ctgaagaaca tgaagagcgg cctgcccact 1740

acaaagagcg acaacttccc aattccactg gtgaagcaga agggggccca gtacacaggg 1800

ttcgagattt ccaaccacaa cagcgacttt attattaaga tcccctttgg caggtggcag 1860

gtcaagaagg agattgacaa gtacaggccc tgggagaagt ttgatttcga gcaggtgcag 1920

aagagcccca agcctatttc cctgctgctg tccacacagc ggcggaagag gaacaagggg 1980

tggtctaagg atgaggggac cgaggccgag attaagaaag tgatgaacgg cgactaccag 2040

acaagctaca tcgaggtcaa gcggggcagt aagattggcg agaagagcgc ctggatgctg 2100

aacctgagca ttgacgtgcc aaagattgat aagggcgtgg atcccagcat catcggaggg 2160

atcgatgtgg gggtcaagag ccccctcgtg tgcgccatca acaacgcctt cagcaggtac 2220

agcatctccg ataacgacct gttccacttt aacaagaaga tgttcgcccg gcggaggatt 2280

ttgctcaaga agaaccggca caagcgggcc ggacacgggg ccaagaacaa gctcaagccc 2340

atcactatcc tgaccgagaa gagcgagagg ttcaggaaga agctcatcga gagatgggcc 2400

tgcgagatcg ccgatttctt tattaagaac aaggtcggaa cagtgcagat ggagaacctc 2460

gagagcatga agaggaagga ggattcctac ttcaacattc ggctgagggg gttctggccc 2520

tacgctgaga tgcagaacaa gattgagttt aagctgaagc agtacgggat tgagatccgg 2580

aaggtggccc ccaacaacac cagcaagacc tgcagcaagt gcgggcacct caacaactac 2640

ttcaacttcg agtaccggaa gaagaacaag ttcccacact tcaagtgcga gaagtgcaac 2700

tttaaggaga acgccgatta caacgccgcc ctgaacatca gcaaccctaa gctgaagagc 2760

actaaggagg agcccaaaag gccggcggcc acgaaaaagg ccggccaggc aaaaaagaaa 2820

aag 2823

<210> 321
<211> 941
<212> PRT
<213> Artificial Sequence

<220>
<223> Cas14a1 with ABE fusion protein(N terminal -Adenine deaminase)

<400> 321
Met Ser Glu Val Glu Phe Ser His Glu Tyr Trp Met Arg His Ala Leu
1               5                   10                  15

Thr Leu Ala Lys Arg Ala Trp Asp Glu Arg Glu Val Pro Val Gly Ala
            20                  25                  30

Val Leu Val His Asn Asn Arg Val Ile Gly Glu Gly Trp Asn Arg Pro
        35                  40                  45

Ile Gly Arg His Asp Pro Thr Ala His Ala Glu Ile Met Ala Leu Arg
    50                  55                  60

Gln Gly Gly Leu Val Met Gln Asn Tyr Arg Leu Ile Asp Ala Thr Leu
65                  70                  75                  80

Tyr Val Thr Leu Glu Pro Cys Val Met Cys Ala Gly Ala Met Ile His
                85                  90                  95

Ser Arg Ile Gly Arg Val Val Phe Gly Ala Arg Asp Ala Lys Thr Gly
            100                 105                 110

Ala Ala Gly Ser Leu Met Asp Val Leu His His Pro Gly Met Asn His
        115                 120                 125

Arg Val Glu Ile Thr Glu Gly Ile Leu Ala Asp Glu Cys Ala Ala Leu
    130                 135                 140

Leu Ser Asp Phe Phe Arg Met Arg Arg Gln Glu Ile Lys Ala Gln Lys
145                 150                 155                 160

Lys Ala Gln Ser Ser Thr Asp Ser Gly Gly Ser Ser Gly Gly Ser Ser
                165                 170                 175

Gly Ser Glu Thr Pro Gly Thr Ser Glu Ser Ala Thr Pro Glu Ser Ser
            180                 185                 190

Gly Gly Ser Ser Gly Gly Ser Ser Glu Val Glu Phe Ser His Glu Tyr
        195                 200                 205

Trp Met Arg His Ala Leu Thr Leu Ala Lys Arg Ala Arg Asp Glu Arg
    210                 215                 220

Glu Val Pro Val Gly Ala Val Leu Val Leu Asn Asn Arg Val Ile Gly
225                 230                 235                 240

Glu Gly Trp Asn Arg Ala Ile Gly Leu His Asp Pro Thr Ala His Ala
                245                 250                 255

Glu Ile Met Ala Leu Arg Gln Gly Gly Leu Val Met Gln Asn Tyr Arg
            260                 265                 270

Leu Ile Asp Ala Thr Leu Tyr Val Thr Phe Glu Pro Cys Val Met Cys
        275                 280                 285

Ala Gly Ala Met Ile His Ser Arg Ile Gly Arg Val Val Phe Gly Val
    290                 295                 300

Arg Asn Ala Lys Thr Gly Ala Ala Gly Ser Leu Met Asp Val Leu His
305                 310                 315                 320

Tyr Pro Gly Met Asn His Arg Val Glu Ile Thr Glu Gly Ile Leu Ala
                325                 330                 335

Asp Glu Cys Ala Ala Leu Leu Cys Tyr Phe Phe Arg Met Pro Arg Gln
            340                 345                 350

Val Phe Asn Ala Gln Lys Lys Ala Gln Ser Ser Thr Asp Ser Gly Gly
        355                 360                 365

Ser Ser Gly Gly Ser Ser Gly Ser Glu Thr Pro Gly Thr Ser Glu Ser
    370                 375                 380

Ala Thr Pro Glu Ser Ser Gly Gly Ser Ser Gly Gly Ser Ala Lys Asn
385                 390                 395                 400

Thr Ile Thr Lys Thr Leu Lys Leu Arg Ile Val Arg Pro Tyr Asn Ser
                405                 410                 415

Ala Glu Val Glu Lys Ile Val Ala Asp Glu Lys Asn Asn Arg Glu Lys
            420                 425                 430

Ile Ala Leu Glu Lys Asn Lys Asp Lys Val Lys Glu Ala Cys Ser Lys
        435                 440                 445

His Leu Lys Val Ala Ala Tyr Cys Thr Thr Gln Val Glu Arg Asn Ala
    450                 455                 460

Cys Leu Phe Cys Lys Ala Arg Lys Leu Asp Asp Lys Phe Tyr Gln Lys
465                 470                 475                 480

Leu Arg Gly Gln Phe Pro Asp Ala Val Phe Trp Gln Glu Ile Ser Glu
                485                 490                 495

Ile Phe Arg Gln Leu Gln Lys Gln Ala Ala Glu Ile Tyr Asn Gln Ser
            500                 505                 510

Leu Ile Glu Leu Tyr Tyr Glu Ile Phe Ile Lys Gly Lys Gly Ile Ala
        515                 520                 525

Asn Ala Ser Ser Val Glu His Tyr Leu Ser Asp Val Cys Tyr Thr Arg
    530                 535                 540

Ala Ala Glu Leu Phe Lys Asn Ala Ala Ile Ala Ser Gly Leu Arg Ser
545                 550                 555                 560

Lys Ile Lys Ser Asn Phe Arg Leu Lys Glu Leu Lys Asn Met Lys Ser
                565                 570                 575

Gly Leu Pro Thr Thr Lys Ser Asp Asn Phe Pro Ile Pro Leu Val Lys
            580                 585                 590

Gln Lys Gly Gly Gln Tyr Thr Gly Phe Glu Ile Ser Asn His Asn Ser
        595                 600                 605

Asp Phe Ile Ile Lys Ile Pro Phe Gly Arg Trp Gln Val Lys Lys Glu
    610                 615                 620

Ile Asp Lys Tyr Arg Pro Trp Glu Lys Phe Asp Phe Glu Gln Val Gln
625                 630                 635                 640

Lys Ser Pro Lys Pro Ile Ser Leu Leu Leu Ser Thr Gln Arg Arg Lys
                645                 650                 655

Arg Asn Lys Gly Trp Ser Lys Asp Glu Gly Thr Glu Ala Glu Ile Lys
            660                 665                 670

Lys Val Met Asn Gly Asp Tyr Gln Thr Ser Tyr Ile Glu Val Lys Arg
        675                 680                 685

Gly Ser Lys Ile Gly Glu Lys Ser Ala Trp Met Leu Asn Leu Ser Ile
    690                 695                 700

Asp Val Pro Lys Ile Asp Lys Gly Val Asp Pro Ser Ile Ile Gly Gly
705                 710                 715                 720

Ile Asp Val Gly Val Lys Ser Pro Leu Val Cys Ala Ile Asn Asn Ala
                725                 730                 735

Phe Ser Arg Tyr Ser Ile Ser Asp Asn Asp Leu Phe His Phe Asn Lys
            740                 745                 750

Lys Met Phe Ala Arg Arg Arg Ile Leu Leu Lys Lys Asn Arg His Lys
        755                 760                 765

Arg Ala Gly His Gly Ala Lys Asn Lys Leu Lys Pro Ile Thr Ile Leu
    770                 775                 780

Thr Glu Lys Ser Glu Arg Phe Arg Lys Lys Leu Ile Glu Arg Trp Ala
785                 790                 795                 800

Cys Glu Ile Ala Asp Phe Phe Ile Lys Asn Lys Val Gly Thr Val Gln
                805                 810                 815

Met Glu Asn Leu Glu Ser Met Lys Arg Lys Glu Asp Ser Tyr Phe Asn
            820                 825                 830

Ile Arg Leu Arg Gly Phe Trp Pro Tyr Ala Glu Met Gln Asn Lys Ile
        835                 840                 845

Glu Phe Lys Leu Lys Gln Tyr Gly Ile Glu Ile Arg Lys Val Ala Pro
    850                 855                 860

Asn Asn Thr Ser Lys Thr Cys Ser Lys Cys Gly His Leu Asn Asn Tyr
865                 870                 875                 880

Phe Asn Phe Glu Tyr Arg Lys Lys Asn Lys Phe Pro His Phe Lys Cys
                885                 890                 895

Glu Lys Cys Asn Phe Lys Glu Asn Ala Asp Tyr Asn Ala Ala Leu Asn
            900                 905                 910

Ile Ser Asn Pro Lys Leu Lys Ser Thr Lys Glu Glu Pro Lys Arg Pro
        915                 920                 925

Ala Ala Thr Lys Lys Ala Gly Gln Ala Lys Lys Lys Lys
    930                 935                 940

<210>    322
<211>    2823
<212>    DNA
<213>    Artificial Sequence

<220>
<223>    Cas14a1 with ABE fusion protein(C terminal -Adenine deaminase)
         encoding DNA

<400>    322
atggccaaga acacaattac aaagacactg aagctgagga tcgtgagacc atacaacagc      60

gctgaggtcg agaagattgt ggctgatgaa aagaacaaca gggaaaagat cgccctcgag     120

aagaacaagg ataaggtgaa ggaggcctgc tctaagcacc tgaaagtggc cgcctactgc     180

accacacagg tggagaggaa cgcctgtctg ttttgtaaag ctcggaagct ggatgataag     240

ttttaccaga agctgcgggg ccagttcccc gatgccgtct tttggcagga gattagcgag     300

atcttcagac agctgcagaa gcaggccgcc gagatctaca accagagcct gatcgagctc     360

tactacgaga tcttcatcaa gggcaagggc attgccaacg cctcctccgt ggagcactac     420

ctgagcgacg tgtgctacac aagagccgcc gagctcttta agaacgccgc tatcgcttcc     480

gggctgagga gcaagattaa gagtaacttc cggctcaagg agctgaagaa catgaagagc     540

ggcctgccca ctacaaagag cgacaacttc ccaattccac tggtgaagca gaaggggggc      600

cagtacacag ggttcgagat ttccaaccac aacagcgact ttattattaa gatccccttt     660

ggcaggtggc aggtcaagaa ggagattgac aagtacaggc cctgggagaa gtttgatttc     720

gagcaggtgc agaagagccc caagcctatt tccctgctgc tgtccacaca gcggcggaag     780

aggaacaagg ggtggtctaa ggatgagggg accgaggccg agattaagaa agtgatgaac     840

ggcgactacc agacaagcta catcgaggtc aagcggggca gtaagattgg cgagaagagc     900

gcctggatgc tgaacctgag cattgacgtg ccaaagattg ataagggcgt ggatcccagc     960

atcatcggag ggatcgatgt gggggtcaag agccccctcg tgtgcgccat caacaacgcc     1020

ttcagcaggt acagcatctc cgataacgac ctgttccact ttaacaagaa gatgttcgcc    1080

cggcggagga ttttgctcaa gaagaaccgg cacaagcggg ccggacacgg ggccaagaac    1140

aagctcaagc ccatcactat cctgaccgag aagagcgaga ggttcaggaa gaagctcatc    1200

gagagatggg cctgcgagat cgccgatttc tttattaaga acaaggtcgg aacagtgcag 1260

atggagaacc tcgagagcat gaagaggaag gaggattcct acttcaacat tcggctgagg 1320

gggttctggc cctacgctga gatgcagaac aagattgagt ttaagctgaa gcagtacggg 1380

attgagatcc ggaaggtggc ccccaacaac accagcaaga cctgcagcaa gtgcgggcac 1440

ctcaacaact acttcaactt cgagtaccgg aagaagaaca agttcccaca cttcaagtgc 1500

gagaagtgca actttaagga gaacgccgat tacaacgccg ccctgaacat cagcaaccct 1560

aagctgaaga gcactaagga ggagccctct ggaggatcta gcggaggatc ctctggcagc 1620

gagacaccag gaacaagcga gtcagcaaca ccagagagca gtggcggcag cagcggcggc 1680

agctccgaag tcgagttttc ccatgagtac tggatgagac acgcattgac tctcgcaaag 1740

agggcttggg atgaacgcga ggtgcccgtg ggggcagtac tcgtgcataa caatcgcgta 1800

atcggcgaag gttggaatag gccgatcgga cgccacgacc ccactgcaca tgcggaaatc 1860

atggcccttc gacagggagg gcttgtgatg cagaattatc gacttatcga tgcgacgctg 1920

tacgtcacgc ttgaaccttg cgtaatgtgc gcgggagcta tgattcactc ccgcattgga 1980

cgagttgtat tcggtgcccg cgacgccaag acgggtgccg caggttcact gatggacgtg 2040

ctgcatcacc caggcatgaa ccaccgggta gaaatcacag aaggcatatt ggcggacgaa 2100

tgtgcggcgc tgttgtccga cttttttcgc atgcggaggc aggagatcaa ggcccagaaa 2160

aaagcacaat cctctactga ctctggtggt tcttctggtg gttctagcgg cagcgagact 2220

cccgggacct cagagtccgc cacacccgaa agttcaggtg gatcttcagg tggatcttcg 2280

gaagtggaat tttcgcacga gtattggatg aggcacgctt taactctcgc taagagagca 2340

cgagacgaac gggaagtgcc ggttggggct gtcctcgtac tcaataatcg agttatcgga 2400

gaaggctgga acagggcaat cggactccac gatcccacag ctcatgccga gataatggcg 2460

cttcgacaag gaggcctagt catgcaaaat tatcgtctta ttgacgcgac cctctacgtg 2520

acctttgagc catgcgttat gtgtgcgggt gcaatgatac attcccggat aggacgtgta 2580

gtatttggag ttcgcaacgc gaagaccggt gcggctggtt ctctcatgga tgtcctgcac 2640

taccctggga tgaatcaccg cgttgaaatc actgaaggca ttttggccga tgaatgcgcg 2700

gccctgttat gttacttttt tcgcatgccc aggcaggtct ttaacgcaca gaagaaagcc 2760

caatcgtcca ctgataaaag gccggcggcc acgaaaaagg ccggccaggc aaaaaagaaa 2820

aag 2823

<210> 323
<211> 941
<212> PRT
<213> Artificial Sequence

<220>

<223>    Cas14a1 with ABE fusion protein(C terminal -Adenine deaminase)

<400>    323
Met Ala Lys Asn Thr Ile Thr Lys Thr Leu Lys Leu Arg Ile Val Arg
1               5                   10                  15

Pro Tyr Asn Ser Ala Glu Val Glu Lys Ile Val Ala Asp Glu Lys Asn
            20                  25                  30

Asn Arg Glu Lys Ile Ala Leu Glu Lys Asn Lys Asp Lys Val Lys Glu
        35                  40                  45

Ala Cys Ser Lys His Leu Lys Val Ala Ala Tyr Cys Thr Thr Gln Val
    50                  55                  60

Glu Arg Asn Ala Cys Leu Phe Cys Lys Ala Arg Lys Leu Asp Asp Lys
65                  70                  75                  80

Phe Tyr Gln Lys Leu Arg Gly Gln Phe Pro Asp Ala Val Phe Trp Gln
                85                  90                  95

Glu Ile Ser Glu Ile Phe Arg Gln Leu Gln Lys Gln Ala Ala Glu Ile
            100                 105                 110

Tyr Asn Gln Ser Leu Ile Glu Leu Tyr Tyr Glu Ile Phe Ile Lys Gly
        115                 120                 125

Lys Gly Ile Ala Asn Ala Ser Ser Val Glu His Tyr Leu Ser Asp Val
    130                 135                 140

Cys Tyr Thr Arg Ala Ala Glu Leu Phe Lys Asn Ala Ala Ile Ala Ser
145                 150                 155                 160

Gly Leu Arg Ser Lys Ile Lys Ser Asn Phe Arg Leu Lys Glu Leu Lys
                165                 170                 175

Asn Met Lys Ser Gly Leu Pro Thr Thr Lys Ser Asp Asn Phe Pro Ile
            180                 185                 190

Pro Leu Val Lys Gln Lys Gly Gly Gln Tyr Thr Gly Phe Glu Ile Ser
        195                 200                 205

Asn His Asn Ser Asp Phe Ile Ile Lys Ile Pro Phe Gly Arg Trp Gln
    210                 215                 220

Val Lys Lys Glu Ile Asp Lys Tyr Arg Pro Trp Glu Lys Phe Asp Phe
225                 230                 235                 240

Glu Gln Val Gln Lys Ser Pro Lys Pro Ile Ser Leu Leu Leu Ser Thr
                245                 250                 255

Gln Arg Arg Lys Arg Asn Lys Gly Trp Ser Lys Asp Glu Gly Thr Glu
            260                 265                 270

Ala Glu Ile Lys Lys Val Met Asn Gly Asp Tyr Gln Thr Ser Tyr Ile
        275                 280                 285

Glu Val Lys Arg Gly Ser Lys Ile Gly Glu Lys Ser Ala Trp Met Leu
    290                 295                 300

Asn Leu Ser Ile Asp Val Pro Lys Ile Asp Lys Gly Val Asp Pro Ser
305                 310                 315                 320

Ile Ile Gly Gly Ile Asp Val Gly Val Lys Ser Pro Leu Val Cys Ala
                325                 330                 335

Ile Asn Asn Ala Phe Ser Arg Tyr Ser Ile Ser Asp Asn Asp Leu Phe
            340                 345                 350

His Phe Asn Lys Lys Met Phe Ala Arg Arg Arg Ile Leu Leu Lys Lys
        355                 360                 365

Asn Arg His Lys Arg Ala Gly His Gly Ala Lys Asn Lys Leu Lys Pro
    370                 375                 380

Ile Thr Ile Leu Thr Glu Lys Ser Glu Arg Phe Arg Lys Lys Leu Ile
385                 390                 395                 400

Glu Arg Trp Ala Cys Glu Ile Ala Asp Phe Phe Ile Lys Asn Lys Val
                405                 410                 415

Gly Thr Val Gln Met Glu Asn Leu Glu Ser Met Lys Arg Lys Glu Asp
            420                 425                 430

Ser Tyr Phe Asn Ile Arg Leu Arg Gly Phe Trp Pro Tyr Ala Glu Met
        435                 440                 445

Gln Asn Lys Ile Glu Phe Lys Leu Lys Gln Tyr Gly Ile Glu Ile Arg
    450                 455                 460

Lys Val Ala Pro Asn Asn Thr Ser Lys Thr Cys Ser Lys Cys Gly His
465                 470                 475                 480

Leu Asn Asn Tyr Phe Asn Phe Glu Tyr Arg Lys Lys Asn Lys Phe Pro
                485                 490                 495

His Phe Lys Cys Glu Lys Cys Asn Phe Lys Glu Asn Ala Asp Tyr Asn
            500                 505                 510

Ala Ala Leu Asn Ile Ser Asn Pro Lys Leu Lys Ser Thr Lys Glu Glu
        515                 520                 525

Pro Ser Gly Gly Ser Ser Gly Gly Ser Ser Gly Ser Glu Thr Pro Gly
    530                 535                 540

Thr Ser Glu Ser Ala Thr Pro Glu Ser Ser Gly Gly Ser Ser Gly Gly
545                 550                 555                 560

Ser Ser Glu Val Glu Phe Ser His Glu Tyr Trp Met Arg His Ala Leu
                565                 570                 575

Thr Leu Ala Lys Arg Ala Trp Asp Glu Arg Glu Val Pro Val Gly Ala
            580                 585                 590

Val Leu Val His Asn Asn Arg Val Ile Gly Glu Gly Trp Asn Arg Pro
        595                 600                 605

Ile Gly Arg His Asp Pro Thr Ala His Ala Glu Ile Met Ala Leu Arg
    610                 615                 620

Gln Gly Gly Leu Val Met Gln Asn Tyr Arg Leu Ile Asp Ala Thr Leu
625                 630                 635                 640

Tyr Val Thr Leu Glu Pro Cys Val Met Cys Ala Gly Ala Met Ile His
                645                 650                 655

Ser Arg Ile Gly Arg Val Val Phe Gly Ala Arg Asp Ala Lys Thr Gly
            660                 665                 670

Ala Ala Gly Ser Leu Met Asp Val Leu His His Pro Gly Met Asn His
        675                 680                 685

Arg Val Glu Ile Thr Glu Gly Ile Leu Ala Asp Glu Cys Ala Ala Leu
    690                 695                 700

Leu Ser Asp Phe Phe Arg Met Arg Arg Gln Glu Ile Lys Ala Gln Lys
705                 710                 715                 720

Lys Ala Gln Ser Ser Thr Asp Ser Gly Gly Ser Ser Gly Gly Ser Ser
                725                 730                 735

Gly Ser Glu Thr Pro Gly Thr Ser Glu Ser Ala Thr Pro Glu Ser Ser
            740                 745                 750

Gly Gly Ser Ser Gly Gly Ser Ser Glu Val Glu Phe Ser His Glu Tyr
        755                 760                 765

Trp Met Arg His Ala Leu Thr Leu Ala Lys Arg Ala Arg Asp Glu Arg
    770                 775                 780

Glu Val Pro Val Gly Ala Val Leu Val Leu Asn Asn Arg Val Ile Gly
785                 790                 795                 800

Glu Gly Trp Asn Arg Ala Ile Gly Leu His Asp Pro Thr Ala His Ala
                805                 810                 815

Glu Ile Met Ala Leu Arg Gln Gly Gly Leu Val Met Gln Asn Tyr Arg
            820                 825                 830

Leu Ile Asp Ala Thr Leu Tyr Val Thr Phe Glu Pro Cys Val Met Cys
        835                 840                 845

Ala Gly Ala Met Ile His Ser Arg Ile Gly Arg Val Val Phe Gly Val
    850                 855                 860

Arg Asn Ala Lys Thr Gly Ala Ala Gly Ser Leu Met Asp Val Leu His
865                 870                 875                 880

Tyr Pro Gly Met Asn His Arg Val Glu Ile Thr Glu Gly Ile Leu Ala
                885                 890                 895

Asp Glu Cys Ala Ala Leu Leu Cys Tyr Phe Phe Arg Met Pro Arg Gln
            900                 905                 910

Val Phe Asn Ala Gln Lys Lys Ala Gln Ser Ser Thr Asp Lys Arg Pro
        915                 920                 925

Ala Ala Thr Lys Lys Ala Gly Gln Ala Lys Lys Lys Lys
    930                 935                 940

<210>    324
<211>    233
<212>    DNA
<213>    Artificial Sequence

<220>

<223>    sgRNA encoding DNA sequence for DYtarget2(T4GT6)

<400>    324
cttcactgat aaagtggaga accgcttcac caaaagctgt cccttagggg attagaactt        60

gagtgaaggt gggctgcttg catcagccta atgtcgagaa gtgctttctt cggaaagtaa       120

ccctcgaaac aaattcattt ttcctctcca attctgcaca agaaagttgc agaacccgaa       180

tagacgaatg aaggaatgca accacacaca cagtgggcta ccttttgttt ttt              233

<210>    325
<211>    56
<212>    DNA
<213>    Artificial Sequence

<220>
<223>    reverse primer of sgRNA for DYtarget2(T4GT6)

<400>    325
aaaaaacaaa aggtagccca ctgtgtgtgt ggttgcattc cttcattcgt ctattc            56